(19) Europäisches Patentamt
European Patent Office
Office européen des brevets

(11) Veröffentlichungsnummer: **0 044 940**
**B1**

(12) # EUROPÄISCHE PATENTSCHRIFT

(45) Veröffentlichungstag der Patentschrift: **19.09.84**

(51) Int. Cl.³: **C 07 D 493/04**, A 61 K 31/34

(21) Anmeldenummer: **81104741.4**

(22) Anmeldetag: **20.06.81**

(54) **Amino-desoxy-1.4; 3.6-dianhydro-hexit-nitrate, Verfahren zu ihrer Herstellung und pharmazeutische Zubereitung.**

(30) Priorität: **25.07.80 DE 3028340**

(43) Veröffentlichungstag der Anmeldung: **03.02.82 Patentblatt 82/5**

(45) Bekanntmachung des Hinweises auf die Patenterteilung: **19.09.84 Patentblatt 84/38**

(84) Benannte Vertragsstaaten: **AT BE CH FR GB IT LI NL SE**

(56) Entgegenhaltungen:
**DE - A - 2 751 934**
**DE - B - 2 903 927**
**US - A - 3 886 186**

(73) Patentinhaber: **Dr. Willmar Schwabe GmbH & Co., Willmar-Schwabe-Strasse 4, D-7500 Karlsruhe 41 (DE)**

(72) Erfinder: **Klessing, Klaus, Dr. Dipl.-Chem., Rheingoldstrasse 3, D-7505 Ettlingen 5 (DE)**
Erfinder: **Chatterjee, Shyam Sunder, Dr. Dipl.-Chem., Werrabronner Strasse 8a, D-7500 Karlsruhe 41 (DE)**
Erfinder: **Gabard, Bernard Louis, Dr. Dipl.-Ing., Gürrickstrasse 44, D-7500 Karlsruhe 31 (DE)**

(74) Vertreter: **Bunke, Holger, Dr. Dipl.-Chem. et al, Patentanwälte Prinz, Bunke & Partner Lessingstrasse 9, D-7000 Stuttgart 1 (DE)**

**EP 0 044 940 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Amino-desoxy-1.4;3.6-dianhydro-hexit-nitrate der allgemeinen Formel I,

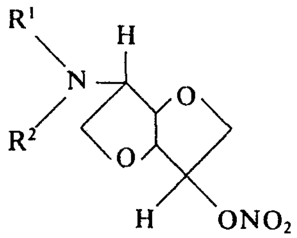

(I)

worin

$R^1$ und $R^2$ jeweils unabhängig voneinander ein Wasserstoffatom oder eine Niedrigalkylgruppe mit 1 bis 4 C-Atomen bedeuten

oder worin

$R^1$ ein Wasserstoffatom oder eine Niedrigalkylgruppe mit 1 bis 4 C-Atomen und
$R^2$ einen Acylrest einer aliphatischen oder einfach ungesättigten, gegebenenfalls methylsubstituierten Monocarbonsäure mit 2 bis 8 C-Atomen, einen Nicotinoyl-, 2-O-Acetylsalicoylrest oder einen 1-Adamantylrest bedeuten

oder worin

$R^1$ ein Wasserstoffatom und
$R^2$ einen 2-Hydroxy-3-(subst.)phenoxy-prop-1-yl-Rest der allgemeinen Formel Ia

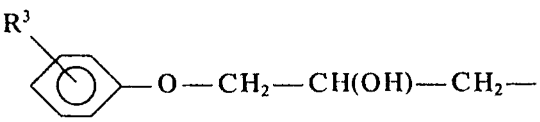

(Ia)

bedeuten, worin

$R^3$ ein Wasserstoffatom, eine Niedrigalkylgruppe mit 1 bis 4 C-Atomen oder eine Niedrigalkenylgruppe mit 2 bis 4 C-Atomen, den Trifluormethylrest, eine Hydroxygruppe, eine Niedrigalkoxygruppe mit 1 bis 4 C-Atomen oder eine Niedrigalkenyloxy-Gruppe mit 2 bis 4 C-Atomen, eine Cyanogruppe oder einen Carbamoylmethylrest bedeuten,

oder worin

$R^1$ ein Wasserstoffatom und
$R^2$ einen 2-Hydroxy-3-($\alpha$-naphthyloxy)-prop-1-yl-Rest bedeuten, wobei der nicht mit der Hydroxypropylgruppe verätherte Ring des Naphthalingerüstes ganz oder teilweise hydriert und durch eine Oxogruppe substituiert sein kann,

oder worin

$R^1$ ein Wasserstoffatom oder eine Niedrigalkylgruppe mit 1 bis 4 C-Atomen und
$R^2$ eine $\omega$-(subst.)-Phenylalkylgruppe der allgemeinen Formel Ib

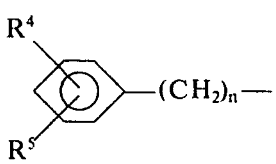

(Ib)

bedeuten, worin

n = eine ganze Zahl von 1–6 sein kann und
$R^4$ und $R^5$ jeweils unabhängig voneinander ein Wasserstoffatom, eine Niedrigalkylgruppe mit 1 bis 4 C-Atomen oder eine Niedrig-alkenylgruppe mit 2 bis 4 C-Atomen, den Trifluormethylrest, eine

Hydroxygruppe, eine Niedrigalkoxygruppe mit 1 bis 4 C-Atomen oder eine Niedrigalkenyloxygruppe mit 2 bis 4 C-Atomen oder ein Fluor- oder Chloratom bedeuten

oder worin

$R^1$ ein Wasserstoffatom oder eine Niedrigalkylgruppe mit 1 bis 4 C-Atomen und
$R^2$ den Diphenylmethyl-Rest oder Cinnamyl-Rest bedeuten

oder worin

$R^1$ ein Wasserstoffatom oder eine Niedrigalkylgruppe mit 1 bis 4 C-Atomen und
$R^2$ eine$\omega$-(subst.)-Phenoxyalkylgruppe der allgemeinen Formel Ic

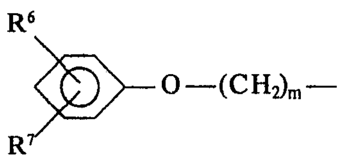

(Ic)

bedeuten, worin

m = eine ganze Zahl von 2–8 sein kann und
$R^6$ und $R^7$ unabhängig voneinander ein Wasserstoffatom, eine Niedrigalkylgruppe mit 1 bis 4 C-Atomen oder eine Niedrigalkenylgruppe mit 2 bis 4 C-Atomen, den Trifluormethylrest, eine Hydroxygruppe, eine Niedrigalkoxygruppe mit 1 bis 4 C-Atomen oder eine Niedrigalkenyloxy-Gruppe mit 2 bis 4 C-Atomen, ein Fluor- oder Chloratom, die Amino- oder Acetylaminogruppe, eine mono- oder di-Niedrigalkylaminogruppe mit 1 bis 4 C-Atomen bedeuten oder
$R^6$ und $R^7$ zusammen mit dem Phenylrest den $\alpha$-Naphthylrest bilden

oder worin

$R^1$ und $R^2$ gemeinsam mit dem Stickstoffatom, an das sie gebunden sind, den Rest eines cyclischen, nicht-aromatischen, gegebenenfalls ein weiteres Heteroatom enthaltenden sekundären Amins mit 5 bis 7 Ringatomen darstellen

oder worin

$R^1$ und $R^2$ gemeinsam den Pyridoxylidenrest bedeuten,

sowie deren pharmakologisch unbedenkliche Säureadditionssalze.

Das Grundgerüst dieser Verbindungen besteht aus einem der stereoisomeren, unter Epimerisierung ineinander umwandelbaren 1.4;3.6-Dianhydro-hexite, nämlich entweder dem 1.4;3.6-Dianhydro-L-idit (= »Isoidid«) (II),

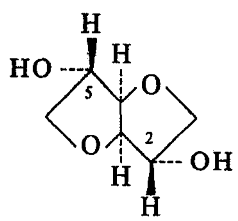

(II)

bei dem die OH-Gruppen in 2- und 5-Stellung jeweils exo-Konfiguration aufweisen, oder dem 1.4;3.6-Dianhydro-D-glucit (= »Isosorbid«) (III),

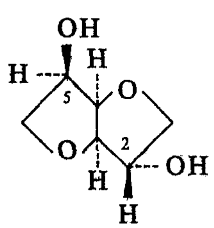

(III)

der eine 2-exo-ständige und eine 5-endo-ständige OH-Gruppe aufweist und somit – bei verschiedenen Substituenten in 2- und 5-Stellung – in zwei isomeren Formen auftritt.

Das Grundgerüst einiger Verbindungen schließlich besteht aus dem 1.4;3.6-Dianhydro-D-mannit (= »Isomannid«) (IV),

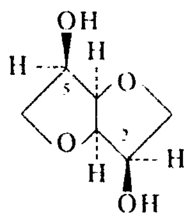

(IV)

der zwei endo-ständige OH-Gruppen aufweist.

Da im Gegensatz zu den Glucit-Derivaten bei den Idit- und Mannit-Derivaten eine Unterscheidung zwischen den 2- und 5-Substituenten nicht möglich ist, weil das $C^2$-Atom bei Drehung des Moleküls um 180° zum $C^5$-Atom wird, sind Hinweise auf die 5-Stellung oder 2-Stellung von Substituenten bei diesen Derivaten an sich überflüssig. Des besseren Vergleiches der Strukturen der einzelnen Verbindungen mit den allgemeinen Formeln wegen werden aber hier die Isoidid-Derivate durchweg als 5-Amino-isoidid-Derivate bezeichnet, da sie aus den in 5-Stellung acylsubstituierten Isosorbid-Derivaten entstehen. Entsprechend werden die als Ausgangsverbindungen verwendeten Isomannid-Acylderivate als 2-Acylisomannid-Derivate bezeichnet, da aus ihnen die in 2-Stellung substituierten Isosorbid-Derivate hergestellt werden.

Eine kurze Zusammenfassung über die Stereoisomerie der 1.4;3.6-Dianhydro-hexite gibt J. A. Mills in Advances in Carbohydrate Chem. 10, 1–53 (1955).

Die Erfindung betrifft weiter Verfahren zur Herstellung der eingangs genannten Amino-desoxy-1.4;3.6-dianhydro-hexit-nitrate sowie pharmazeutische Zubereitungen, die die erfindungsgemäßen Verbindungen enthalten.

Die Nitrate des 1.4;3.6-Dianhydro-D-glucits (auch 1.4;3.6-Dianhydro-D-sorbit genannt) sind z. B. aus der US-PS 3 886 186 bekannt, und zwar sowohl die 2- und 5-Mononitrate als auch das 2,5-Dinitrat des Isosorbids. Diese Nitrate, insbesondere das Dinitrat, das bereits als Arzneimittel im Handel ist, sind pharmakologisch wirksame Substanzen mit hämodynamischer, vasodilatorischer und antianginöser Wirksamkeit, die insbesondere bei Koronarinsuffizienz und zur Behandlung von Angina Pectoris verwendet werden.

Die Pharmakokinetik des Dinitrats und der Mononitrate von Isosorbid, Isomannid und Isoidid wurde von Bogaert und Rosseel in Naunyn-Schmiedeberg's Arch. Pharmacol. 275, 339 (1972) beschrieben.

Es hat sich jedoch gezeigt, daß die Nitrate unangenehme Nebenwirkungen, insbesondere Kopfschmerzen, verursachen. Die Mononitrate sind außerdem schlechter resorbierbar als etwa das Isosorbiddinitrat (ISDN). Es kommt hinzu, daß sich die Dinitrate des Isosorbids, Isomannids und Isoidids nur unter besonderen Vorsichtsmaßnahmen herstellen und handhaben lassen, da sie explosiv sind.

Somit bestand ein Bedürfnis nach der Bereitstellung neuer pharmazeutischer Mittel mit gleichem Wirkungsspektrum, die jedoch die genannten Nachteile nicht aufweisen, und nach der Schaffung neuer 1.4;3.6-Dianhydro-hexit-mononitrate, die als wirksame Bestandteile solcher pharmazeutischer Mittel verwendet werden können.

Die der Erfindung zugrundeliegende Aufgabe besteht darin, das angeführte Bedürfnis zu befriedigen, die Lösung dieser Aufgabe darin, die erfindungsgemäßen Stoffe bereitzustellen.

Gegenstand der Erfindung sind demnach

1. N-substituierte 5-Amino-5-desoxy-1.4;3.6-dianhydro-L-idit-2-nitrate sowie das N-unsubstituierte 5-Amino-5-desoxy-1.4;3.6-dianhydro-L-idit-2-nitrat der allgemeinen Formel V,

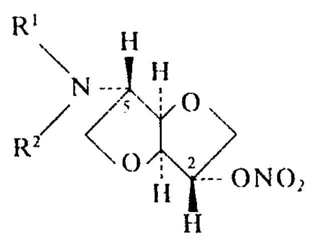

(V)

worin $R^1$ und $R^2$ die in Anspruch 1 genannten Bedeutungen besitzen, sowie deren physiologisch unbedenkliche Säureadditionssalze;

2. die entsprechend N-substituierten 5-Amino-5-desoxy-1.4;3.6-dianhydro-D-glucit-2-nitrate sowie das N-unsubstituierte 5-Amino-5-desoxy-1.4;3.6-dianhydro-D-glucit-2-nitrat der allgemeinen Formel VI,

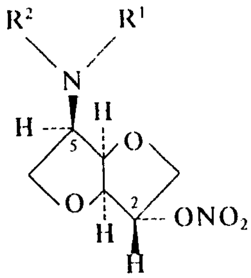

(VI)

sowie deren physiologisch unbedenkliche Säureadditionssalze;

3. die entsprechend N-substituierten 2-Amino-2-desoxy-1.4;3.6-dianhydro-D-glucit-5-nitrate und das N-unsubstituierte 2-Amino-2-desoxy-1.4;3.6-dianhydro-D-glucit-5-nitrat der allgemeinen Formel VII,

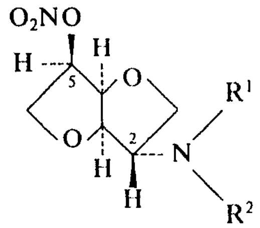

(VII)

sowie deren physiologisch unbedenkliche Säureadditionssalze;

4. die entsprechend N-substituierten 5-Amino-5-desoxy-1.4;3.6-dianhydro-D-mannit-2-nitrate und das N-unsubstituierte 5-Amino-5-desoxy-1.4;3.6-dianhydro-D-mannit-2-nitrat der allgemeinen Formel VIII,

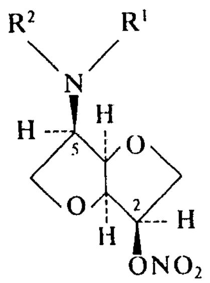

(VIII)

sowie deren physiologisch unbedenkliche Säureadditionssalze.

Die erfindungsgemäßen Verbindungen besitzen koronardurchflußsteigernde, spasmolytische, blutdrucksenkende, negativ inotrope und die Herzfrequenz senkende Wirksamkeit. Sie sind zur Behandlung von Konorarerkrankungen, zur Kupierung und Prophylaxe von Angina-Pectoris-Anfällen, zur Nachbehandlung von Herzinfarkten und zur Behandlung von Herzinsuffizienzen geeignet. Die neuen Verbindungen besitzen eine gute therapeutische Breite. Die orale Absorption ist besonders gut und die Wirkungsdauer lang. Darüber hinaus bewirken sie eine Verbesserung der peripheren Durchblutung und der Gehirndurchblutung.

Die Handhabung und Herstellung der erfindungsgemäßen Verbindungen ist weit weniger gefährlich als etwa beim bekannten ISDN, weil sie nicht explosiv sind.

Die erfindungsgemäßen Verbindungen besitzen im 1.4;3.6-Dianhydrohexit-Grundgerüst vier asymmetrische C-Atome und liegen in optisch aktiver Form vor, da als Ausgangsverbindungen optisch reine 1.4;3.6-Dianhydro-hexite verwendet werden, die aus natürlich vorkommenden Zuckeralkoholen leicht zugänglich sind.

Die erfindungsgemäßen Verbindungen können ausgehend von den epimeren, unsubstituierten 1.4;3.6-Dianhydro-hexiten, also ausgehend von L-Isoidid, D-Isosorbid und D-Isomannid, hergestellt werden, wobei im Falle des D-Isosorbids als Ausgangsverbindung mehrere verschiedene Synthesewege möglich sind.

Einer dieser Wege besteht erfindungsgemäß darin, daß der entsprechende 1.4;3.6-Dianhydro-hexit mit einem Sulfonsäurechlorid, vorzugsweise mit Methansulfonsäurechlorid oder Toluolsulfonsäurechlorid, in einem geeigneten wasserfreien Lösungsmittel und in Gegenwart einer Hilfsbase, vorzugsweise in Pyridin oder in Chloroform/Triäthylamin, bei erniedrigter Temperatur, vorzugsweise zwischen

−20° und +10°C, in den entsprechenden Mono-O-acyl-1.4;3.6-dianhydro-hexit übergeführt wird, welcher dann durch Zusatz einer wäßrigen, beispielsweise 25%igen Ammoniaklösung oder durch Zusatz eines primären oder sekundären Alkylamins mit 1 bis 4 C-Atomen oder eines primären Amins wie beispielsweise 1-Aminoadamantan oder eines cyclischen, nicht-aromatischen, sekundären Amins einer Ammonolyse bzw. Aminolyse unterworfen wird, und zwar vorteilhaft unter erhöhtem Druck, vorzugsweise bei einem Druck von 2—20 at, und erhöhter Temperatur, vorzugsweise bei 90 bis 180°C. Die Ammonolyse bzw. Aminolyse wird zweckmäßig in einem geschlossenen Stahlautoklaven, gegebenenfalls unter Zusatz eines geeigneten Lösungsmittels wie z. B. Äthanol, Butanol oder Dioxan, bis zur quantitativen Umsetzung durchgeführt.

Bei der Ammonolyse bzw. Aminolyse zum entsprechenden, gegebenenfalls N-substituierten Aminodesoxy-1.4;3.6-dianhydro-hexit wird die Mesylat- bzw. Tosylatgruppe durch die Amino- oder die entsprechend substituierte Aminogruppe nach dem Reaktionsmuster einer typischen bimolekularen nukleophilen Substitution ($S_N2$-Reaktion) ausgetauscht, die stets mit einer Umkehr der Konfiguration am zentralen Kohlenstoffatom verbunden ist. Diese Konfigurationsumkehr, dem Fachmann auch unter den Begriffen »Inversion« oder »Walden-Umkehr« geläufig, ist der Grund dafür, daß aus dem 1.4;3.6-Dianhydro- D-glucit-5-acyl-Derivat, bei dem der Acylrest in 5-Stellung endo-ständig vorliegt, stets das in 5-Stellung durch die Aminogruppe oder die entsprechend substituierte Aminogruppe substituierte 1.4;3.6-Dianhydro-L-idit-Derivat entsteht, bei dem der anstelle des Acylrests in das Molekül eingetretene Substituent nicht mehr in endo-, sondern in exo-Stellung steht. Die mit der $S_N2$-Reaktion verbundene Walden-Umkehr ist in ganz entsprechender Weise verantwortlich dafür, daß aus dem entsprechenden Idit-Acylat stets das in 5-Stellung endosubstituierte Glucit-Derivat, aus dem Mannit-Acylat das entsprechende, in 2-Stellung exo-substituierte Glucit-Derivat und aus dem Glucit-2-exo-acylat das entsprechende, in 2-Stellung endo-substituierte Mannit-Derivat entsteht.

Die im Verlauf des bisher beschriebenen ersten Syntheseweges entstandenen, gegebenenfalls N-substituierten Aminodesoxy-1.4;3.6-dianhydro-hexite weisen in 2- oder 5-Stellung des 1.4;3.6-Dianhydro-hexit-Ringsystems jeweils eine freie Hydroxylgruppe auf. Diese freie Hydroxylgruppe wird mit Salpetersäure, Nitriersäure oder mit einem Gemisch aus Salpetersäure und Eisessig/Acetanhydrid in Gegenwart von Harnstoff bei erniedrigter Temperatur, vorzugsweise bei —20 bis 0°C, zu dem entsprechenden Amino-desoxy-1.4;3.6-dianhydro-hexit-mononitrat verestert, wobei anstelle des Amino-desoxy-1.4;3.6-dianhydro-hexits in Form der freien Base zur Veresterung auch ein geeignetes Säureadditionssalz, beispielsweise das entsprechende Salz der Methansulfonsäure, Salpetersäure oder Schwefelsäure eingesetzt werden kann.

Das so entstandene Mononitrat kann im Falle des N-unsubstituierten oder N-monoalkylsubstituierten Amino-desoxy-1.4;3.6-dianhydro-hexit-nitrates anschließend mit einem Säurechlorid, mit Pyridoxal, mit Benzylchlorid oder einem geeigneten, gegebenenfalls phenylsubstituierten Phenylalkylhalogenid oder Phenylalkylmethansulfonat, mit einem gegebenenfalls phenylsubstituierten 1.2-Epoxy-$\omega$-phenoxyalkan oder mit einem gegebenenfalls phenylsubstituierten 1-Halogen- oder 1-Methansulfonyloxy-$\omega$-phenoxy-alkan zu der gewünschten erfindungsgemäßen Verbindung in an sich bekannter Weise kondensiert werden, vorteilhaft unter Zusatz eines geeigneten Lösungsmittels und gegebenenfalls einer Hilfsbase oder in Gegenwart eines Überschusses des entsprechenden Amino-desoxy-1.4;3.6-dianhydro-hexit-nitrats.

Zur Herstellung derjenigen erfindungsgemäßen Verbindungen der allgemeinen Formel I, worin $R^1$ Wasserstoff und $R^2$ eine Niedrigalkylgruppe mit 1 bis 4 C-Atomen oder den 1-Adamantylrest bedeuten, wird das entsprechende 1.4;3.6-Dianhydro-hexit-acylat, z. B. 1.4;3.6-Dianhydro-D-glucit-2- oder -5-methansulfonat, 1.4;3.6-Dianhydro-D-mannit-2-methansulfonat oder 1.4;3.6-Dianhydro-L-idit-5-methansulfonat, der Aminolyse mit primären oder sekundären Alkylaminen mit 1 bis 4 C-Atomen oder mit 1-Aminoadamantan unterworfen, und zwar vorteilhaft unter erhöhtem Druck, vorzugsweise bei einem Druck von 2—20 at, und erhöhter Temperatur, vorzugsweise bei 90 bis 180°C, und gegebenenfalls in Gegenwart eines Lösungsmittels, vorzugsweise von Äthanol, Butanol, Dioxan oder Glykoldiäthern. Die Aminolyse wird zweckmäßigerweise in einem geschlossenen Stahlautoklaven bis zur quantitativen Umsetzung durchgeführt.

Die Aminolyse findet unter Konfigurationsumkehr des substituierten Kohlenstoffatoms statt, so daß beispielsweise aus dem 1.4;3.6-Dianhydro-D-glucit-2-methansulfonat der N-mono- oder -dialkylsubstituierte 2-Amino-2-desoxy-1.4;3.6-dianhydro-D-mannit, aus dem 1.4;3.6-Dianhydro-D-glucit-5-methansulfonat der entsprechend N-substituierte 5-Amino-5-desoxy-1.4;3.6-dianhydro-L-idit, aus dem 1.4;3.6-dianhydro-D-mannit-2-methansulfonat das 2-Amino-2-desoxy-1.4;3.6-dianhydro-D-glucit-Derivat und aus dem 1.4;3.6-Dianhydro-L-idit-5-methansulfonat das 5-Amino-5-desoxy-1.4;3.6-dianhydro-D-glucit-Derivat entsteht.

Als primäre oder sekundäre Amine können z. B. Methylamin, Äthylamin, Propylamin, Isopropylamin, Butylamin, Isobutylamin, tert.-Butylamin, Dimethylamin, Diäthylamin, Dipropylamin, Dibutylamin, Methyläthylamin, Methylpropylamin usw. eingesetzt werden.

Anschließend an die Aminolyse wird die freie Hydroxylgruppe der entsprechend N-mono- oder N-disubstituierten Amino-desoxy-1.4;3.6-dianhydro-hexite in der zuvor beschriebenen, an sich bekannten Weise mit Salpetersäure verestert.

Zur Herstellung derjenigen erfindungsgemäßen Verbindungen der allgemeinen Formel I, worin $R^1$ und

$R^2$ gemeinsam mit dem Stickstoffatom, an das sie gebunden sind, den Rest eines cyclischen, nicht-aromatischen, gegebenenfalls ein weiteres Heteroatom enthaltenden sekundären Amins mit 5 bis 7 Ringatomen darstellen, wird das entsprechende 1.4;3.6-Dianhydro-hexit-acylat – ebenfalls unter Konfigurationsumkehr – der Aminolyse mit sekundären cyclischen nicht-aromatischen Aminen unterworfen, beispielsweise mit Pyrrolidin, Piperidin, Hexamethylenimin, Morpholin, 4-Methylpiperazin usw., wobei die Reaktion vorteilhaft, wie oben beschrieben, unter erhöhtem Druck und erhöhter Temperatur im Stahlautoklaven durchgeführt wird. Die so dargestellten cyclischen Amino-desoxy-1.4;3.6-dianhydrohexit-Derivate werden anschließend ganz analog der zuvor beschriebenen Weise an ihrer freien Hydroxylgruppe mit Salpetersäure verestert.

Zur Herstellung derjenigen erfindungsgemäßen Verbindungen der allgemeinen Formel I, worin $R^1$ ein Wasserstoffatom oder eine Niedrigalkylgruppe und $R^2$ einen Acylrest einer aliphatischen oder einfach ungesättigten, gegebenenfalls methylsubstituierten Monocarbonsäure, einen Nicotinoyl- oder 2-O-Acetyl-salicoylrest bedeuten, setzt man das entsprechende, gegebenenfalls N-alkylsubstituierte Aminoisohexid-nitrat beispielsweise um mit Acetylchlorid, Propionylchlorid, Butyrylchlorid, n-Valeroylchlorid, iso-Valeroylchlorid, Caproylchlorid, Pivaloylchlorid, Nicotinsäurechlorid-Hydrochlorid oder O-Acetyl-salicylsäurechlorid.

Zur Herstellung derjenigen erfindungsgemäßen Verbindungen der allgemeinen Formel I, worin $R^1$ ein Wasserstoffatom oder eine Niedrigalkylgruppe und $R^2$ der Diphenylmethylrest, der Cinnamylrest oder ein $\omega$-(subst.)-Phenylalkylrest der allgemeinen Formel Ib ist, wird das entsprechende, gegebenenfalls N-alkylsubstituierte Amino-isohexid-nitrat umgesetzt mit Verbindungen wie z. B. Bromdiphenylmethan, Cinnamylbromid, Benzylchlorid, Phenyläthylbromid, Phenylpropylbromid, Phenylbutylbromid, Phenylpentylbromid, 3-(3.4-Dimethoxyphenyl)-propyl-1-methansulfonat, 4-(4-Methoxyphenyl)-butyl-1-methansulfonat, 4-(4-Chlorphenyl)-butyl-1-methansulfonat oder 3-(4-Chlorphenyl)-propyl-1-methansulfonat usw.

Zur Herstellung derjenigen erfindungsgemäßen Verbindungen der allgemeinen Formel I, worin $R^1$ ein Wasserstoffatom oder eine Niedrigalkylgruppe und $R^2$ eine $\omega$-Phenoxy-alkylgruppe ist, wird das entsprechende gegebenenfalls N-alkylsubstituierte Amino-isohexid-nitrat umgesetzt mit Verbindungen wie z. B. 2-Phenoxy-1-bromäthan, 3-Phenoxy-1-brompropan, 4-Phenoxy-1-brom-butan, 5-Phenoxy-1-brom-pentan, 6-Phenoxy-1-brom-hexan, 7-Phenoxy-1-brom-heptan, 8-Phenoxy-1-brom-octan.

Zur Herstellung derjenigen erfindungsgemäßen Verbindungen der allgemeinen Formel I, worin $R^1$ ein Wasserstoffatom und $R^2$ ein 2-Hydroxy-3(subst.)phenoxy-prop-1-yl der allgemeinen Formel Ia oder ein 2-Hydroxy-3-(subst.)-naphthyloxy-prop-1-yl-Rest ist, wird das entsprechende Amino-isohexid-nitrat in an sich bekannter Weise umgesetzt mit Verbindungen wie 1.2-Epoxy-3-(4-methoxyphenoxy)-propan, 1.2-Epoxy-3-(3-methoxyphenoxy)-propan, 1.2-Epoxy-3-(2-methoxyphenoxy)-propan, 1.2-Epoxy-3-(2-äthoxyphenoxy)-propan, 1.2-Epoxy-3-(2-allyloxyphenoxy)-propan, 1.2-Epoxy-3-(2-allylphenoxy)-propan, 1.2-Epoxy-3-(2-cyanophenoxy)-propan, 1.2-Epoxy-3-(3-tolyloxy)-propan, 1.2-Epoxy-3-(3-trifluormethylphenoxy)-propan, 4-(2.3-Epoxypropoxy)-phenylessigsäureamid, 1.2-Epoxy-3-(1-naphthyloxy)-propan, 5-(2.3-Epoxypropoxy)-1-tetralon, 5-(2.3-Epoxypropoxy)-tetralin.

Zur Herstellung derjenigen erfindungsgemäßen Verbindungen der allgemeinen Formel I, worin $R^1$ ein Wasserstoffatom oder eine Niedrigalkylgruppe und $R^2$ eine $\omega$-Phenoxyalkylgruppe der allgemeinen Formel Ic mit substituiertem Phenylrest oder eine $\omega$-Naphthyloxy-alkylgruppe ist, wird das entsprechende, gegebenenfalls N-niedrigalkylsubstituierte Amino-isohexid-nitrat umgesetzt mit Verbindungen wie 3-(3-Trifluormethylphenoxy)-1-brompropan, 3-(4-Tolyloxy)-1-brompropan, 3-(4-Fluorphenoxy)-1-brompropan, 3-(1-Naphthyloxy)-1-brompropan, 3-(4-Methoxyphenoxy)-1-brompropan, 3-(3-Methoxyphenoxy)-1-brompropan, 3-(2-Methoxyphenoxy)-1-brompropan, 3-(2.6-Dimethoxyphenoxy)-1-brompropan, 3-(3.5-Dimethoxyphenoxy)-1-brompropan, 3-(2.3-Dimethoxyphenoxy)-1-brompropan, 3-(4-Chlorphenoxy)-1-brompropan, 3-(2-Chlorphenoxy)-1-brompropan, 3-(3-Chlorphenoxy)-propyl-1-methansulfonat, 3-(3.4-Dichlorphenoxy)-propyl-1-methansulfonat, 3-(2.4-Dichlorphenoxy)-propyl-1-methansulfonat, 3-(4-Acetamidophenoxy)-1-brompropan, 3-(3-Dimethylaminophenoxy)-propyl-1-methansulfonat.

Zur Herstellung derjenigen erfindungsgemäßen Verbindungen der allgemeinen Formel I, worin $R^1$ und $R^2$ gemeinsam den Pyridoxylidenrest bedeuten, kondensiert man das entsprechende Amino-desoxy-1.4;3.6-dianhydro-hexit-nitrat mit Pyridoxal, und zwar vorteilhaft in einem geeigneten Lösungsmittel oder Lösungsmittelgemisch, z. B. Äthanol, Benzol, Toluol oder einer Mischung aus diesen Lösungsmitteln, so daß das entstehende Reaktionswasser leicht durch azeotrope Destillation entfernt und eine nahezu quantitative Umsetzung erreicht werden kann.

Wie sich aus der vorstehenden Beschreibung des erfindungsgemäßen Verfahrens zur Herstellung der erfindungsgemäßen Verbindungen der allgemeinen Formel I ergibt, muß man zur Herstellung des 5-Amino-5-desoxy-1.4;3.6-dianhydro-L-idit-2-nitrats sowie seiner N-mono- und N-disubstituierten Derivate der allgemeinen Formel V als Substrat der Ammonolyse bzw. Aminolyse stets das entsprechende 1.4;3.6-Dianhydro-D-glucit-5-acylat, beispielsweise das -5-methansulfonat oder -5-toluolsulfonat, einsetzen, während man zur Herstellung des 5-Amino-5-desoxy-1.4;3.6-dianhydro-D-glucit-2-nitrats sowie seiner N-mono- und N-disubstituierten Derivate der allgemeinen Formel VI als Substrat der Ammonolyse bzw. Aminolyse stets das entsprechende 1.4;3.6-Dianhydro-L-idit-5-acylat, beispielsweise das -5-methansulfonat oder -5-toluolsulfonat, einsetzen muß.

Ganz analog muß zur Herstellung des 2-Amino-2-desoxy-1.4;3.6-dianhydro-D-glucit-5-nitrats sowie seiner N-mono- und N-disubstituierten Derivate der allgemeinen Formel VII als Substrat der Ammonolyse bzw. Aminolyse stets das entsprechende 1.4;3.6-Dianhydro-D-mannit-2-acylat, beispielsweise das -2-methansulfonat oder -2-toluolsulfonat, eingesetzt werden, während zur Herstellung des 5-Amino-5-desoxy-1.4;3.6-dianhydro-D-mannit-2-nitrats sowie seiner N-mono- und N-disubstituierten Derivate der allgemeinen Formel VIII als Substrat der Ammonolyse bzw. Aminolyse stets das entsprechende 1.4;3.6-Dianhydro-D-glucit-2-acylat, beispielsweise das -2-methansulfonat oder -2-toluolsulfonat, eingesetzt werden muß, wonach in der zuvor beschriebenen Weise die Veresterung mit Salpetersäure und danach gegebenenfalls die gewünschte weitere Substitution des Aminstickstoffs durch Acylierung mit den entsprechenden Säurechloriden oder die weitere Kondensation mit den entsprechenden reaktiven Arylalkyl-, Aryloxyalkyl- oder Aryloxy-epoxypropyl-Derivaten oder mit Pyridoxal erfolgt.

Der bisher beschriebene erste erfindungsgemäße Weg zur Herstellung der erfindungsgemäßen Verbindungen, bei dem der entsprechende Isohexid mit einem Sulfonsäurechlorid, vorzugsweise mit Methansulfonsäurechlorid oder Toluolsulfonsäurechlorid, in den entsprechenden Monoacyl-1.4;3.6-dianhydro-hexit übergeführt wird; hat den Nachteil, daß bei der Acylierung nicht nur das entsprechende 5-O-Acyl-Derivat bzw. 2-O-Acyl-Derivat entsteht, sondern gleichzeitig auch das 2,5-Diacyl-Derivat, so daß bei den Isoidid- und Isomannid-Derivaten jeweils die Monoacyl-Verbindung vom Diacylat abgetrennt werden muß, während beim Isosorbid, bei dem zwei stereoisomere Monoacyl-Derivate neben dem Diacylat entstehen, das gewünschte Acylat aus dem Gemisch der drei Acyl-Derivate isoliert werden muß. Die Auftrennung des Acylatgemisches erfolgt entweder durch fraktionierte Kristallisation, fraktionierte Extraktion oder mit Hilfe anderer an sich bekannter Methoden.

Die mühsame und aufwendige Auftrennung des Acylat-Gemisches entfällt jedoch bei Anwendung des erfindungsgemäßen zweiten Syntheseweges zu den 5-Amino-isoidid-Derivaten dadurch, daß 1.4;3.6-Dianhydro-D-glucit mit einem Überschuß an Sulfonsäurechlorid, vorzugsweise Methansulfonylchlorid oder Toluolsulfonylchlorid, in Pyridin oder Chloroform/Triäthylamin quantitativ zum entsprechenden 1.4;3.6-Dianhydro-D-glucit-2.5-diacylat umgesetzt wird.

Das Diacylat wird dann unter entsprechenden Bedingungen, wie bei dem ersten Syntheseweg beschrieben, der Ammonolyse bzw. Aminolyse unterworfen, wobei infolge der bevorzugten Substitution der 5-endo-Acylgruppe unter Konfigurationsumkehr und infolge teilweiser Hydrolyse der 2-exo-Acylgruppe unter Beibehaltung der Konfiguration neben einer geringen, in der wäßrigen Phase verbleibenden Menge an 2.5-Diamino-2.5-didesoxy-1.4;3.6-dianhydro-D-glucit ein Gemisch aus 5-Amino-5-desoxy-1.4;3.6-dianhydro-L-idit und 5-Amino-5-desoxy-1.4;3.6-dianhydro-L-idit-2-methansulfonat im Verhältnis von etwa 1 : 4 entsteht.

Zur Vervollständigung der Hydrolyse der 2-exo-Acylgruppe wird dann dieses 1 : 4-Gemisch einer alkalischen oder sauren Hydrolyse unterworfen, und der entstehende 5-Amino-5-desoxy-1.4;3.6-dianhydro-L-idit wird anschließend, wie beim ersten Syntheseweg beschrieben, zum entsprechenden Mononitrat verestert und gegebenenfalls — ganz analog — durch Kondensation mit den entsprechenden Acylchloriden, $\omega$-(subst.)-Arylalkylhalogeniden oder -methansulfonaten, $\omega$-(subst.)-Aryloxyalkylhalogeniden oder -methansulfonaten, 1.2-Epoxy-3-(subst.)-aryloxypropanen oder mit Pyridoxal in die Verbindungen der allgemeinen Formel V übergeführt, worin $R^1$ ein Wasserstoffatom bedeutet und $R^2$ eine der angegebenen Bedeutungen besitzt oder in denen $R^1$ und $R^2$ zusammen den Pyridoxylidenrest darstellen.

Wie sich aus der Tatsache, daß die Ammonolyse des Isosorbid-2.5-diacylats überwiegend zum 5-Amino-isoidid-2-acylat führt, ergibt, ist die nucleophile Substitution der 2-exo-Acylatgruppe im Isosorbid-diacylat sterisch gehindert. Der Grad der sterischen Hinderung ist temperaturabhängig. Um aus dem entsprechenden Diacylat möglichst quantitativ das 5-Amino-2-acylat zu erhalten, arbeitet man deshalb erfindungsgemäß vorzugsweise bei einer Temperatur von 80 bis 120°C, da bei Temperaturen über 120°C auch die 2-exo-Acylgruppe, wenn auch in geringem Ausmaß, durch Ammoniak oder durch anstelle des Ammoniaks eingesetztes Mono- oder Dialkylamin angegriffen wird. Als Lösungsvermittler kann der wäßrigen Ammoniaklösung oder dem Mono- oder Dialkylamin ein Alkohol, vorzugsweise Äthanol, zugesetzt werden.

Zur Herstellung derjenigen erfindungsgemäßen Verbindungen der allgemeinen Formel V, worin $R^1$ und/oder $R^2$ eine Niedrigalkylgruppe mit 1 bis 4 C-Atomen bedeuten, wird das 1.4;3.6-Dianhydro-D-glucit-2.5-diacylat der Aminolyse mit primären oder sekundären Alkylaminen mit 1 bis 4 C-Atomen unterworfen, und zwar vorteilhaft unter erhöhtem Druck, vorzugsweise bei einem Druck von 2 bis 20 at, und erhöhter Temperatur, vorzugsweise bei 90 bis 180°C. Die Aminolyse wird ganz analog der oben beschriebenen Ammonolyse zweckmäßig in einem geschlossenen Stahlautoklaven bis zur quantitativen Umsetzung durchgeführt. Anschließend wird in der zuvor beschriebenen Weise die Hydrolyse durchgeführt, gefolgt von der Veresterung der 2-Hydroxygruppe mit Salpetersäure.

Die Aminolyse kann nicht nur mit Methylamin, Äthylamin, Propylamin, Butylamin, Dimethylamin, Diäthylamin, Dipropylamin, Dibutylamin usw. durchgeführt werden, sondern beispielsweise auch mit 1-Aminoadamantan sowie cyclischen, nicht-aromatischen, sekundären Aminen wie Pyrrolidin, Piperidin, Morpholin, 4-Methylpiperazin und ähnlichen heterocyclischen Stickstoffbasen und führt dann zu den erfindungsgemäßen Verbindungen der allgemeinen Formel V, worin $R^1$ und $R^2$ gemeinsam mit dem

Stickstoffatom, an das sie gebunden sind, den Rest eines cyclischen, nicht-aromatischen, gegebenenfalls ein weiteres Heteroatom enthaltenden sekundären Amins bedeuten.

Zur Herstellung derjenigen Verbindungen der allgemeinen Formel V, worin $R^1$ eine Niedrigalkylgruppe mit 1 bis 4 C-Atomen und $R^2$ einen Acylrest, einen $\omega$-(subst.)-Arylalkyl- oder -alkenylrest, einen $\omega$-(subst.)-Aryloxyalkylrest oder einen 2-Hydroxy-3-(subst.)-aryloxy-prop-1-yl-rest bedeuten, werden die bei der Aminolyse des Isosorbid-2.5-diacylats mit primären Aminen und nachfolgender alkalischer Hydrolyse und Veresterung mit Salpetersäure entstehenden 5-Niedrigalkylamino-5-desoxy-1.4;3.6-dianhydro-L-idit-2-nitrate ganz analog zu der zuvor beschriebenen Weise kondensiert, und zwar mit den entsprechenden Acylhalogeniden, $\omega$-(subst.)-Arylalkyl- oder -alkenyl-halogeniden oder -methansulfonaten, $\omega$-(subst.)-Aryloxyalkyl-halogeniden oder -methansulfonaten oder mit den 1.2-Epoxy-3(subst.)-arylpropanen.

Bei einem weiteren erfindungsgemäßen Weg zur Herstellung der erfindungsgemäßen Verbindungen der allgemeinen Formel VI macht man sich die überraschend aufgefundene Tatsache zunutze, daß 1.4;3.6-Dianhydro-D-glucit-2.5-diacylate (Dimesylat oder Ditosylat) von Natriumbenzoat in einem geeigneten Lösungsmittel, vorzugsweise einem dipolaren aprotischen Lösungsmittel, beispielsweise in wasserfreiem Dimethylformamid, Dimethylsulfoxid oder Diäthern des Äthylenglykols, bei Temperaturen von 100 bis 180°C, vorzugsweise 120 bis 150°C, selektiv am $C^5$-Atom angegriffen werden, so daß unter Konfigurationsumkehr in hoher Ausbeute 1.4;3.6-Dianhydro-L-idit-2-methansulfonat-5-benzoat bzw. 1.4;3.6-Dianhydro-L-idit-2-toluolsulfonat-5-benzoat entsteht. Dieses Produkt wird nun wieder der Ammonolyse mit 25%iger Ammoniaklösung oder der Aminolyse unter erhöhtem Druck und erhöhter Temperatur unterworfen, wobei der Benzoesäureester nicht substituiert, sondern hydrolytisch abgespalten wird, und zwar unter Beibehaltung der Konfiguration am $C^5$-Atom, während der Acylatrest am $C^2$-Atom unter Konfigurationsumkehr durch die Amino- bzw. Alkylaminogruppe zum entsprechenden 5-Amino-5-desoxy-1.4;3.6-dianhydro-D-glucit bzw. 5-Alkylamino-5-desoxy-1.4;3.6-dianhydro-D-glucit substituiert wird.

Da das zuletzt genannte elegante Verfahren mit einer zweimaligen selektiven Konfigurationsumkehr verbunden ist, ist die Konfiguration des Endproduktes bezüglich seiner Substituenten identisch mit der Konfiguration der Ausgangsverbindung; aus dem Isosorbid-disulfonat entsteht wieder ein Isosorbid-Derivat, nämlich 5-Amino- bzw. 5-Alkylamino-5-desoxy-1.4;3.6-dianhydro-D-glucit, das dann, wie zuvor beschrieben, in die gewünschten Verbindungen der allgemeinen Formel VI weiter verarbeitet werden kann.

Um die erfindungsgemäßen Verbindungen in ihre physiologisch unbedenklichen Salze überzuführen, können anorganische Säuren und Mineralsäuren wie Halogenwasserstoffsäuren und Phosphorsäuren sowie organische Säuren wie Carbon- und Sulfonsäuren, beispielsweise Malon-, Bernstein-, Milch-, Wein-, Äpfel-, Benzoe-, Salicyl-, Citronen-, Ascorbin-, Nicotin- oder p-Toluolsulfonsäure verwendet werden. Die freien Basen können aus den Säureadditionssalzen wieder durch Behandlung mit starken Basen, beispielsweise Natrium- oder Kaliumhydroxid, freigesetzt werden.

Gegenstand der Erfindung sind ferner pharmazeutische Zubereitungen, die neben üblichen Träger- und Zusatzstoffen mindestens eine der erfindungsgemäßen Verbindungen oder eines ihrer physiologisch unbedenklichen Salze enthalten. Diese Zubereitungen können als Arzneimittel in der Human- und Veterinärmedizin verwendet werden. Übliche Trägerstoffe sind beispielsweise Wasser, pflanzliche Öle, Polyäthylenglykole, Glycerinester, Gelatine, Kohlenhydrate wie Laktose oder Stärke, Magnesiumstearat, Talk oder Vaseline. Übliche Zusatzstoffe sind beispielsweise Konservierungs-, Stabilisierungs-, Gleit-, Netzmittel, Emulgatoren, physiologisch unbedenkliche Salze, Puffersubstanzen, Farb-, Geschmacks- und Aromastoffe. Die Auswahl der Träger- und Zusatzstoffe hängt davon ab, ob die erfindungsgemäßen Verbindungen enteral, parenteral oder topikal appliziert werden sollen.

Die erfindungsgemäßen Verbindungen können auch im Gemisch mit anderen Wirkstoffen, beispielsweise Vitaminen oder bekannten, im Handel befindlichen Herz-Kreislauf-Mitteln, insbesondere auch mit β-Rezeptorenblockern, verabreicht werden.

Beispiel für eine pharmazeutische Zubereitung

Für die Herstellung von Tabletten von je 100 mg Einzelgewicht, die je 5 mg Wirkstoff enthalten, benötigt man

I. 5 g 5-(4-Phenylbutylamino)-5-desoxy-1.4;3.6-dianhydro-L-idit-2-nitrat-Hydrochlorid
II. 54 g mikrokristalline Cellulose
III. 20 g Milchzucker
IV. 20 g Maisstärke
V. 0,5 g kolloidale Kieselsäure
VI. 0,5 g Magnesiumstearat.

Die Substanzen I bis IV werden trocken 10 Minuten lang gemischt, anschließend wird das Gemisch der Substanzen V und VI zugegeben, man mischt weitere 10 Minuten und verpreßt das so erhaltene

Pulver auf einer Tablettiermaschine zu Tabletten von 100 mg Einzelgewicht.

Jede der in den nachfolgenden Beispielen genannten erfindungsgemäßen Verbindungen und Zwischenprodukte stellt ein zur Herstellung pharmazeutischer Zubereitungen besonders geeignetes Mittel dar.

Die in den Beispielen enthaltenen Abkürzungen haben folgende Bedeutungen:

| | | |
|---|---|---|
| Schmp. | = | Schmelzpunkt (unkorrigiert) |
| (Z) | = | Zersetzung |
| d | = | Dichte |
| $[\alpha]_D^{25}$ | = | optische Drehung bei 25°C, Natrium-D-Linie. |

Hinter den optischen Drehwerten sind die Konzentrationen der gemessenen Lösungen angegeben, wobei »c2« beispielsweise eine Konzentration von 2 g/100 ml Lösung bedeutet; das Lösungsmittel ist jeweils gesondert angegeben. Alle Temperaturen sind in Grad Celsius angegeben.

## Beispiel Nr. 1

### 5-Amino-5-desoxy-1.4;3.6-dianhydro-L-idit-2-nitrat

a) 1.4;3.6-Dianhydro-D-glucit-2-methansulfonat, -5-methansulfonat und -2.5-demethansulfonat: Zur Lösung von 4.82 kg (33 Mol) 1.4;3.6-Dianhydro-D-glucit in 24 Liter Pyridin tropft man unter Feuchtigkeitsausschluß, Rühren und Kühlen auf −15° bis −20° innerhalb mehrerer Stunden 3.1 Liter (40 Mol) Methansulfonsäurechlorid. Anschließend rührt man 15 Stunden ohne Kühlung weiter. Man destilliert im Vak. das Pyridin ab, gibt zum öligen Rückstand 15 Liter Wasser, kocht auf und läßt abkühlen. Absaugen, Waschen mit 4 Litern Wasser und Trocknen des kristallinen Niederschlags ergibt 2.22 kg (7.34 Mol) 1.4;3.6-Dianhydro-D-glucit-2.5-dimethansulfonat. Das Filtrat wird unter Rühren und Wasserkühlung mit ca. 1.5 kg Natriumhydroxid neutralisiert und bei ca. 70° im Vakuum bis zur Trockne eingedampft. Der Trockenrückstand wird mit insgesamt 30 Litern Chloroform kontinuierlich heiß extrahiert und der Extrakt heiß filtriert. Man läßt den Extrakt 15 Stunden bei 20° stehen, saugt den kristallinen Niederschlag ab, wäscht ihn zweimal mit je 2 Litern Chloroform, trocknet und erhält 2.3 kg (10.26 Mol) 1.4;3.6-Dianhydro-D-glucit-5-methansulfonat. Die vereinigten Filtrate werden im Vakuum eingedampft und der Rückstand heiß in 22 Litern Aethanol gelöst. Man läßt 15 Stunden bei 20° stehen, saugt den kristallinen Niederschlag ab, wäscht ihn zweimal mit je 3 Litern Aethanol, trocknet und erhält 0.65 kg (2.90 Mol) 1.4;3.6-Dianhydro-D-glucit-2-methansulfonat. Eindampfen der Filtrate ergibt 2.21 kg (9.85 Mol) eines Gemisches der beiden isomeren Mono-methansulfonate, das je nach Bedarf durch Wiederholung der abwechselnden Kristallisationen aus Chloroform und Aethanol weiter aufgetrennt werden kann, bzw. durch Veresterung mit Methansulfonsäurechlorid in Pyridin vollständig in 1.4;3.6-Dianhydro-D-glucit-2.5-dimethansulfonat übergeführt wird.

Analytische Mengen der Methansulfonate ergeben nach Umkristallisation korrekte Elementaranalysen und die in Tabelle 1 aufgeführten Schmelzpunkte und optischen Drehungen:

Tabelle 1

| 1.4;3.6-Dianhydro-D-glucit- | Umkristallisiert aus | Schmp. [°C] | $[\alpha]_D^{25}$ |
|---|---|---|---|
| -2-methansulfonat | Chloroform | 135–138.5 | 62.5 (c 2; Aceton) |
| -5-methansulfonat | Chloroform | 123–124 | 75.9 (c 2; Methanol) |
| 2.5-dimethansulfonat | Äthanol/Aceton | 127–128 | 74 (c 2; Aceton) |

Anmerkung:

Setzt man 1.4;3.6-Dianhydro-D-glucit mit der 2- bis 2.5fach molaren Menge Methansulfonsäurechlorid unter den gleichen Reaktionsbedingungen um, erhält man das 1.4;3.6-Dianhydro-D-glucit-2.5-dimethansulfonat in nahezu quantitativer Ausbeute.

b) 5-Amino-5-desoxy-1.4;3.6-dianhydro-L-idit:

Dieses Zwischenprodukt kann auf 2 Wegen erhalten werden:

Verfahren 1

Herstellung durch Ammonolyse von 1.4;3.6-Dianhydro-D-glucit-5-methansulfonat. Das Gemisch aus 448 g (2 Mol) 1.4;3.6-Dianhydro-D-glucit-5-methansulfonat und 1.5 Liter 25%igem wäßrigen Ammoniak (20 Mol) wird im geschlossenen Stahlautoklaven 24 Stunden bei 130° gerührt. Danach ist die Umsetzung quantitativ. Man dampft unter vermindertem Druck ein und trocknet azeotrop durch aufeinanderfolgende Zugabe und erneutes Eindampfen von je 1 Liter Äthanol und Chloroform nach. Der ölige Rückstand wird unter Erwärmen in 500 ml Äthanol gelöst und mit Isopropanol auf 2 Liter verdünnt. Beim Erkalten kristallisieren 311 g (1.3 Mol) 5-Amino-5-desoxy-1.4;3.6-dianhydro-L-idit als methansulfonsaures Salz aus. Weitere 100 g (0.4 Mol) kristallines Reinprodukt fallen nach Behandeln der Mutterlauge mit 30 g Aktivkohle und Konzentrieren des Filtrats aus. Zur Analyse kristallisiert man aus Äthanol/Chloroform um.
Schmp. 151—4°; $[\alpha]_D^{25}$ 27.6 (c 1; Wasser)

Elementaranalyse: $C_6H_{11}NO_3 \times CH_3SO_3H$ (241.27)
Ber.: C (34.83), H (6.27), N (5.81)
Gef.: C (34.71), H (6.45), N (5.36)

Ein kleiner Teil des Produkts wird in die freie Base übergeführt und aus Chloroform/Äther umkristallisiert.
Schmp. 103—104°; $[\alpha]_D^{25}$ 31.6 (c 2; Wasser)

Verfahren 2

Ammonolyse von 1.4;3.6-Dianhydro-D-glucit-2.5-dimethansulfonat gefolgt von alkalischer Hydrolyse des erhaltenen 5-Amino-5-desoxy-1.4;3.6-dianhydro-L-idit-2-methansulfonats.

Das Gemisch aus 302 g (1 Mol) 1.4;3.6-Dianhydro-D-glucit-2.5-dimethansulfonat, 750 ml 25%igem wäßrigen Ammoniak (10 Mol) und 750 ml Äthanol wird im verschlossenen Stahlautoklaven 4 Tage bei 100° gerührt. Nach dem Abkühlen versetzt man mit 1 Liter Wasser und saugt vom auskristallisierten nicht umgesetzten Dimethansulfonat (106 g = 0.35 Mol) ab. Das Filtrat wird zur Entfernung von Ammoniak mit 104 g (1.3 Mol) Natriumhydrogencarbonat versetzt und unter vermindertem Druck eingedampft. Man löst in 5 Liter Wasser und extrahiert mit 500 ml Chloroform Eliminierungsprodukte heraus. Die wäßrige Phase wird 48 Stunden lang im Rotationsperforator (Normag) kontinuierlich mit Chloroform extrahiert. In der Wasserphase verbleibt als Nebenprodukt entstandener 2.5-Diamino-2.5-didesoxy-1.4;3.6-dianhydro-D-glucit. Der Chloroformextrakt ergibt nach dem Trocknen über wasserfreiem Natriumsulfat, Filtrieren und Eindampfen 105 g (ca. 0.55 Mol) eines 1 : 4-Gemisches aus 5-Amino-5-desoxy-1.4;3.6-dianhydro-L-idit und 5-Amino-5-desoxy-1.4;3.6-dianhydro-L-idit-2-methansulfonat.

Zur Charakterisierung des letzteren Produkts wird ein kleiner Teil des Gemisches in Chloroform gelöst, 2mal mit Wasser gewaschen, die Chloroformphase eingedampft, in das methansulfonsaure Salz übergeführt und 2mal aus Äthanol umkristallisiert.
Schmp. 213—5°; $[\alpha]_D^{25}$ 39.0 (c 0.50; Wasser)

Elementaranalyse: $C_7H_{13}NO_5S \times CH_3SO_3H$ (319.37)
Ber.: C (30.09), H (5.37), N (4.39), S (20.08)
Gef.: C (30.13), H (5.49), N (4.25), S (20.6)

Das oben erhaltene Gemisch wird zu einer Lösung von 60 g (1.5 Mol) Natriumhydroxid in 1.5 Litern Wasser gegeben und 24 Stunden unter Rückfluß gekocht. Nach dem Abkühlen stellt man durch Zugabe von konz. Salzsäure auf pH = 10 ein, filtriert und dampft unter vermindertem Druck ein, trocknet aceotrop mit n-Butanol nach, erwärmt den Rückstand mit 500 ml n-Butanol und filtriert von anorganischen Salzen ab. Die butanolische Lösung wird eingedampft, der Rückstand in 200 ml Isopropanol gelöst und mit 34 g (0.35 Mol) Methansulfonsäure versetzt. Es kristallisieren 80 g (0.33 Mol) 5-Amino-5-desoxy-1.4;3.6-dianhydro-L-idit in Form des methansulfonsauren Salzes aus. Schmp. 150—2°. Ausbeute bezogen auf umgesetztes 1.4;3.6-Dianhydro-D-glucit-2.5-dimethansulfonat: 50%.

c) 5-Amino-5-desoxy-1.4;3.6-dianhydro-L-idit-2-nitrat

241 g (1 Mol) 5-Amino-5-desoxy-1.4;3.6-dianhydro-L-idit-Hydrogenmethansulfonat (bzw. 145 g der freien Base) werden in 50 ml Wasser gelöst und unter Kühlung tropfenweise mit 30 ml konz. Schwefelsäure (d = 1.84; 0.54 Mol) versetzt (Lösung A).

Zu 200 ml 96%iger Salpetersäure (d = 1.5; 4.5 Mol) tropft man unter Rühren und Kühlen auf −15° die Lösung von 14 g (0.23 Mol) Harnstoff in 300 ml konz. Schwefelsäure (d = 1.84; 5.4 Mol). Anschließend tropft man bei −15° die Lösung A innerhalb 3—4 Stdn. zu und rührt noch 2 Stdn. bei dieser Temperatur. Die Reaktionsmischung wird langsam in 1.5 Liter Wasser eingerührt. Unter Kühlen neutralisiert man durch langsame Zugabe einer Lösung von 630 g (15.75 Mol) Natriumhydroxid (bzw. 590 g NaOH, falls zuvor die freie Base eingesetzt wurde) in 2 Litern Wasser und filtriert. Das Filtrat wird 16 Stdn. im 5-Liter-Rotationsperforator kontinuierlich mit Chloroform extrahiert. Aus dem Chloroformextrakt erhält man nach Trocknen über wasserfreiem Natriumsulfat, Filtrieren und Eindampfen unter vermindertem Druck 158 g (0.83 Mol) 5-Amino-5-desoxy-1.4;3.6-dianhydro-L-idit-2-nitrat als langsam kristallisierendes Öl, das in Isopropanol gelöst und mit der äquimolaren Menge Chlorwasserstoff versetzt wird. Man erhitzt bis zur vollständigen Auflösung, läßt abkühlen, saugt das auskristallisierte Produkt ab, wäscht mit wenig Isopropanol nach, konzentriert die Mutterlauge auf ein kleines Volumen und saugt erneut ab. Man erhält 170 g (0.75 Mol) kristallines 5-Amino-5-desoxy-1.4;3.6-dianhydro-L-idit-2-nitrat-Hydrochlorid, das bei 201—208° unter Zersetzung schmilzt.
$[\alpha]_D^{25}$ 54.1 (c 2; Wasser)

Elementaranalyse: $C_6H_{10}N_2O_5 \times HCl$ (226.62)
Ber.: C (31.80), H (4.89), N (12.36), Cl (15.6)
Gef.: C (31.78), H (4.92), N (12.20), Cl (15.6)

Beispiel Nr. 2

5-Amino-5-desoxy-1.4;3.6-dianhydro-D-glucit-2-nitrat

a) 5-Amino-5-desoxy-1.4;3.6-dianhydro-D-glucit:

Dieses Ausgangsprodukt kann auf zwei Wegen erhalten werden.

Verfahren 1

Herstellung und Ammonolyse von 1.4;3.6-Dianhydro-L-idit-2-methansulfonat

Die Lösung von 73 g (480 mmol) 96%igem 1.4;3.6-Dianhydro-L-idit in 500 ml wasserfreiem Pyridin wird unter Feuchtigkeitsausschluß, Rühren und Kühlen auf −20° tropfenweise mit 52 ml (660 mmol) 98%igem Methansulfonylchlorid versetzt und 15 Stdn. bei −20° nachgerührt. Unter vermindertem Druck destilliert man das Pyridin soweit wie möglich ab und erwärmt den Rückstand nach Zugabe von 500 ml heißem Wasser bis zur Lösung. Beim Abkühlen kristallisieren 47.6 g (157 mmol) 1.4;3.6-Dianhydro-L-idit-2.5-dimethansulfonat aus, die abgesaugt und zweimal mit je 100 ml Wasser nachgewaschen werden. Die vereinigten Filtrate werden durch Zugabe von Natriumhydrogencarbonat neutralisiert (pH = 7), unter vermindertem Druck eingedampft und getrocknet. Der gepulverte Trockenrückstand wird zweimal mit je 400 ml Chloroform ausgekocht und noch heiß filtriert. Nach dem Abkühlen des Filtrats kristallisiert 1.4;3.6-Dianhydro-L-idit-2-methansulfonat aus. Die Mutterlauge liefert nach Konzentration weiteres Monomethansulfonat. Insgesamt erhält man 51.5 g (213 mmol) 1.4;3.6-Dianhydro-L-idit-2-methansulfonat. Zur Analyse wird eine kleine Menge aus Methanol umkristallisiert. Schmp. 124—5°; $[\alpha]_D^{25}$ 33.7 (c 1.0; Aceton).

Elementaranalyse: $C_7H_{12}O_6S$ (224.24)
Ber.: C (37.50), H (5.40), S (14.30)
Gef.: C (37.58), H (6.53), S (14.0)

33.6 g (150 mmol) des so erhaltenen 1.4;3.6-Dianhydro-L-idit-2-methansulfonats werden zusammen mit einer Lösung von 17 g (1 Mol) Ammoniak in 250 ml n-Butanol im geschlossenen Stahlautoklaven 3 Tage auf 170° erhitzt. Nach dem Abkühlen saugt man auskristallisiertes Ammoniummethansulfonat ab und wäscht mit 100 ml n-Butanol nach. Das Filtrat wird zweimal mit je 200 ml Wasser extrahiert. Die vereinigten wäßrigen Extrakte werden mit 200 ml Chloroform gewaschen, zur Trockne eingedampft und aceotrop mit Butanol nachgetrocknet. Der Trockenrückstand wird unter Zusatz von 10 g wasserfreiem Natriumsulfat mit 50 ml n-Butanol aufgekocht, heiß filtriert und das Filtrat eingedampft. Das so erhaltene ölige Rohprodukt wird in 50 ml Chloroform aufgenommen, filtriert und eingedampft.

Man erhält 14 g (96 mmol) langsam erstarrenden 5-Amino-5-desoxy-1.4;3.6-dianhydro-D-glucit. Zur Charakterisierung überführt man einen kleinen Anteil des Produkts in das Hydrochlorid und kristallisiert aus Isopropanol um.
Zersetzungspunkt: 240°; $[\alpha]_D^{25}$ 39.1 (c 1.0; Wasser)

Elementaranalyse: $C_6H_{11}NO_3 \times HCl$ (181.63)
Ber.: C (39.68), H (6.66), N (7.71), Cl (19.52)
Gef.: C (39.85), H (6.89), N (7.66), Cl (19.3)

## Verfahren 2

### Herstellung und selektive Ammonolyse von 1.4;3.6-Dianhydro-L-idit-2-methansulfonat-5-benzoat

Die Mischung aus 604 g (2 Mol) 1.4;3.6-Dianhydro-D-glucit-2.5-dimethansulfonat, 317 g (2.2 Mol) Natriumbenzoat und 8 Liter wasserfreiem Dimethylformamid wird 2 Tage bei 145° im Stahlautoklaven unter Stickstoffschutzatmosphäre gerührt. Unter vermindertem Druck destilliert man das Dimethylformamid ab, nimmt den Rückstand in 5 Litern Chloroform auf, extrahiert nacheinander mit je 2 Litern 1 molarer Natronlauge und Wasser, trocknet die Chloroformphase über wasserfreiem Natriumsulfat, filtriert und konzentriert auf 1500 ml Volumen. Das beim Stehenlassen kristallisierende Rohprodukt wird abgesaugt, unter Erwärmen in 500 ml Aceton gelöst und die heiße Lösung in 1000 ml Äthanol gegossen. Beim Abkühlen kristallisieren 273 g (0.83 Mol) 1.4;3.6-Dianhydro-L-idit-2-methansulfonat-5-benzoat aus. Die Mutterlauge ergibt nach Eindampfen und Umkristallisation weitere 120 g (0.37 Mol) schwach durch Ausgangssubstanz verunreinigtes Produkt. Die analytische Probe hat nach Umkristallisation aus Äthanol den Schmp. 117° und $[\alpha]_D^{25}$ 76.6 (c 2; Chloroform).

Elementaranalyse: $C_{14}H_{16}O_7S$ (328.35)
Ber.: C (51.21), H (4.91), S (9.76)
Gef.: C (51.60), H (5.05), S (9.6)

328 g (1 Mol) des so erhaltenen 1.4;3.6-Dianhydro-L-idit-2-methansulfonat-5-benzoats werden zusammen mit 1 Liter Äthanol und 1.5 Liter 25%igem wäßrigen Ammoniak 1 Tag bei 130° im geschlossenen Stahlautoklaven gerührt. Man dampft unter vermindertem Druck ein, löst den Rückstand in 1 Liter Wasser, stellt durch Zugabe von konz. Salzsäure auf pH = 1 ein und saugt den Niederschlag – bestehend aus Benzoesäure und Benzamid – ab. Das Filtrat wird nach zweimaligem Waschen mit je 500 ml Chloroform durch Zugabe von Natriumhydrogencarbonat auf pH = 8 gebracht, erneut eingedampft und der Rückstand mit 2 Litern Äthanol extrahiert. Der Äthanol-Extrakt wird nach dem Eindampfen mit 2 Litern Chloroform extrahiert, der Chloroformextrakt mit 60 g Aktivkohle aufgekocht, filtriert und eingedampft. Die so erhaltenen 105 g Rohprodukt liefern nach fraktionierter Destillation bei 0.2 Torr und 136–142° Übergangstemperatur 86.3 g (0.59 Mol) 5-Amino-5-desoxy-1.4;3.6-dianhydro-D-glucit.

### a) 5-Amino-5-desoxy-1.4;3.6-dianhydro-D-glucit-2-nitrat

55 g (0.38 Mol) des nach Verfahren 1 oder 2 erhaltenen 5-Amino-5-desoxy-1.4;3.6-dianhydro-D-glucits werden durch Vermischen mit 3.5 ml Chloroform verflüssigt und diese Lösung tropfenweise unter Rühren einer auf –15° gekühlten Lösung von 14 g (0.23 Mol) Harnstoff in 202 ml (4.6 Mol) 96%iger Salpetersäure (d = 1.5) zugefügt. Man rührt noch 15 Stdn. bei –15° nach, verdünnt mit 750 ml Wasser und neutralisiert unter Kühlen mit einer Lösung von 168 g (4.2 Mol) Natriumhydroxid in 1.5 Litern Wasser. Mit Natriumhydrogencarbonat stellt man auf pH = 8 ein, filtriert und extrahiert die wäßrige Lösung 16 Stdn. lang im 5-l-Rotationsperforator (Normag) kontinuierlich mit Chloroform. Der Chloroformextrakt wird nach dem Trocknen über wasserfreiem Natriumsulfat und Filtrieren unter vermindertem Druck eingedampft. Der Rückstand wird in 200 ml Dichlormethan gelöst, durch Filtration von anorganischen Verunreinigungen befreit und erneut unter reduziertem Druck eingedampft. Man erhält 53 g (0.28 Mol) 5-Amino-5-desoxy-1.4;3.6-dianhydro-D-glucit-2-nitrat als schwach gelbliches Öl. Zur Charakterisierung überführt man einen Teil in das Hydrochlorid und kristallisiert aus Äthanol/Chloroform/Petroläther um.
Schmp. 170–1° (Z); $[\alpha]_D^{25}$ 50.7 (c 0.53; Wasser)

Elementaranalyse: $C_6H_{10}N_2O_5 \times HCl$ (226.62)
Ber.: C (31.80), H (4.89), N (12.36), Cl (15.6)
Gef.: C (32.00), H (5.10), N (12.14), Cl (15.6)

Beispiel Nr. 3

2-Amino-2-desoxy-1.4;3.6-dianhydro-D-glucit-5-nitrat

a) 1.4;3.6-Dianhydro-D-mannit-2-methansulfonat

Der Lösung von 877 g (6 Mol) 1.4;3.6-Dianhydro-D-mannit in 6 Litern Pyridin tropft man unter Rühren und Feuchtigkeitsausschluß sowie Kühlen auf −15° innerhalb 6 Stdn. 525 ml (6.6 Mol) Methansulfonylchlorid zu, rührt weitere 3 Tage bei −15° und destilliert dann unter vermindertem Druck das Pyridin ab. Beim Versetzen des öligen Rückstands mit 2.7 Litern Wasser kristallisiert reines 1.4;3.6-Dianhydro-D-mannit-2.5-dimethansulfonat aus, das abgetrennt und 2mal mit je 700 ml Wasser gewaschen wird. Die vereinigten Filtrate werden mit einer Lösung von 264 g (6.6 Mol) Natriumhydroxid in 2.5 Litern Wasser versetzt, durch Zugabe von Natriumhydrogencarbonat auf pH = 7 eingestellt, unter reduziertem Druck eingedampft und aceotrop mit Chloroform getrocknet. Der Rückstand wird zweimal mit je 2.5 Litern Chloroform heiß extrahiert und filtriert. Die vereinigten Chloroformextrakte werden 5mal mit je 1 Liter Wasser extrahiert. Beim Konzentrieren der Wasserphasen kristallisiert das 1.4;3.6-Dianhydro-D-mannit-2-methansulfonat aus. Die nach dem Absaugen verbleibende Mutterlauge ergibt nach dem Eindampfen und Umkristallisieren aus Äthanol weiteres Produkt. Restliches Produkt wird durch Eindampfen der äthanolischen Mutterlauge, Lösen des Rückstandes in Wasser und kontinuierliche Extraktion der wäßrigen Lösung mit Chloroform im Rotationsperforator gewonnen. In der Wasserphase verbleibt nicht umgesetzter 1.4;3.6-Dianhydro-D-mannit. Insgesamt erhält man 396 g (1.77 Mol) 1.4;3.6-Dianhydro-D-mannit-2-methansulfonat (neben 465 g = 1.54 Mol des Dimethansulfonats). Die analytische Probe hat nach Umkristallisation aus Chloroform den Schmp. 111–112° und $[\alpha]_D^{25}$ 118 (c 1.0; Aceton).

Elementaranalyse: $C_7H_{12}O_6S$ (224.24)
Ber.: C (37.50), H (5.40), S (14.30)
Gef.: C (37.41), H (5.59), S (13.7)

b) 2-Amino-2-desoxy-1.4;3.6-dianhydro-D-glucit

Die Mischung aus 224 g (1 Mol) des zuvor erhaltenen 1.4;3.6-Dianhydro-D-mannit-2-methansulfonats und 1 Liter 25%igem wäßrigen Ammoniak wird 24 Stdn. bei 120° im geschlossenen Stahlautoklaven gerührt. Nach dem Abkühlen gibt man 84 g (1 Mol) Natriumhydrogencarbonat zu, dampft unter reduziertem Druck ein und kocht den Rückstand mit 2 Litern n-Butan aus. Der eingedampfte Butanol-Extrakt wird in 1 Liter Chloroform aufgenommen, restliches Natriummethansulfonat abfiltriert und das Filtrat eingedampft. Man erhält 130 g (0.9 Mol) 2-Amino-2-desoxy-1.4;3.6-dianhydro-D-glucit als schwach gelbliches Öl. Zur Charakterisierung überführt man einen kleinen Teil in das Hydrochlorid und kristallisiert aus Isopropanol/Methanol/Chloroform um.
Schmp. 230° (Z); $[\alpha]_D^{25}$ 52.1 (c 1.0; Wasser)
Elementaranalyse: $C_6H_{11}NO_3 \times HCl$ (181.62)
Ber.: C (39.68), H (6.66), N (7.71), Cl (19.52)
Gef.: C (39.59), H (6.89), N (7.52), Cl (19.3)

c) 2-Amino-2-desoxy-1.4;3.6-dianhydro-D-glucit-5-nitrat

Unter Rühren und Kühlen auf −15° tropft man zu 200 ml (4.5 Mol) 96%iger Salpetersäure (d = 1.5) zunächst die Lösung von 14 g (0.23 Mol) Harnstoff in 300 ml (5.4 Mol) Schwefelsäure (d = 1.84) und anschließend innerhalb 4 Stdn. die Lösung von 145 g (1 Mol) 2-Amino-2-desoxy-1.4;3.6-dianhydro-D-glucit in 50 ml Wasser und rührt 2 Stdn. bei −15° nach. Man rührt dann in 1.5 Liter Wasser ein, stellt durch allmähliche Zugabe von 570 g (14.3 Mol) Natriumhydroxid – gelöst in 2 Litern Wasser – unter Kühlen auf pH 8 bis 9 ein, filtriert auskristallisiertes Natriumsulfat ab, wäscht mit 500 ml Chloroform lipophile Nebenprodukte heraus und extrahiert 16 Stdn. lang im Rotationsperforator kontinuierlich mit Chloroform. Der Chloroformextrakt ergibt nach dem Trocknen über wasserfreiem Natriumsulfat, Filtrieren und Eindampfen unter vermindertem Druck 152 g (0.8 Mol) 2-Amino-2-desoxy-1.4;3.6-dianhydro-D-glucit-5-nitrat als langsam erstarrendes Öl. Zur Charakterisierung wird ein Teil in das Hydrochlorid übergeführt und aus Isopropanol/Äthanol umkristallisiert.
Schmp.: 181–2° (Z); $[\alpha]_D^{25}$ 130 (c 0.52; Wasser)

Elementaranalyse: $C_6H_{10}N_2O_5 \times HCl$ (226.62)
Ber.: C (31.80), H (4.89), N (12.36), Cl (15.6)
Gef.: C (31.95), H (4.90), N (12.18), Cl (15.9)

Beispiel Nr. 4

5-Amino-5-desoxy-1.4;3.6-dianhydro-D-mannit-2-nitrat

a) 2-Amino-2-desoxy-1.4;3.6-dianhydro-D-mannit

Das Gemisch aus 448 g (2 Mol) 1.4;3.6-Dianhydro-D-glucit-2-methansulfonat (Herstellung siehe Beispiel 1a) und 1500 ml 25%igem wäßrigen Ammoniak wird 1 Tag bei 130° im geschlossenen Stahlautoklaven gerührt. Nach dem Abkühlen, gibt man 30 g Aktivkohle zu, filtriert und extrahiert mit 1 Liter Chloroform den als Nebenprodukt gebildeten 1.4;2.5;3.6-Trianhydro-D-mannit [nach Eindampfen der Chloroformphase und Umkristallisation aus Äther/Petroläther insgesamt 104 g (0.81 Mol)]. Die Wasserphase wird nach dem Eindampfen unter reduziertem Druck und aceotropem Trocknen mit Äthanol und Chloroform in der Siedehitze mit 2 Litern Isopropanol extrahiert. Beim Einengen des Isopropanolextrakts auf 0.5 Liter auskristallisierendes Ammoniummethansulfat wird abfiltriert, das Filtrat mit verdünnter Natronlauge neutralisiert, eingedampft und mit 1 Liter n-Butanol heiß extrahiert. Der Butanolextrakt wird eingedampft und der Rückstand mit 1 Liter Chloroform extrahiert. Eindampfen des filtrierten Chloroformextrakts ergibt 60 g (0.41 Mol) ölige Rohbase, die in 100 ml Essigsäure gelöst und tropfenweise mit einer Lösung von 15 ml 96%iger Salpetersäure (d = 1.5) in 75 ml Essigsäure versetzt wird. Das auskristallisierende Hydrogennitrat wird abgesaugt und aus Isopropanol/Äthanol umkristallisiert. Man erhält 32 g (154 mmol) 2-Amino-2-desoxy-1.4;3.6-dianhydro-D-mannit-Hydrogennitrat.
Schmp. 192–3° (Z); $[\alpha]_D^{25}$ 63.4 (c 0.51; Wasser)

Elementaranalyse: $C_6H_{11}NO_3 \times HNO_3$ (208.18)
Ber.: C (34.62), H (5.81), N (13.45)
Gef.: C (34.52), H (5.97), N (13.53)

Ein kleiner Teil wird in das Hydrochlorid übergeführt und aus Äthanol umkristallisiert.
Schmp. 263–8° (Z); $[\alpha]_D^{25}$ 77.8 (c 1; Wasser)

Elementaranalyse: $C_6H_{11}NO_3 \times HCl$ (181.63)
Ber.: C (39.68), H (6.66), N (7.71), Cl (19.52)
Gef.: C (39.82), H (6.68), N (7.59), Cl (19.4)

b) 5-Amino-5-desoxy-1.4;3.6-dianhydro-D-mannit-2-nitrat

Zu 60 ml (1.35 Mol) 96%iger Salpetersäure (d = 1.5) tropft man unter Rühren und Kühlen auf −15° die Lösung von 4.2 g (70 mmol) Harnstoff in 90 ml (1.6 Mol) Schwefelsäure (d = 1.84). Bei gleicher Temperatur tropft man langsam die Lösung von 21.8 g (150 mmol) 2-Amino-2-desoxy-1.4;3.6-dianhydro-D-mannit (hergestellt aus dem zuvor erhaltenen Hydrogennitrat) in 15 ml Wasser zu, rührt 2 Stdn. bei −15° nach, gießt das Reaktionsgemisch in 1 Liter Wasser unter Rühren ein und stellt durch langsame Zugabe von 175 g (4.38 Mol) Natriumhydroxid – gelöst in 1 l Wasser – den pH = 9 ein. Anschließend extrahiert man 8 Stdn. im Rotationsperforator kontinuierlich mit Chloroform. Der Chloroformextrakt ergibt nach dem Trocknen über wasserfreiem Natriumsulfat, Filtrieren und Eindampfen unter reduziertem Druck 18.8 g (99 mmol) 5-Amino-5-desoxy-1.4;3.6-dianhydro-D-mannit-2-nitrat als langsam kristallisierendes Öl. Zur Charakterisierung überführt man einen Teil in das Hydrochlorid und kristallisiert aus Äthanol um.
Schmp. 172° (Z); $[\alpha]_D^{25}$ 170.9 (c 0.5; Wasser)

Elementaranalyse: $C_6H_{10}N_2O_5 \times HCl$ (226.62)
Ber.: C (31.80), H (4.89), N (12.36), Cl (15.64)
Gef.: C (31.76), H (4.93), N (12.67), Cl (16.0)

Beispiel Nr. 5

5-Pivaloylamino-5-desoxy-1.4;3.6-dianhydro-L-idit-2-nitrat

Zur Lösung von 3.8 g (20 mmol) 5-Amino-5-desoxy-1.4;3.6-dianhydro-L-idit-2-nitrat (Herstellung gemäß Beispiel 1c) in 30 ml Wasser gibt man 30 ml 1molare Natronlauge und 30 ml Äther. Unter kräftigem Rühren tropft man die Lösung von 3.6 g (30 mmol) Pivaloylchlorid in 30 ml Äther zu und erwärmt anschließend 2 Stdn. unter Rückfluß. Nach dem Abkühlen trennt man die Ätherphase ab, extrahiert die wäßrige Phase mit 50 ml Äther, trocknet die vereinigten Ätherphasen über wasserfreiem Natriumsulfat, filtriert und fällt das Produkt mit Petroläther aus. Der Niederschlag ergibt nach Umkristallisation aus Äther/Petroläther 4.95 g (18 mmol) 5-Pivaloylamino-5-desoxy-1.4;3.6-dianhydro-L-idit-2-nitrat.

Schmp. 96–97°; $[\alpha]_D^{25}$ 35.7 (c 0.53; Chloroform)

Elementaranalyse: $C_{11}H_{18}N_2O_6$ (274.27)
Ber.: C (48.17), H (6.61), N (10.21)
Gef.: C (47.97), H (6.74), N (10.29)

Beispiel Nr. 6

5-Acetylamino-5-desoxy-1.4;3.6-dianhydro-L-idit-2-nitrat

Unter kräftigem Rühren tropft man zur Mischung aus 3.8 g (20 mmol) 5-Amino-5-desoxy-1.4;3.6-dianhydro-L-idit-2-nitrat, 30 ml Äther und 20 ml Wasser die Lösung von 2.2 ml (30 mmol) Acetylchlorid in 20 ml Äther. Durch gleichzeitiges Zutropfen von 1 molarer Natronlauge hält man das Reaktionsgemisch auf pH = 7–8. Man rührt 2 Stdn. bei pH = 7 nach, trennt die Ätherphase ab, extrahiert die Wasserphase 2mal mit 30 ml Chloroform, dampft die Äther- und Chloroformphasen unter reduziertem Druck ein und kristallisiert den Rückstand aus Chloroform/Äther um. Man erhält 3.2 g (14 mmol) 5-Acetylamino-5-desoxy-1.4;3.6-dianhydro-L-idit-2-nitrat.
Schmp. 104–6°; $[\alpha]_D^{25}$ 33.7 (c 0.67; Methanol)

Elementaranalyse: $C_8H_{12}N_2O_6$ (232.20)
Ber.: C (41.38), H (5.21), N (12.06)
Gef.: C (41.44), H (5.34), N (11.32)

Beispiel Nr. 7

5-Nicotinoylamino-5-desoxy-1.4;3.6-dianhydro-L-idit-2-nitrat

Zur Suspension von 5.4 g (30 mmol) Nicotinsäurechlorid-Hydrochlorid in 100 ml Chloroform tropft man unter Rühren die Lösung von 19 g (100 mmol) 5-Amino-5-desoxy-1.4;3.6-dianhydro-L-idit-2-nitrat in 50 ml Chloroform zu und rührt weitere 15 Stdn. Überschüssiges Ausgangsamin fällt als Hydrochlorid aus und wird abgesaugt. Restliches Ausgangsamin wird durch Extraktion des Filtrats mit insgesamt 100 ml 0.2molarer Essigsäure zurückgewonnen. Die Chloroformlösung ergibt nach dem Waschen mit 50 ml 1 molarer Natronlauge, Trocknen über wasserfreiem Natriumsulfat und Eindampfen unter reduziertem Druck 5.3 g (18 mmol) Rohprodukt, das in Äthanol gelöst und durch Zugabe der äquimolaren Menge Salzsäure in das Hydrochlorid übergeführt wird. Eindampfen unter reduziertem Druck und Umkristallisation des Rückstandes aus Isopropanol/Äthanol ergeben 4.3 g (13 mmol) 5-Nicotinoyl-5-desoxy-1.4;3.6-dianhydro-L-idit-2-nitrat-Hydrochlorid.
Schmp. 173–5° (Z); $[\alpha]_D^{25}$ 41.8 (c 1; Wasser)

Elementaranalyse: $C_{12}H_{13}N_3O_6 \times HCl$ (331.71)
Ber.: C (43.45), H (4.25), N (12.67), Cl (10.69)
Gef.: C (43.58), H (4.29), N (12.21), Cl (10.8)

Beispiel Nr. 8

5-(2-Acetoxybenzoylamino)-5-desoxy-1.4;3.6-dianhydro-L-idit-2-nitrat

Zur Mischung aus 3.8 g (20 mmol) 5-Amino-5-desoxy-1.4;3.6-dianhydro-L-idit-2-nitrat, 20 ml Wasser und 30 ml Äther tropft man unter kräftigem Rühren die Lösung von 4.0 g (20 mmol) O-Acetylsalicylsäurechlorid in 50 ml Äther. Durch gleichzeitiges Zutropfen von 1 molarer Natronlauge hält man die Reaktionsmischung bei pH = 7. Man rührt noch 2 Stdn. bei pH 7 nach, saugt ausgefallenes Produkt (4 g) ab und wäscht den Niederschlag mit Wasser nach. Weitere 1.3 g Produkt gewinnt man aus der Ätherphase des Filtrats, die nacheinander mit 50 ml 0.1 molarer Salzsäure und mit 59 ml wäßriger Natriumhydrogencarbonatlösung gewaschen, über wasserfr. Natriumsulfat getrocknet und eingedampft wird. Die so erhaltenen 5.3 g Rohprodukt liefern nach der Umkristallisation aus Äthanol (unter Zusatz von Aktivkohle) reines 5-(2-Acetoxybenzoylamino)-5-desoxy-1.4;3.6-dianhydro-L-idit-2-nitrat.
Schmp. 123–5°; $[\alpha]_D^{25}$ 64.2 (c 0.52; Chloroform)

Elementaranalyse: $C_{15}H_{16}N_2O_8$ (352.31)
Ber.: C (51.14), H (4.58), N (7.95)
Gef.: C (51.59), H (4.69), N (7.93)

Beispiel Nr. 9

5-Methylamino-5-desoxy-1.4;3.6-dianhydro-L-idit-2-nitrat

a) 5-Methylamino-5-desoxy-1.4;3.6-dianhydro-L-idit

Die Mischung aus 22.4 g (0.1 Mol) 1.4;3.6-Dianhydro-D-glucit-5-methansulfonat (Herstellung siehe Beispiel 1a), 31 g (1 Mol) Methylamin und 150 ml n-Butanol wird im geschlossenen Stahlautoklaven 15 Stdn. bei 150° unter Stickstoffatmosphäre gerührt. Nach dem Abkühlen setzt man die Lösung von 4 g (0.1 Mol) Natriumhydroxid in 200 ml n-Butanol zu, rührt durch, fällt mit 600 ml Chloroform das gebildete Natriummethansulfonat aus, filtriert und dampft das Filtrat unter vermindertem Druck ein. Die so erhaltene ölige Rohbase wird in 100 ml Isopropanol gelöst und mit 6.5 ml 65%iger Salpetersäure in das Hydrogennitrat übergeführt. Nach dem Eindampfen unter reduziertem Druck kristallisiert man aus Isopropanol um und erhält 15.3 g (68,9 mmol) 5-Methylamino-5-desoxy-1.4;3.6-dianhydro-L-idit-Hydrogennitrat.
Schmp. 108–9°; $[\alpha]_D^{25}$ 41.8 (c 1.0; Wasser)

Elementaranalyse: $C_7H_{13}NO_3 \times HNO_3$ (222.20)
Ber.: C (37.84), H (6.35), N (12.61)
Gef.: C (38.00), H (6.60), N (12.23)

b) 5-Methylamino-5-desoxy-1.4;3.6-dianhydro-L-idit-2-nitrat

Unter Rühren und Kühlen auf 10° tropft man der Mischung aus 90 ml Essigsäure, 2.3 g (38 mmol) Harnstoff und 10.2 g (46 mmol) 5-Methylamino-5-desoxy-1.4;3.6-dianhydro-L-idit-Hydrogennitrat die Lösung von 5.9 ml (138 mmol) 95%iger Salpetersäure in 46 ml Essigsäure und anschließend noch 46 ml Acetanhydrid zu. Nach weiteren 4 Stdn. Rühren bei 10° fällt man mit Äther/Petroläther das kristalline Reaktionsprodukt aus und saugt ab. Der in 200 ml Wasser gelöste Niederschlag wird durch Zugabe von Natriumhydrogencarbonat neutralisiert und die Lösung 4mal mit je 150 ml Chloroform extrahiert. Die Chloroformextrakte werden nach dem Waschen mit 100 ml Wasser, Trocknen über wasserfreiem Natriumsulfat/Natriumcarbonat und Filtrieren unter vermindertem Druck eingedampft. Die so erhaltene Rohbase wird in 100 ml Äthanol gelöst, mit 46 ml 1molarer Salzsäure in das Hydrochlorid übergeführt, erneut unter reduziertem Druck eingedampft und zweimal aus Äthanol/Isopropanol umkristallisiert. Man erhält 5.58 g (23.2 mmol) 5-Methylamino-5-desoxy-1.4;3.6-dianhydro-L-idit-2-nitrat-Hydrochlorid.
Schmp. 163–6° (Z); $[\alpha]_D^{25}$ 49 (c 1.0; Wasser)

Elementaranalyse: $C_7H_{12}N_2O_5 \times HCl$ (240.64)
Ber.: C (34.94), H (5.44), N (11.64), Cl (14.73)
Gef.: C (35.00), H (5.57), N (11.92), Cl (14.8)

Beispiel Nr. 10

5-Äthylamino-5-desoxy-1.4;3.6-dianhydro-L-idit-2-nitrat

a) 5-Äthylamino-5-desoxy-1.4;3.6-dianhydro-L-idit

Das Gemisch aus 11.2 g (50 mmol) 1.4;3.6-Dianhydro-D-glucit-5-methansulfonat, 40.5 g (500 mmol) Äthylamin-Hydrochlorid, 20 g (500 mmol) Natriumhydroxid und 200 ml Wasser wird im geschlossenen Stahlautoklaven 15 Stdn. bei 150° unter Stickstoffatmosphäre gerührt. Nach dem Abkühlen und Entspannen gibt man 2 g (50 mmol) Natriumhydroxid zu, engt unter reduziertem Druck zur Trockne ein, extrahiert den Rückstand heiß mit Chloroform und dampft den Extrakt zur Trockne ein. Man erhält 9 g (ca. 50 mmol) öligen 5-Äthylamino-5-desoxy-1.4;3.6-dianhydro-L-idit, der für die nachfolgende Veresterung mit Salpetersäure eingesetzt wird. Zur Charakterisierung wird ein kleiner Anteil in das Hydrochlorid übergeführt und 2mal aus Isopropanol umkristallisiert.
Schmp. 181–3° (Z); $[\alpha]_D^{25}$ 51.3 (c 1; Wasser).

Elementaranalyse: $C_8H_{15}NO_3 \times HCl$ (209.68)
Ber.: C (45.83), H (7.69), N (6.68), Cl (16.91)
Gef.: C (46.26), H (8.06), N (6.69), Cl (16.9)

b) 5-Äthylamino-5-desoxy-1.4;3.6-dianhydro-L-idit-2-nitrat

Zur Mischung aus 6 g (35 mmol) 5-Äthylamino-5-desoxy-1.4;3.6-dianhydro-L-idit, 70 ml Essigsäure und 2.1 g (35 mmol) Harnstoff tropft man unter Rühren und Kühlen auf 10° zunächst die Lösung von 6.2 ml (140 mmol) 96%iger Salpetersäure (d = 1.5) in 35 ml Essigsäure und anschließend 35 ml Acetanhydrid zu. Man rührt die entstandene Suspension 3 Stdn. bei 10°, wobei eine klare Lösung entsteht. Mit 800 ml Äther fällt man das Rohprodukt als Hydrogennitrat aus, trennt die Fällung ab, löst sie in 100 ml Wasser und neutralisiert mit Natriumhydrogencarbonat. Dreimalige Extraktion mit je 200 ml Chloroform, Trocknen über wasserfreiem Natriumsulfat und Eindampfen unter reduziertem Druck liefern öliges Rohprodukt in Form der freien Base. Man überführt in äthanolischer Lösung mit der äquimolaren Menge Salzsäure in das Hydrochlorid, dampft erneut unter reduziertem Druck ein, kristallisiert aus Dichlormethan um und erhält 4.2 g (16.5 mmol) 5-Äthylamino-5-desoxy-1.4;3.6-dianhydro-L-idit-2-nitrat-Hydrochlorid.

Schmp. 146–7° (Z); $[\alpha]_D^{25}$ 59.5 (c 0.7; Wasser)

Elementaranalyse: $C_8H_{14}N_2O_5 \times HCl$ (254.67)
Ber.: C (37.73), H (5.94), N (11.00), Cl (13.92)
Gef.: C (38.07), H (6.30), N (10.84), Cl (14.2)

Beispiel Nr. 11

5-Adamant-1-ylamino-5-desoxy-1.4;3.6-dianhydro-L-idit-2-nitrat

a) 5-Adamant-1-ylamino-5-desoxy-1.4;3.6-dianhydro-L-idit

Das Gemisch aus 11.2 g (50 mmol) 1.4;3.6-Dianhydro-D-glucit-5-methansulfonat, 39 g (250 mmol) 1-Aminoadamantan und 100 ml n-Butanol wird 3 Tage im geschlossenen Stahlautoklaven auf 150° erhitzt. Anschließend dampft man unter reduziertem Druck zur Trockne ein, löst den Rückstand in 250 ml Chloroform, gewinnt durch Extraktion mit 1 molarer Salzsäure zunächst überschüssiges Adamantylamin zurück und bei weiterer Extraktion das Reaktionsprodukt. Die salzsaure Lösung des Reaktionsproduktes wird mit verdünnter Natronlauge auf pH = 8 gestellt und mit Chloroform reextrahiert. Eindampfen der über wasserfr. Natriumsulfat getrockneten Chloroformphasen liefert 3.85 g (13.8 mmol) 5-Adamant-1-ylamino-5-desoxy-1.4;3.6-dianhydro-L-idit. Zur Analyse kristallisiert man aus Cyclohexan um.
Schmp. 126–7°; $[\alpha]_D^{25}$ 26.0 (c 1; Äthanol)

Elementaranalyse: $C_{16}H_{25}NO_3$ (279.39)
Ber.: C (68.79), H (9.02), N (5.01)
Gef.: C (68.80), H (9.25), N (5.07)

b) 5-Adamant-1-ylamino-5-desoxy-1.4;3.6-dianhydro-L-idit-2-nitrat

Zur Mischung aus 2.08 g (7.44 mmol) 5-Adamant-1-ylamino-1.4;3.6-dianhydro-L-idit, 0.5 g (8.3 mmol) Harnstoff und 40 ml Essigsäure tropft man unter Rühren und Kühlen auf 10–15° die Lösung von 1.3 ml (30 mmol) 96%iger Salpetersäure in 10 ml Essigsäure und anschließend 10 ml Acetanhydrid zu. Man rührt 3 Stdn. bei 15° nach und fällt das Produkt mit Äther/Petroläther aus. Das so erhaltene ölige Rohprodukt wird in 200 ml Chloroform gelöst und mit wäßriger Natriumhydrogencarbonatlösung säurefrei gewaschen. Man trocknet über wasserfreiem Natriumsulfat, gibt 8 mmol äthanolische Salzsäure zu, dampft unter reduziertem Druck ein, kristallisiert aus Äthanol/Isopropanol um und erhält 2.11 g (5.85 mmol) 5-Adamant-1-ylamino-5-desoxy-1.4;3.6-dianhydro-L-idit-2-nitrat-Hydrochlorid.
Schmp. 207–10° (Z); $[\alpha]_D^{25}$ 30.1 (c 1; Äthanol)

Elementaranalyse: $C_{16}H_{24}N_2O_5 \times HCl$ (360.84)
Ber.: C (53.26), H (6.98), N (7.76), Cl (9.82)
Gef.: C (53.22), H (7.25), N (7.60), Cl (10.2)

Beispiel Nr. 12

5-Dimethylamino-5-desoxy-1.4;3.6-dianhydro-L-idit-2-nitrat

a) 5-Dimethylamino-5-desoxy-1.4;3.6-dianhydro-L-idit

22.5 g (0.5 Mol) Dimethylamin werden unter Kühlen in 100 ml n-Butanol gelöst, mit 11.2 g (50 mmol) 1.4;3.6-Dianhydro-D-glucit-5-methansulfonat versetzt und 15 Std. im geschlossenen Stahlautoklaven auf 150° erhitzt. Nach dem Abkühlen und Entspannen gibt man 100 ml 0.5 molare butanolische Natriumhydroxidlösung zu, filtriert und dampft unter reduziertem Druck ein. Der Rückstand wird mit Chloroform extrahiert und der Chloroformextrakt unter reduziertem Druck eingedampft. Man erhält 8.5 g (49 mmol) öligen 5-Dimethylamino-5-desoxy-1.4;3.6-dianhydro-L-idit. Zur Charakterisierung überführt man einen kleinen Teil in das Hydrochlorid und kristallisiert aus Isopropanol um.
Schmp. 227–9°; $[\alpha]_D^{25}$ 46.8 (c 0.58; Wasser)

Elementaranalyse: $C_8H_{15}NO_3 \times HCl$ (209.68)
Ber.: C (45.82), H (7.69), N (6.68), Cl (16.91)
Gef.: C (46.02), H (7.96), N (6.79), Cl (16.6)

b) 5-Dimethylamino-5-desoxy-1.4;3.6-dianhydro-L-idit-2-nitrat

Der zuvor erhaltene 5-Dimethylamino-5-desoxy-1.4;3.6-dianhydro-L-idit wird in Isopropanol gelöst und durch Zugabe der äquimolaren Menge 65%iger Salpetersäure als Hydrogennitrat ausgefällt. 4.7 g (20 mmol) des Hydrogennitrats werden analog Beispiel 9b mit Salpetersäure verestert, anschließend in das Hydrochlorid überführt und aus Äthanol/Isopropanol umkristallisiert. Man erhält 3.67 g (14.4 mmol) 5-Dimethylamino-5-desoxy-1.4;3.6-dianhydro-L-idit-2-nitrat-Hydrochlorid.
Schmp. 184–9° (Z); $[\alpha]_D^{25}$ 50.9 (c 1; Wasser)

Elementaranalyse: $C_8H_{14}N_2O_5 \times HCl$ (254.67)
Ber.: C (37.73), H (5.94), N (11.00), Cl (13.92)
Gef.: C (37.70), H (6.07), N (10.93), Cl (14.3)

Beispiel Nr. 13

5-Diäthylamino-5-desoxy-1.4;3.6-dianhydro-L-idit-2-nitrat

a) 5-Diäthylamino-5-desoxy-1.4;3.6-dianhydro-L-idit

Herstellung analog Beispiel 12a) durch Umsetzung von überschüssigem Diäthylamin mit 1.4;3.6-Dianhydro-D-glucit-5-methansulfonat in Butanol bei 150°. Isolierung als Hydrogennitrat.
Schmp. 129° (aus Isopropanol); $[\alpha]_D^{25}$ 39.8 (c 1; Wasser)

Elementaranalyse: $C_{10}H_{19}NO_3 \times HNO_3$ (264.28)
Ber.: C (45.45), H (7.63), N (10.60)
Gef.: C (44.97), H (7.86), N (10.65)

b) 5-Diäthylamino-5-desoxy-1.4;3.6-dianhydro-L-idit-2-nitrat

2.1 g (8 mmol) des zuvor erhaltenen Hydrogennitrats werden analog Beispiel 9b mit Salpetersäure verestert und ergeben nach Überführung in das Hydrochlorid und Umkristallisation aus Äthanol/Isopropanol 1.6 g (5.7 mmol) Reinprodukt.
Schmp. 182–6° (Z); $[\alpha]_D^{25}$ 45.3 (c 1; Wasser)

Elementaranalyse: $C_{10}H_{18}N_2O_5 \times HCl$ (282.72)
Ber.: C (42.48), H (6.77), N (9.91), Cl (12.54)
Gef.: C (42.62), H (7.24), N (9.64), Cl (12.8)

Beispiel Nr. 14

5-Pyrrolidino-5-desoxy-1.4;3.6-dianhydro-L-idit-2-nitrat

a) 5-Pyrrolidino-5-desoxy-1.4;3.6-dianhydro-L-idit

Das Gemisch aus 11.2 g (50 mmol) 1.4;3.6-Dianhydro-D-glucit-5-methansulfonat, 36 g (0.5 Mol) Pyrrolidin und 100 ml n-Butanol wird im geschlossenen Stahlautoklaven unter Stickstoffschutzatmosphäre 24 Stdn. auf 150° erhitzt. Nach dem Abkühlen gibt man 10 ml 5molare Natronlauge zu, dampft unter reduziertem Druck das überschüssige Pyrrolidin ab — gegen Ende unter Zusatz von Wasser — und extrahiert das Produkt aus dem Rückstand mit Chloroform heraus. Der eingedampfte Chloroformextrakt wird durch Lösen in Äther von unlöslichen Nebenprodukten befreit. Das Ätherfiltrat liefert nach Trocknen über wasserfr. Natriumsulfat und Eindampfen 8.7 g (43.7 mmol) 5-Pyrrolidino-5-desoxy-1.4;3.6-dianhydro-L-idit. Zur Charakterisierung wird ein kleiner Teil in das Hydrogennitrat übergeführt und aus Isopropanol umkristallisiert.
Schmp. 123—4°; $[\alpha]_D^{25}$ 45.9 (c 1; Äthanol)

Elementaranalyse: $C_{10}H_{17}NO_3 \times HNO_3$ (262.26)
Ber.: C (45.80), H (6.92), N (10.68)
Gef.: C (45.74), H (7.16), N (10.79)

b) 5-Pyrrolidino-5-desoxy-1.4;3.6-dianhydro-L-idit-2-nitrat

Zum Gemisch aus 6 g (30 mmol) 5-Pyrrolidino-5-desoxy-1.4;3.6-dianhydro-L-idit, 1.5 g (25 mmol) Harnstoff und 60 ml Essigsäure tropft man unter Rühren und Kühlen auf 10° die Lösung von 4 ml (90 mmol) 95%iger Salpetersäure in 30 ml Essigsäure und anschließend 30 ml Acetanhydrid. Man rührt 4 Stdn. bei 10°, verdünnt mit 400 ml Wasser, rührt ½ Stde. und gibt 7.5 g Natriumhydrogencarbonat zu. Nach beendeter Hydrolyse des Acetanhydrids stellt man mit weiterem Natriumhydrogencarbonat auf pH = 6—7 ein und extrahiert 3mal mit je 180 ml Chloroform. Die vereinigten Chloroformextrakte werden unter vermindertem Druck eingedampft, mit 30 ml 1molarer Salzsäure in das Hydrochlorid übergeführt, erneut eingedampft und aus Isopropanol umkristallisiert. Man erhält 3.4 g (12.1 mmol) 5-Pyrrolidino-5-desoxy-1.4;3.6-dianhydro-L-idit-2-nitrat-Hydrochlorid.
Schmp. 172—3° (Z); $[\alpha]_D^{25}$ 52.7 (c 0.52; Wasser)

Elementaranalyse: $C_{10}H_{16}N_2O_5 \times HCl$ (280.71)
Ber.: C (42.79), H (6.11), N (9.98), Cl (12.63)
Gef.: C (43.05), H (6.20), N (10.01), Cl (12.4)

Beispiel Nr. 15

5-Piperidino-5-desoxy-1.4;3.6-dianhydro-L-idit-2-nitrat

a) 5-Piperidino-5-desoxy-1.4;3.6-dianhydro-L-idit

Das Gemisch aus 11.2 g (50 mmol) 1.4;3.6-Dianhydro-D-glucit-5-methansulfonat, 42.5 (0.5 Mol) Piperidin und 100 ml n-Butanol wird 24 Stdn. im geschlossenen Stahlautoklaven unter Stickstoffatmosphäre auf 150° erhitzt. Nach dem Eindampfen unter reduziertem Druck extrahiert man den Rückstand mit Äther, wobei Piperidin-Hydrogenmethansulfonat ungelöst bleibt. Der über wasserfr. Natriumsulfat getrocknete Ätherextrakt ergibt nach dem Eindampfen 10 g (47 mmol) Produkt, die in 50 ml Essigsäure gelöst und mit der äquimolaren Menge 30%iger Salpetersäure in das Hydrogennitrat übergeführt werden. Dieses wird mit Äther ausgefällt und ergibt nach Umkristallisation aus Isopropanol 11.6 g (42 mmol) 5-Piperidino-5-desoxy-1.4;3.6-dianhydro-L-idit-Hydrogennitrat.
Schmp. 182—4° (Z); $[\alpha]_D^{25}$ 46.0 (c 1; Äthanol)

Elementaranalyse: $C_{11}H_{19}NO_3 \times HNO_3$ (276.29)
Ber.: C (47.82), H (7.30), N (10.14)
Gef.: C (47.80), H (7.44), N (10.21)

b) 5-Piperidino-5-desoxy-1.4;3.6-dianhydro-L-idit-2-nitrat

8.3 g (30 mmol) des zuvor erhaltenen Hydrogennitrats werden analog Beispiel 9b) mit Salpetersäure verestert, in das Hydrochlorid übergeführt und aus Isopropanol umkristallisiert.
Ausbeute: 7.2 g (24.4 mmol)

Schmp. 186–8° (Z); $[\alpha]_D^{25}$ 47.0 (c 0.5; Wasser)

Elementaranalyse: $C_{11}H_{18}N_2O_5 \times HCl$ (294.73)
Ber.: C (44.83), H (6.50), N (9.50), Cl (12.03)
Gef.: C (44.73), H (6.58), N (9.53), Cl (11.8)

Beispiel Nr. 16

5-Morpholino-5-desoxy-1.4;3.6-dianhydro-L-idit-2-nitrat

a) 5-Morpholino-5-desoxy-1.4;3.6-dianhydro-L-idit

Herstellung analog Beispiel 12a) durch Umsetzung von 50 mmol 1.4;3.6-Dianhydro-D-glucit-5-methansulfonat mit 0.5 Mol Morpholin. Ausbeute 8.6 g (40 mmol) öliges Produkt. Charakterisierung als Hydrochlorid.
Schmp. 159–161° (aus Isopropanol/Äthanol); $[\alpha]_D^{25}$ 48.5 (c 1; Äthanol).

Elementaranalyse: $C_{10}H_{17}NO_4 \times HCl$ (251.71)
Ber.: C (47.72), H (7.21), N (5.57)
Gef.: C (47.42), H (7.20), N (5.61)

b) 5-Morpholino-5-desoxy-1.4;3.6-dianhydro-L-idit-2-nitrat

3.7 g (17 mmol) des zuvor erhaltenen 5-Morpholino-5-desoxy-1.4;3.6-dianhydro-L-idits werden analog Beispiel 14b) mit Salpetersäure verestert, nach der Aufarbeitung in das Hydrochlorid übergeführt und aus Isopropanol umkristallisiert. Man erhält 2.5 g (8.4 mmol) 5-Morpholino-5-desoxy-1.4;3.6-dianhydro-L-idit-2-nitrat-Hydrochlorid.
Schmp. 195–6° (Z); $[\alpha]_D^{25}$ 46.5 (c 0.51; Wasser)

Elementaranalyse: $C_{10}H_{16}N_2O_6 \times HCl$ (296.71)
Ber.: C (40.48), H (5.78), N (9.44), Cl (11.95)
Gef.: C (40.51), H (5.57), N (9.44), Cl (12.1)

Beispiel Nr. 17

5-(4-Methylpiperazino)-5-desoxy-1.4;3.6-dianhydro-L-idit-2-nitrat

a) 5-(4-Methylpiperazino)-5-desoxy-1.4;3.6-dianhydro-L-idit

Die Mischung aus 11.2 g (50 mmol) 1.4;3.6-Dianhydro-D-glucit-5-methansulfonat und 15 g (150 mmol) N-Methylpiperazin wird unter Stickstoffatmosphäre bei 150° 24 Stdn. im Stahlautoklaven gerührt. Nach dem Abkühlen gibt man eine Lösung von 2.0 g (50 mmol) Natriumhydroxid in 100 ml n-Butanol zu und dampft unter reduziertem Druck zur Trockne ein. Der ölige Rückstand wird mit 500 ml Chloroform behandelt und vom Ungelösten (Natriummethansulfonat) abfiltriert. Die Chloroformphase wird 2mal mit je 200 ml Wasser extrahiert; die vereinigten Wasserphasen ergeben nach dem Eindampfen i. Vak. und azeotrop. Trocknung mit Äthanol und Chloroform die Rohbase. Diese wird in 50 ml Eisessig gelöst und durch Zugabe der doppelt molaren Menge an verd. Salpetersäure in das Dihydrogennitrat übergeführt, das größtenteils direkt auskristallisiert. Restliches Produkt fällt man mit Äther aus. Umkristallisation aus Isopropanol/Äthanol ergibt 8.9 g (25 mmol) 5-(4-Methylpiperazino)-5-desoxy-1.4;3.6-dianhydro-L-idit-Dihydrogennitrat.
Schmp. 124–5° (Z); $[\alpha]_D^{25}$ 27.9 (c 0.5; Wasser)

Elementaranalyse: $C_{11}H_{20}N_2O_3 \times 2\ HNO_3$ (354.32)
Ber.: C (37.29), H (6.26), N (15.81)
Gef.: C (37.10), H (6.36), N (15.54)

b) 5-(4-Methylpiperazino)-5-desoxy-1.4;3.6-dianhydro-L-idit-2-nitrat

Zur Mischung aus 5.82 g (16 mmol) des zuvor erhaltenen Dihydrogennitrats, 1,0 g (16.5 mmol) Harnstoff und 40 ml Eisessig läßt man unter Rühren und Kühlen auf 10°C zunächst eine Lösung von 2.6 ml (59 mmol) 96%ig. Salpetersäure (d = 1.5) in 20 ml Eisessig und anschließend 20 ml Acetanhydrid zutropfen und rührt über Nacht bei 10° weiter. Zu der Suspension gibt man zur vollständigen Ausfällung

des Produkts noch Äther/Petroläther zu, dekantiert ab und löst den Niederschlag in ca. 200 ml Wasser auf. Man gibt bis pH = ca. 6 Natriumhydrogencarbonat zu. Nach ½ Std. Rühren extrahiert man 3mal mit je 150 ml Chloroform, wäscht die vereinigten Chloroformphasen mit 100 ml Wasser, trocknet über wfr. Natriumsulfat/Natriumcarbonat, konzentriert unter reduziertem Druck und überführt durch Zugabe von 33 ml 1molarer HCl in das Dihydrochlorid. Nach dem Eindampfen unter reduziertem Druck und Umkristallisation aus Isopropanol erhält man 3.2 g (9.2 mmol) 5-(4-Methylpiperazino)-5-desoxy-1.4;3.6-dianhydro-L-idit-2-nitrat-Dihydrochlorid.
Schmp. 201—9° (Z) $[\alpha]_D^{25}$ 32.7 (c 0.53; Wasser)

Elementaranalyse: $C_{11}H_{19}N_3O_5 \times 2\ HCl$ (346.22)
Ber.: C (38.16), H (6.11), N (12.14), Cl (20.48)
Gef.: C (38.08), H (6.30), N (12.02), Cl (19.9)

## Beispiel Nr. 18

### 5-Pyridoxylidenamino-5-desoxy-1.4;3.6-dianhydro-L-idit-2-nitrat

Die Lösung von 1.2 g (30 mmol) Natriumhydroxid in 80 ml Äthanol wird nacheinander mit 6.1 g (30 mmol) Pyridoxyl-Hydrochlorid, 5.7 g (30 mmol) 5-Amino-5-desoxy-1.4;3.6-dianhydro-L-idit-2-nitrat (gelöst in 20 ml Äthanol) und 100 ml Benzol versetzt und zum Sieden erhitzt. Man destilliert das Aceotrop aus Benzol/Äthanol/Wasser (Kp = 64.9°) ab, wobei das Reaktionsvolumen konstant gehalten wird. Sobald die Siedetemperatur auf 68° (Aceotrop Äthanol/Benzol) gestiegen ist, kühlt man ab, saugt vom Natriumchlorid ab, dampft das Filtrat unter reduziertem Druck ein und erhält 10 g (29.5 mmol) öliges Rohprodukt. Zur weiteren Reinignug laugt man das Rohprodukt mit Äther aus, löst den eingedampften Ätherextrakt in wenig Benzol, verdünnt mit der ca. 20fachen Menge Äther und fällt vorsichtig mit Petroläther an. Man erhält 7.1 g (21 mmol) kristallines 5-Pyridoxylidenamino-5-desoxy-1.4;3.6-dianhydro-L-idit-2-nitrat.
Schmp. 123—6° $[\alpha]_D^{25}$ 140.5 (c 0.2; Chloroform)

Elementaranalyse: $C_{14}H_{17}N_3O_7$ (339.32)
Ber.: C (49.56), H (5.05), N (12.38)
Gef.: C (49.28), H (4.95), N (12.16)

## Beispiel Nr. 19

### 5-(N-Benzyl-N-methylamino)-5-desoxy-1.4;3.6-dianhydro-L-idit-2-nitrat

Die Lösung von 4.1 g (20 mmol) 5-Methylamino-5-desoxy-1.4;3.6-dianhydro-L-idit-2-nitrat (Herstellung siehe Beispiel 9b) in 50 ml Äthanol wird mit 2.5 ml (21 mmol) Benzylchlorid versetzt und 2 Stdn. unter Rückfluß erhitzt. Man läßt abkühlen, gibt 800 ml (20 mmol) Natriumhydroxid zu und erwärmt erneut 2 Stdn. unter Rückfluß. Eindampfen unter reduziertem Druck, Extraktion des Rückstandes mit Dichlormethan, Zugabe von 20 mmol äthanolischer Chlorwasserstofflösung, erneutes Eindampfen und Umkristallisation aus Isopropanol ergibt 4.1 g (12.4 mmol) 5-(N-Benzyl-N-methylamino)-5-desoxy-1.4;3.6-dianhydro-L-idit-2-nitrat-Hydrochlorid.
Schmp. 170—3°; $[\alpha]_D^{25}$ 39.2 (c 1; Wasser)

Elementaranalyse: $C_{14}H_{18}N_2O_3 \times HCl$ (330.77)
Ber.: C (50.84), H (5.79), N (8.47), Cl (10.72)
Gef.: C (50.03), H (5.77), N (8.25), Cl (11.2)

## Beispiel Nr. 20

### 5-[N-(2-Phenyläthyl)-N-methylamino]-5-desoxy-1.4;3.6-dianhydro-L-idit-2-nitrat

Die Mischung aus 10 g (49 mmol) 5-Methylamino-5-desoxy-1.4;3.6-dianhydro-L-idit-2-nitrat, 50 ml Äthanol und 3.0 ml (20 mmol) 2-Phenyläthylbromid wird 14 Stdn. unter Rückfluß erhitzt und unter reduziertem Druck eingedampft. Der Rückstand wird zwischen 50 ml Chloroform und 30 ml Wasser verteilt. Die abgetrennte Chloroformphase wird durch zweimalige Extraktion mit je 25 ml 0.2molarer Salzsäure von überschüssigem Ausgangsamin befreit, nach dem Trocknen über wasserfr. Natriumsulfat unter reduziertem Druck eingedampft, in äthanolischer Lösung mit 20 mmol Salzsäure in das Hydrochlorid übergeführt und aus Äthanol umkristallisiert. Man erhält 4.1 g (12 mmol) 5-[N-(2-Phenyläthyl)-N-methylamino]-5-desoxy-1.4;3.6-dianhydro-L-idit-2-nitrat-Hydrochlorid.

Schmp. 165–9°; $[\alpha]_D^{25}$ 35.2 (c 0.4; Wasser)

Elementaranalyse: $C_{15}H_{20}N_2O_5 \times HCl$ (344.80)
Ber.: C (52.25), H (6.14), N (8.12), Cl (10.28)
Gef.: C (52.18), H (6.28), N (7.58), Cl (10.7)

## Beispiel Nr. 21

### 5-[N-Methyl-N-(3-phenylpropyl)-amino]-5-desoxy-1.4;3.6-dianhydro-L-idit-2-nitrat

Herstellung analog Beispiel 20 durch Umsetzung von 49 mmol 5-Methylamino-5-desoxy-1.4;3.6-dianhydro-L-idit-2-nitrat mit 20 mmol 3-Phenyl-1-brompropan in Äthanol. Isolierung als Hydrochlorid. Ausbeute nach Umkristallisation aus Isopropanol/Äthanol: 5.2 g (14.5 mmol).
Schmp. 142–5°; $[\alpha]_D^{25}$ 32.2 (c 0.4; Wasser)

Elementaranalyse: $C_{16}H_{22}N_2O_5 \times HCl$ (358.82)
Ber.: C (53.56), H (6.46), N (7.81), Cl (9.88)
Gef.: C (53.33), H (6.62), N (7.65), Cl (10.3)

## Beispiel Nr. 22

### 5-[N-(4-Phenylbutyl)-N-methylamino]-5-desoxy-1.4;3.6-dianhydro-L-idit-2-nitrat

Herstellung analog Beispiel 20. Isolierung als Hydrochlorid. Ausbeute nach Umkristallisation aus Isopropanol: 60%.
Schmp.: 143–6°; $[\alpha]_D^{25}$ 35.1 (c 1; Wasser)

Elementaranalyse: $C_{17}H_{24}N_2O_5 \times HCl$ (372.85)
Ber.: C (54.76), H (6.76), N (7.51), Cl (9.51)
Gef.: C (54.78), H (7.02), N (7.47), Cl (9.4)

## Beispiel Nr. 23

### 5-[N-Methyl-N-(5-phenylpentyl)-amino]-5-desoxy-1.4;3.6-dianhydro-L-idit-2-nitrat

Herstellung analog Beispiel 20. Isolierung als Hydrochlorid. Ausbeute nach Umkristallisation aus Isopropanol/Äther: 51%.
Schmp. 114–7°; $[\alpha]_D^{25}$ 36.2 (c 1.0; Wasser)

Elementaranalyse: $C_{18}H_{26}N_2O_5 \times HCl$ (386.88)
Ber.: C (55.88), H (7.04), N (7.24), Cl (9.16)
Gef.: C (55.89), H (7.20), N (7.19), Cl (9.4)

## Beispiel Nr. 24

### 5-(N-Cinnamyl-N-methylamino)-5-desoxy-1.4;3.6-dianhydro-L-idit-2-nitrat

Herstellung analog Beispiel 20 durch Umsetzung von 5-Methylamino-5-desoxy-1.4;3.6-dianhydro-L-idit-2-nitrat mit 3-Phenyl-1-brom-2-propen. Isolierung als Hydrochlorid.
Schmp. 155–7° (aus Isopropanol umkrist.) $[\alpha]_D^{25}$ 23.2 (c 0.44; Wasser)

Elementaranalyse: $C_{18}H_{20}N_2O_5 \times HCl$ (356.81)
Ber.: C (53.86), H (5.93), N (7.85), Cl (9.94)
Gef.: C (53.91), H (6.05), N (7.64), Cl (9.9)

## Beispiel Nr. 25

### 5-Benzylamino-5-desoxy-1.4;3.6-dianhydro-L-idit-2-nitrat

Die Mischung aus 15.2 g (80 mmol) 5-Amino-5-desoxy-1.4;3.6-dianhydro-L-idit-2-nitrat (Herstellung siehe Beispiel 1 c), 70 ml Äthanol und 3.6 ml (30 mmol) Benzylbromid wird 5 Stdn. unter Rückfluß gekocht. Nach dem Abkühlen saugt man von auskristallisiertem überschüssigem Ausgangsamin-Hydrobromid ab, dampft das Filtrat unter reduziertem Druck ein, löst den Rückstand in 60 ml Chloroform und extrahiert das Produkt mit 0.5molarer Salzsäure. Die Salzsäurephasen werden mit verdünnter Natronlauge auf pH = 8 bis 9 gebracht und das Produkt mit Chloroform reextrahiert. Der Chloroformextrakt wird nach dem Eindampfen mit 30 mmol Salzsäure in das Hydrochlorid übergeführt und aus Methanol/Isopropanol umkristallisiert. Man erhält 6.0 g (18.9 mmol) 5-Benzylamino-5-desoxy-1.4;3.6-dianhydro-L-idit-2-nitrat-Hydrochlorid.
Schmp. 171—5° (Z); $[\alpha]_D^{25}$ 51.2 (c 0.4; Wasser)

Elementaranalyse: $C_{13}H_{16}N_2O_5 \times HCl$ (316.74)
Ber.: C (49.30), H (5.41), N (8.84), Cl (11.19)
Gef.: C (49.28), H (5.42), N (8.87), Cl (11.3)

## Beispiel Nr. 26

### 5-Diphenylmethylamino-5-desoxy-1.4;3.6-dianhydro-L-idit-2-nitrat

Die Mischung aus 5.7 g (30 mmol) 5-Amino-5-desoxy-1.4;3.6-dianhydro-L-idit-2-nitrat, 40 ml Dimethylformamid und 3.9 g (15 mmol) Bromdiphenylmethan wird 2 Tage bei 80°C gerührt. Nach dem Abkühlen stellt man durch Zugabe von 640 mg (16 mmol) Natriumhydroxid auf pH = 8—9 und dampft unter vermindertem Druck ein. Der Rückstand wird mit Dichlormethan heiß extrahiert, der Extrakt mit Chlorwasserstoff gesättigt, erneut unter reduziertem Druck eingedampft und mit Toluol heiß extrahiert. Der Toluolextrakt wird mehrmals mit Wasser gewaschen und unter reduziertem Druck eingedampft. Man erhält 2.5 g (6.4 mmol) 5-Diphenylmethyl-amino-5-desoxy-1.4;3.6-dianhydro-L-idit-2-nitrat-Hydrochlorid. Die analytische Probe fällt man aus Dichlormethan mit Äther/Petroläther um.
Schmp. 189—195° (Z); $[\alpha]_D^{25}$ 78.3 (c 1; Chloroform).

Elementaranalyse: $C_{19}H_{20}N_2O_5 \times HCl$ (392.84)
Ber.: C (58.09), H (5.39), N (7.13), Cl (9.02)
Gef.: C (57.91), H (5.44), N (7.08), Cl (8.8)

## Beispiel Nr. 27

### 5-(2-Phenyläthylamino)-5-desoxy-1.4;3.6-dianhydro-L-idit-2-nitrat

Herstellung analog Beispiel 25. Isolierung als Hydrochlorid. Ausbeute: 53%. Schmp. 185° (Z) (nach Umkristallisation aus Wasser). $[\alpha]_D^{25}$ 48.6 (c 0.21; Wasser).

Elementaranalyse: $C_{14}H_{18}N_2O_5 \times HCl$ (330.77)
Ber.: C (50.84), H (5.79), N (8.47), Cl (10.72)
Gef.: C (50.54), H (5.76), N (8.46), Cl (10.3)

## Beispiel Nr. 28

### 5-(3-Phenylpropylamino)-5-desoxy-1.4;3.6-dianhydro-L-idit-2-nitrat

Herstellung analog Beispiel 25. Isolierung als Hydrochlorid. Ausbeute nach Umkristallisation aus Methanol/Wasser: 49%.
Schmp. 174° (Z); $[\alpha]_D^{25}$ 48.8 (c 0.5; Methanol).

Elementaranalyse: $C_{15}H_{20}N_2O_5 \times HCl$ (344.80)
Ber.: C (52.25), H (6.14), N (8.12), Cl (10.28)
Gef.: C (52.42), H (6.50), N (7.90), Cl (10.7)

## Beispiel Nr. 29

### 5-(4-Phenylbutylamino)-5-desoxy-1.4;3.6-dianhydro-L-idit-2-nitrat

Die Mischung aus 16 g (75 mmol) 4-Phenyl-1-brombutan, 250 ml Äthanol und 35 g (184 mmol) 5-Amino-5-desoxy-1.4;3.6-dianhydro-L-idit-2-nitrat wird 24 Stdn. unter Rückfluß gekocht. Nach

dem Abkühlen saugt man von überschüssigem Ausgangsamin-Hydrobromid ab, wäscht den Niederschlag mit Dichlormethan nach, engt die Filtrate unter reduziertem Druck ein, versetzt den Rückstand mit 200 ml Dichlormethan und extrahiert 2mal mit je 50 ml 5%iger Essigsäure noch enthaltenes Ausgangsamin. Nach dem Waschen mit 50 ml 1molarer Natronlauge, Trocknen über wasserfr. Natriumsulfat und Eindampfen unter reduziertem Druck erhält man aus der Dichlormethanphase 21.5 g (67 mmol) Rohprodukt, das in 100 ml Äthanol gelöst, mit 68 mmol Salzsäure versetzt und erneut unter reduziertem Druck eingedampft wird. Zweimalige Umkristallisation aus Äthanol unter Zusatz von Aktivkohle ergibt 10.3 g (29 mmol) 5-(4-Phenylbutylamino)-5-desoxy-1.4;3.6-dianhydro-L-idit-2-nitrat-Hydrochlorid.
Schmp. 166–8°; $[\alpha]_D^{25}$ 41.6 (c 0.39; Wasser)

Elementaranalyse: $C_{16}H_{22}N_2O_5 \times HCl$ (358.82)
Ber.: C (53.56), H (6.46), N (7.81), Cl (9.88)
Gef.: C (53.48), H (6.51), N (7.89), Cl (10.1)

Beispiel Nr. 30

5-(5-Phenylpentylamino)-5-desoxy-1.4;3.6-dianhydro-L-idit-2-nitrat

Herstellung analog Beispiel 29. Isolierung als Hydrochlorid. Ausbeute nach Umkristallisation aus Wasser (unter Zusatz von Aktivkohle): 37%.
Schmp. 146–8°; $[\alpha]_D^{25}$ 41.1 (c 0.3; Wasser)

Elementaranalyse: $C_{17}H_{24}N_2O_5 \times HCl$ (372.85)
Ber.: C (54.76), H (6.76), N (7.51), Cl (9.51)
Gef.: C (54.88), H (6.91), N (7.40), Cl (9.6)

Beispiel Nr. 31

5-[3-(3.4-Dimethoxyphenyl)-propylamino]-5-desoxy-1.4;3.6-dianhydro-L-idit-2-nitrat

a) 3-(3.4-Dimethoxyphenyl)-propyl-1-methansulfonat

Dem Gemisch aus 10 g (50 mmol) 3-(3.4-Dimethoxyphenyl)-1-propanol, 50 ml Chloroform und 14 ml (100 mmol) Triäthylamin wird unter Rühren Feuchtigkeitsausschluß und Kühlen auf −15° die Lösung von 5.5 ml (70 mmol) Methansulfonsäurechlorid in 20 ml Chloroform zugetropft. Anschließend rührt man 2 Stdn. im Kältebad, läßt auf Raumtemperatur kommen und gießt den Ansatz auf die Mischung aus 100 ml Eiswasser und 3 ml 37%ige Salzsäure. Man rührt gut durch, trennt die Chloroformphasen ab, wäscht sie mit 20 ml Wasser und 20 ml wäßriger Natriumhydrogencarbonatlösung, trocknet über wasserfr. Natriumsulfat und dampft unter reduziertem Druck ein. Man erhält das 3-(3.4-Dimethoxyphenyl)-propyl-1-methansulfonat in quantitativer Ausbeute (14 g) in Form eines gelblichen Öls.

b) 5-[3-(3.4-Dimethoxyphenyl)-propylamino]-5-desoxy-1.4;3.6-dianhydro-L-idit-2-nitrat

11 g (40 mmol) des zuvor erhaltenen Öls werden zusammen mit 80 ml Äthanol und 19 g (100 mmol) 5-Amino-5-desoxy-1.4;3.6-dianhydro-L-idit-2-nitrat 12 Stdn. unter Rückfluß erhitzt und dann unter reduziertem Druck eingedampft. Der Rückstand wird in 80 ml Chloroform aufgenommen, wobei überschüssiges Ausgangsamin als Hydrogenmethansulfonat ungelöst bleibt. Nach dem Absaugen und Waschen mit Chloroform befreit man das Filtrat von weiterem Ausgangsamin durch aufeinanderfolgende Extraktion 2mal mit 40 ml Wasser und 5mal mit je 20 ml 0.2molarer Essigsäure. Man wäscht mit verd. Natronlauge, trocknet die Chloroformphase über wasserfr. Natriumsulfat, dampft unter reduziertem Druck ein, löst die so erhaltene Rohbase in Äthanol, gibt 40 mmol Salzsäure zu und dampft erneut ein. Man erhält 10.8 g (26.7 mmol) 5-[3-(3.4-Dimethoxyphenyl)-propylamino-5-desoxy-1.4;3.6-dianhydro-L-idit-2-nitrat-Hydrochlorid. Umkristallisation aus Äthanol unter Zusatz von Aktivkohle ergibt 5.7 g (14.1 mmol) Reinprodukt.
Schmp. 160–163°; $[\alpha]_D^{25}$ 41.3 (c 0.33; Methanol)

Elementaranalyse: $C_{17}H_{24}N_2O_7 \times HCl$ (404.85)
Ber.: C (50.44), H (6.22), N (6.92), Cl (8.96)
Gef.: C (50.51), H (6.37), N (6.89), Cl (9.1)

Beispiel Nr. 32

5-[4-(4-Methoxyphenyl)-butylamino]-5-desoxy-1.4;3.6-dianhydro-L-idit-2-nitrat

Herstellung analog Beispiel 31a, b. 5 g (28 mmol) 4-(4-Methoxyphenyl)-1-butanol ergeben nach der Umsetzung mit Methansulfonsäurechlorid 7.2 g (28 mmol) öliges 4-(4-Methoxyphenyl)-butyl-1-methansulfonat. Diese, mit 14.3 g (75 mmol) 5-Amino-5-desoxy-1.4;3.6-dianhydro-L-idit-2-nitrat umgesetzt, ergeben 6.7 g (19 mmol) Rohbase. Nach Überführung in das Hydrochlorid und Umkristallisation aus Äthanol erhält man 2.8 g (7.2 mmol) 5-[4-(4-Methoxyphenyl)-butylamino-5-desoxy-1.4;3.6-dianhydro-L-idit-2-nitrat-Hydrochlorid.
Schmp. 182–4°; $[\alpha]_D^{25}$ 38; (c 0.2; Wasser)

Elementaranalyse: $C_{17}H_{24}N_2O_6$ × HCl (388.85)
Ber.: C (52.51), H (6.48), N (7.20), Cl (9.12)
Gef.: C (52.24), H (6.44), N (7.02), Cl (9.3)

Beispiel Nr. 33

5-[N-(2-Phenoxyäthyl)-N-methylamino]-5-desoxy-1.4;3.6-dianhydro-L-idit-2-nitrat

Herstellung analog Beispiel 20 durch Umsetzung von überschüssigem 5-Methylamino-5-desoxy-1.4;3.6-dianhydro-L-idit-2-nitrat mit 2-Phenoxy-1-bromäthan. Isolierung als Hydrochlorid. Umkristallisation aus Äthanol/Isopropanol.
Schmp. 153–6°; $[\alpha]_D^{25}$ 43.4; (c 0.5; Wasser)

Elementaranalyse: $C_{15}H_{20}N_2O_6$ × HCl (360.80)
Ber.: C (49.94), H (5.97), N (7.76), Cl (9.83)
Gef.: C (50.27), H (6.06), N (7.51), Cl (10.1)

Beispiel Nr. 34

5-[N-Methyl-N-(3-phenoxypropyl)-amino]-5-desoxy-1.4;3.6-dianhydro-L-idit-2-nitrat

Herstellung analog Beispiel 20 durch Umsetzung von überschüssigem 5-Methylamino-1.4;3.6-dianhydro-L-idit-2-nitrat mit 3-Phenoxy-1-brompropan. Isolierung als Hydrochlorid. Umkristallisation aus Isopropanol/Äthanol.
Schmp. 132–4°; $[\alpha]_D^{25}$ 35.5 (c 0.95; Wasser)

Elementaranalyse: $C_{16}H_{22}N_2O_6$ × HCl (374.82)
Ber.: C (51.27), H (6.18), N (7.47), Cl (9.46)
Gef.: C (51.03), H (6.30), N (7.24), Cl (9.3)

Beispiel Nr. 35

5-(2-Phenoxyäthylamino)-5-desoxy-1.4;3.6-dianhydro-L-idit-2-nitrat

Die Mischung aus 14.1 g (70 mmol) 2-Phenoxy-1-bromäthan, 250 ml Äthanol und 32 g (168 mmol) 5-Amino-5-desoxy-1.4;3.6-dianhydro-L-idit-2-nitrat wird 24 Stdn. unter Rückfluß gekocht. Nach dem Abkühlen saugt man überschüssiges Ausgangsamin-Hydrobromamid ab, wäscht mit Chloroform nach, dampft das Filtrat unter reduziertem Druck ein, nimmt den Rückstand in 80 ml Chloroform auf und gewinnt weiteres Ausgangsamin durch 4malige Extraktion mit je 50 ml Wasser und 3mal mit je 35 ml 0.2molarer Essigsäure zurück. Das Monosubstitutionsprodukt wird durch wiederholte Extraktion mit 0.5molarer Salzsäure (insgesamt 120 ml) aus der Chloroformphase extrahiert, wobei es zum Teil in Form des Hydrochlorids auskristallisiert. Einengen der salzsauren Extrakte ergibt weiteres Hydrochlorid. Nach Umkristallisation aus Methanol erhält man 11.9 g (34.3 mmol) 5-(2-Phenoxyäthylamino)-5-desoxy-1.4;3.6-dianhydro-L-idit-2-nitrat-Hydrochlorid.
Schmp. 182–6° (Z); $[\alpha]_D^{25}$ 50.7 (c 0.41; Wasser)

Elementaranalyse: $C_{14}H_{18}N_2O_6$ × HCl (346.77)
Ber.: C (48.49), H (5.52), N (8.08), Cl (10.22)
Gef.: C (48.57), H (5.63), N (8.04), Cl (10.8)

Beispiel Nr. 36

5-(3-Phenoxypropylamino)-5-desoxy-1.4;3.6-dianhydro-L-idit-2-nitrat

Herstellung analog Beispiel 35. Isolierung als Hydrochlorid. Ausbeute nach Umkristallisation aus Methanol/Wasser: 60.7%. Schmp. 205—7° (Z); $[\alpha]_D^{25}$ 42.8 (c 0.5; Wasser)

Elementaranalyse: $C_{15}H_{20}N_2O_6 \times HCl$ (360.80)
Ber.: C (49.94), H (5.87), N (7.76), Cl (9.83)
Gef.: C (49.88), H (5.90), N (7.62), Cl (10.0)

Beispiel Nr. 37

5-(4-Phenoxybutylamino)-5-desoxy-1.4;3.6-dianhydro-L-idit-2-nitrat

Herstellung analog Beispiel 35. Isolierung als Hydrochlorid. Ausbeute nach Umkristallisation aus Wasser: 48.4%.
Schmp. 162—5° (Z); $[\alpha]_D^{25}$ 41.7 (c 0.42; Wasser)

Elementaranalyse: $C_{16}H_{22}N_2O_6 \times HCl$ (374.82)
Ber.: C (51.27), H (6.18), N (7.47), Cl (9.46)
Gef.: C (51.74), H (6.35), N (7.56), Cl (9.6)

Beispiel Nr. 38

5-(5-Phenoxypentylamino)-5-desoxy-1.4;3.6-dianhydro-L-idit-2-nitrat

a) 5-Phenoxy-1-brompentan

Zur Lösung von 3.5 g (0.15 Mol) Natrium in 100 ml wasserfr. Äthanol gibt man 14.1 g (0.15 Mol) Phenol, kocht 5 Min. unter Rückfluß und gibt nach Abkühlen auf 40—50° schnell 100 ml (0.73 Mol) 1.5-Dibrompentan zu. Anschließend kocht man 5 Stdn. unter Rückfluß, saugt nach dem Abkühlen von Natriumbromid ab, zieht das Lösungsmittel unter reduziertem Druck ab, gibt 50 ml Chloroform zu und extrahiert die Lösung 4mal mit je 30 ml 1molarer Natronlauge. Man wäscht 1mal mit 50 ml Wasser, destilliert das Chloroform ab und fraktioniert über eine Vigreuxkolonne im Wasserstrahlvakuum. Bei 94° (11 Torr) gehen 99 g (0.43 Mol) 1.5-Dibrompentan über. Der Zwischenlauf von 94—160° wird verworfen. Zwischen 160° und 170° (10—12 Torr) destillieren 22.3 g (0.092 Mol) 5-Phenoxy-1-brompentan über.

b) 5-(5-Phenoxypentylamino)-5-desoxy-1.4;3.6-dianhydro-L-idit-2-nitrat

Die Mischung aus 14.6 g (60 mmol) des zuvor erhaltenen 5-Phenoxy-1-brompentans, 200 ml Äthanol und 28.5 g (150 mmol) 5-Amino-5-desoxy-1.4;3.6-dianhydro-L-idit-2-nitrat wird 24 Stdn. unter Rückfluß gekocht. Nach dem Abkühlen saugt man vom Niederschlag ab, kocht ihn mit Chloroform aus, vereinigt Chloroformextrakt und Filtrat und dampft unter reduziertem Druck ein. Die Lösung des Rückstands in 200 ml Chloroform wird zur Rückgewinnung überschüssigen Ausgangsamins 2mal mit je 100 ml Wasser und 4mal mit je 30 ml 0.3molarer Essigsäure extrahiert. Die Chloroformphase ergibt nach dem Waschen mit 30 ml 1molarer Natronlauge und Eindampfen unter reduziertem Druck 18.3 g (52 mmol) Rohprodukt, das in Äthanol gelöst, mit 52 ml 1molarer Salzsäure versetzt, erneut eingedampft und aus Isopropanol umkristallisiert wird. Man erhält 13.3 g (34.2 mmol) 5-(5-Phenoxypentylamino)-5-desoxy-1.4;3.6-dianhydro-L-idit-2-nitrat-Hydrochlorid.
Schmp. 129—131°; $[\alpha]_D^{25}$ 40.2 (c 0.41; Wasser)

Elementaranalyse: $C_{17}H_{24}N_2O_6 \times HCl$ (388.85)
Ber.: C (52.51), H (6.48), N (7.20), Cl (9.12)
Gef.: C (52.44), H (6.69), N (7.08), Cl (9.6)

Beispiel Nr. 39

5-(6-Phenoxyhexylamino)-5-desoxy-1.4;3.6-dianhydro-L-idit-2-nitrat

Herstellung analog Beispiel 38 durch Reaktion von Natriumphenolat mit 1.6-Dibromhexan zu 6-Phenoxy-1-bromhexan ($Kp_{11}$ = 172–5°; Ausbeute 77%), das mit überschüssigem 5-Amino-5-desoxy-1.4;3.6-dianhydro-L-idit-2-nitrat umgesetzt wird. Isolierung als Hydrochlorid. Ausbeute nach Umkristallisation aus Isopropanol: 53%.
Schmp. 114–6°; $[\alpha]_D^{25}$ 38.5 (c 0.39; Wasser)

Elementaranalyse: $C_{18}H_{26}N_2O_6$ × HCl (402.88)
Ber.: C (53.66), H (6.76), N (6.95), Cl (8.80)
Gef.: C (53.48), H (6.96), N (6.82), Cl (9.3)

Beispiel Nr. 40

5-(7-Phenoxyheptylamino)-5-desoxy-1.4;3.6-dianhydro-L-idit-2-nitrat

Herstellung analog Beispiel 38 durch Reaktion von Natriumphenolat mit 1.7-Dibromheptan zu 7-Phenoxy-1-bromheptan ($Kp_{11}$ = 184–190°; Ausbeute 71%), das mit überschüssigem 5-Amino-5-desoxy-1.4;3.6-dianhydro-L-idit-2-nitrat umgesetzt wird. Isolierung als Hydrochlorid.
Ausbeute nach Umkristallisation aus Äthanol/Isopropanol: 59%. Schmp. 136–8°; $[\alpha]_D^{25}$ 43.9 (c 0.3; Methanol).

Elementaranalyse: $C_{19}H_{28}N_2O_6$ × HCl (416.90)
Ber.: C (54.74), H (7.01), N (6.72), Cl (8.50)
Gef.: C (54.78), H (7.15), N (6.66), Cl (9.5)

Beispiel Nr. 41

5-(8-Phenoxyoctylamino)-5-desoxy-1.4;3.6-dianhydro-L-idit-2-nitrat

Herstellung analog Beispiel 38 durch Reaktion von Natriumphenolat mit 1.8-Dibromoctan zu 8-Phenoxy-1-bromoctan ($Kp_{11}$ = 196–202°; Ausbeute 75%), das mit überschüssigem 5-Amino-5-desoxy-1.4;3.6-dianhydro-L-idit-2-nitrat umgesetzt wird. Isolierung als Hydrochlorid. Ausbeute nach Umkristallisation aus Äthanol/Isopropanol: 62%.
Schmp. 117–9°; $[\alpha]_D^{25}$ 35.3 (c 0.3; Methanol).

Elementaranalyse: $C_{20}H_{30}N_2O_6$ × HCl (430.93)
Ber.: C (55.74), H (7.25), N (6.50), Cl (8.23)
Gef.: C (55.89), H (7.42), N (6.40), Cl (8.6)

Beispiel Nr. 42

2-(3-Phenoxypropylamino)-2-desoxy-1.4;3.6-dianhydro-D-glucit-5-nitrat

Das Gemisch aus 8.8 g (40 mmol) 98%igem 3-Phenoxy-1-brompropan, 130 ml Äthanol und 19 g (100 mmol) 2-Amino-2-desoxy-1.4;3.6-dianhydro-D-glucit-5-nitrat (Herstellung siehe Beispiel 3c) wird 24 Stunden unter Rückfluß gekocht und dann unter reduziertem Druck eingedampft. Der Rückstand wird in 50 ml Chloroform aufgenommen und nacheinander 2mal mit je 50 ml Wasser, 3mal mit je 30 ml 0.4molarer Essigsäure und 5mal mit je 30 ml 0.1molarer Salzsäure extrahiert; bei den Ausschüttelungen mit Salzsäure fällt ein Teil des Produkts als Hydrochlorid aus und wird abgesaugt.
Die Essigsäureextrakte 2 und 3 werden mit den Salzsäureextrakten vereinigt, mit verdünnter Natronlauge alkalisch gestellt und mit Chloroform extrahiert. Der Chloroformextrakt ergibt nach dem Trocknen über wasserfreiem Natriumsulfat und Eindampfen 7 g (21.7 mmol) Rohprodukt, das mit 22 ml 1molarer Salzsäure in das Hydrochlorid übergeführt und erneut eingedampft wird. Man vereinigt mit dem zuvor gewonnenen Hydrochlorid, kristallisiert 2mal aus Äthanol um und erhält 7.3 g (20.2 mmol) 2-(3-Phenoxypropylamino)-2-desoxy-1.4;3.6-dianhydro-D-glucit-5-nitrat-Hydrochlorid.
Schmp. 165–8° (Z); $[\alpha]_D^{25}$ 94.6 (c 0.43; Wasser)

Elementaranalyse: $C_{15}H_{20}N_2O_6$ × HCl (360.80)
Ber.: C (49.94), H (5.87), N (7.76), Cl (9.83)
Gef.: C (50.08), H (5.92), N (7.68), Cl (10.3)

Beispiel Nr. 43

2-(4-Phenylbutylamino)-2-desoxy-1.4;3.6-dianhydro-D-glucit-5-nitrat

Das Gemisch aus 6.5 g (30.5 mmol) 4-Phenyl-1-brombutan, 100 ml Äthanol und 14.4 g (76 mmol) 2-Amino-2-desoxy-1.4;3.6-dianhydro-D-glucit-5-nitrat (Herstellung siehe Beispiel 3c) wird 17 Stdn. unter Rückfluß gekocht und dann unter reduziertem Druck eingedampft. Der Rückstand wird in 50 ml Chloroform aufgenommen und nacheinander 2mal mit je 50 ml Wasser und 2mal mit je 30 ml 0.2molarer Salzsäure extrahiert. Die wäßrigen und salzsauren Extrakte werden zur Rückgewinnung überschüssigen Ausgangsamins verwendet. Die Chloroformphase wird mit 50 ml verdünnter Natronlauge gewaschen, unter reduziertem Druck eingedampft und der Rückstand durch Lösen in Äthanol und Zugabe der äquimolaren Menge Salzsäure in das Hydrochlorid übergeführt. Man dampft erneut ein, kristallisiert 2mal aus Äthanol/Isopropanol um und erhält 5.33 g (14.9 mmol) 2-(4-Phenylbutylamino)-2-desoxy-1.4;3.6-dianhydro-D-glucit-5-nitrat-Hydrochlorid.
Schmp. 154—8° (Z); $[\alpha]_D^{25}$ 91.4 (c 0.4; Wasser)

Elementaranalyse: $C_{16}H_{22}N_2O_6 \times HCl$ (358.82)
Ber.: C (53.56), H (6.46), N (7.81), Cl (9.88)
Gef.: C (53.64), H (6.50), N (7.87), Cl (10.2)

Beispiel Nr. 44

5-[2-Hydroxy-3-(4-methoxyphenoxy)-propylamino]
-5-desoxy-1.4;3.6-dianhydro-L-idit-2-nitrat

Zur unter Rückfluß siedenden Lösung von 7.6 g (40 mmol) 5-Amino-5-desoxy-1.4;3.6-dianhydro-L-idit-2-nitrat in 50 ml Äthanol tropft man innerhalb 2—3 Stdn. die Lösung von 5.4 g (30 mmol) 1.2-Epoxy-3-(4-methoxyphenoxy)-propan in 50 ml Äthanol, kocht weitere 24 Stdn. unter Rückfluß und dampft unter reduziertem Druck ein. Die Lösung des Rückstands in 50 ml Chloroform wird zur Abtrennung überschüssigen Ausgangsamins 3mal mit je 20 ml 0.2molarer Essigsäure extrahiert und mit verdünnter Natronlauge gewaschen. Anschließend extrahiert man mehrfach mit 20-ml-Portionen 1molarer Salzsäure, bis alles Monosubstitutionsprodukt in die Salzsäurephasen übergegangen ist. Diese werden unter reduziertem Druck eingedampft und aus Äthanol/Isopropanol umkristallisiert. Man erhält 5.62 g (13.8 mmol) 5-[2-Hydroxy-3-(4-methoxyphenoxy)-propylamino]-5-desoxy-1.4;3.6-dianhydro-L-idit-2-nitrat-Hydrochlorid.
Schmp. 174—6° (Z); $[\alpha]_D^{25}$ 46.8 (c 0.98; Wasser)

Elementaranalyse: $C_{16}H_{22}N_2O_8 \times HCl$ (406.82)
Ber.: C (47.24), H (5.70), N (6.89), Cl (8.71)
Gef.: C (47.66), H (5.84), N (6.76), Cl (9.1)

Das als Ausgangsprodukt verwendete Epoxid-Derivat kann man z. B. folgendermaßen herstellen:
Zur Mischung aus 82 g (0.66 Mol) 4-Methoxyphenol und 62 g (0.67 Mol) Epichlorhydrin werden unter Rühren bei 50°C Innentemperatur innerhalb 2—3 Stdn. 89 g (0.67 Mol) 30%ige Natronlauge zugetropft. Man rührt bis zur vollständigen Umsetzung bei 50° weiter, läßt abkühlen, gibt 200 ml Chloroform zu, trennt die organische Phase ab, extrahiert die wäßrige Phase noch zweimal mit je 50 ml Chloroform und wäscht die vereinigten Chloroformphasen 3mal mit verdünnter Natronlauge. Die Chloroformphase wird nach dem Trocknen über wasserfr. Natriumsulfat eingedampft und der ölige Rückstand im Vakuum über eine Vigreux-Kolonne fraktioniert destilliert.
Zwischen 154° und 158° gehen bei 8 Torr 63 g (0.35 Mol) 1.2-Epoxy-3-(4-methoxyphenoxy)-propan über.

Beispiel Nr. 45

5-[2-Hydroxy-3-(3-methoxyphenoxy)-propylamino]-
5-desoxy-1.4;3.6-dianhydro-L-idit-2-nitrat

Herstellung analog Beispiel 44 aus 1.2-Epoxy-3-(3-methoxyphenoxy)-propan ($Kp_9$ = 158—161°) und überschüssigem 5-Amino-5-desoxy-1.4;3.6-dianhydro-L-idit-2-nitrat. Isolierung als Hydrochlorid. Ausbeute nach Umkristallisation aus Äthanol/Isopropanol : 43%.
Schmp. 172—4° (Z); $[\alpha]_D^{25}$ 41.9 (c 0.4; Äthanol)

Elementaranalyse: $C_{19}H_{22}N_2O_8 \times HCl$ (406.82)
Ber.: C (47.24), H (5.70), N (6.89), Cl (8.71)
Gef.: C (47.46), H (5.87), N (6.70), Cl (9.2)

Beispiel Nr. 46

5-[2-Hydroxy-3-(2-methoxyphenoxy)-propylamino]-
5-desoxy-1.4;3.6-dianhydro-L-idit-2-nitrat

Herstellung analog Beispiel 44 aus 1.2-Epoxy-3-(2-methoxyphenoxy)-propan ($K_8$ = 146–148°) und überschüssigem 5-Amino-5-desoxy-1.4;3.6-dianhydro-L-idit-2-nitrat. Isolierung als Hydrochlorid. Ausbeute nach Umfällen mit Äther aus Methylenchlorid 45%.
Schmelzbereich: 80–104°. $[\alpha]_D^{25}$ 34 (c 1; Wasser)

Elementaranalyse nach Trocknen bei 60°/0.1 Torr: $C_{16}H_{22}N_2O_8 \times HCl$ (406.82)
Ber.: C (47.24), H (5.70), N (6.89), Cl (8.71)
Gef.: C (46.72), H (5.69), N (6.60), Cl (9.6)
(Die Analysensubstanz enthält noch ca. 1.5% Dichlormethan).

Beispiel Nr. 47

5-[2-Hydroxy-3-(2-äthoxyphenoxy)-propylamino]-
5-desoxy-1.4;3.6-dianhydro-L-idit-2-nitrat

Herstellung analog Beispiel 44 aus 1.2-Epoxy-3-(2-äthoxyphenoxy)-propan ($Kp_{0.8}$ = 120–121°) und überschüssigem 5-Amino-5-desoxy-1.4;3.6-dianhydro-L-idit-2-nitrat. Reinigung durch Säulenchromatographie an Kieselgel mit Chloroform/Methanol 95/5 als Eluens. Die Fraktionen mit Rf = 0.62 (Kieselgel-Fertigplatten f 254; Chloroform/Methanol 9/1) werden vereinigt, unter reduziertem Druck eingedampft, in das Hydrochlorid übergeführt und aus Dichlormethan mit Äther umgefällt. Ausbeute 10%.
Schmp. 55°; $[\alpha]_D^{25}$ 31.5 (c 1; Wasser)
Elementaranalyse nach Trocknung bei 40°/1 Torr: $C_{17}H_{22}N_2O_8 \times HCl$ (420.85)
Ber.: C (48.52), H (5.99), N (6.66), Cl (8.42)
Gef.: C (47.07), H (5.89), N (6.21), Cl (10.7)
(Die Analysensubstanz enthält noch ca. 3% Dichlormethan)

Beispiel Nr. 48

5-[2-Hydroxy-3-(2-allyloxyphenoxy)-propylamino]-
5-desoxy-1.4;3.6-dianhydro-L-idit-2-nitrat

Herstellung analog Beispiel 44 durch Umsetzung von 1.2-Epoxy-3-(2-allyloxyphenoxy)-propan mit überschüssigem 5-Amino-5-desoxy-1.4;3.6-dianhydro-L-idit-2-nitrat. Reinigung durch Säulenchromatographie an Kieselgel mit Chloroform/Methanol 9/1 als Eluens. Die Fraktionen mit Rf = 0.64 (Kieselgel-Fertigplatten F 254; Chloroform/Methanol 9/1) werden vereinigt und ergeben nach dem Eindampfen das reine Produkt in Form der freien Base. Ausbeute: 20%.
Schmp. 106–109° (aus Dichlormethan/n-Pentan). $[\alpha]_D^{25}$ 20 (c 0.5; Äthanol)

Elementaranalyse: $C_{18}H_{24}N_2O_8$ (396.40)
Ber.: C (54.54), H (6.10), N (7.07)
Gef.: C (54.96), H (6.15), N (7.04)

Beispiel Nr. 49

5-[2-Hydroxy-3-(2-allylphenoxy)-propylamino]-
5-desoxy-1.4;3.6-dianhydro-L-idit-2-nitrat

Herstellung analog Beispiel 44 durch Umsetzung von 1.2-Epoxy-3-(2-allylphenoxy)-propan ($Kp_{0.6}$ = 99–100°) mit überschüssigem 5-Amino-5-desoxy-1.4;3.6-dianhydro-L-idit-2-nitrat. Reinigung durch Säulenchromatographie an Kieselgel mit Chloroform/Methanol 9/1 als Eluens. Die Fraktionen mit Rf = 0.62 (Kieselgel-Fertigplatten F 254; Chloroform/Methanol 9/1) ergeben nach Eindampfen

unter reduziertem Druck, Überführung in das Hydrochlorid und zweimaligem Umfällen aus Chloroform mit Äther das Reinprodukt in 36% Ausbeute.
Schmelzbereich 74–102°. $[\alpha]_D^{25}$ 24.6 (c 1; Wasser).

Elementaranalyse nach Trocknen bei 60°/1 Torr: $C_{18}H_{24}N_2O_7 \times HCl$ (416.86)
Ber.: C (51.86), H (6.05), N (6.72), Cl (8.50)
Gef.: C (51.23), H (6.12), N (6.28), Cl (9.1)
(Die Analysensubstanz enthält noch ca. 1% Chloroform)

Beispiel Nr. 50

5-[2-Hydroxy-3-(2-cyanophenoxy)-propylamino]-5-desoxy-1.4;3.6-dianhydro-L-idit-2-nitrat

Herstellung analog Beispiel 44 durch Umsetzung von 1.2-Epoxy-3-(2-cyanophenoxy)-propan mit überschüssigem 5-Amino-4-desoxy-1.4;3.6-dianhydro-L-idit-2-nitrat. Reinigung durch Säulenchromatographie an Kieselgel mit Chloroform/Methanol 9/1 als Eluens. Die Fraktionen mit Rf = 0.56 (Kieselgel-Fertigplatten F 254; Chloroform/Methanol 9/1) ergeben nach Eindampfen unter reduziertem Druck, Überführung in das Hydrochlorid und Umfällen aus Chloroform mit Äther/Petroläther das Reinprodukt in 33% Ausbeute.
Schmelzbereich 75–107°; $[\alpha]_D^{25}$ 29.8 (c 1; Wasser).

Elementaranalyse nach Trocknung bei 60°/1 Torr: $C_{16}H_{19}N_3O_7 \times HCl$ (401.81)
Ber.: C (47.83), H (5.02), N (10.46), Cl (8.82)
Gef.: C (47.04), H (5.05), N (9.79), Cl (10.5)
(Die Analysensubstanz enthält noch ca. 2.2% Chloroform).

Beispiel Nr. 51

5-[2-Hydroxy-3-(3-tolyloxy)-propylamino]-5-desoxy-1.4;3.6-dianhydro-L-idit-2-nitrat

Herstellung analog Beispiel 44 durch Umsetzung von 1.2-Epoxy-3-(3-tolyloxy)-propan ($Kp_{10}$ =129–130°) mit überschüssigem 5-Amino-5-desoxy-1.4;3.6-dianhydro-L-idit-2-nitrat. Isolierung als Hydrochlorid. Ausbeute nach Umkristallisation aus Wasser: 36%.
Schmp. 171–5°; $[\alpha]_D^{25}$ 48.3 (c 0.52; Methanol)

Elementaranalyse: $C_{16}H_{22}N_2O_7 \times HCl$ (390.82)
Ber.: C (49.17), H (5.93), N (7.17), Cl (9.07)
Gef.: C (49.59), H (6.06), N (7.62), Cl (9.5)

Beispiel Nr. 52

5-[2-Hydroxy-3-(3-trifluormethylphenoxy)-propylamino]-5-desoxy-1.4;3.6-dianhydro-L-idit-2-nitrat

Herstellung analog Beispiel 44 durch Umsetzung von 1.2-Epoxy-3-(3-trifluormethylphenoxy)-propan ($Kp_{17}$ 127–131°) mit überschüssigem 5-Amino-5-desoxy-1.4;3.6-dianhydro-L-idit-2-nitrat. Isolierung als Hydrochlorid. Ausbeute nach Umkristallisation aus Methanol/Chloroform: 29%.
Schmp. 135–8°; $[\alpha]_D^{25}$ 36 (c 1; Methanol)

Elementaranalyse: $C_{16}H_{19}F_3N_2O_7 \times HCl$ (440.80)
Ber.: C (43.21), H (4.53), N (6.30), Cl (7.97) F (12.81)
Gef.: C (43.18), H (4.60), N (6.23), Cl (8.5)

Beispiel Nr. 53

5-[2-Hydroxy-3-(4-carbamoylmethylphenoxy)-propylamino]-5-desoxy-1.4;3.6-dianhydro-L-idit-2-nitrat

Herstellung analog Beispiel 44 durch Umsetzung von 4-(2.3-Epoxypropoxy)-phenylessigsäureamid

(Schmp. = 169.5–171°; aus Aceton) mit überschüssigem 5-Amino-5-desoxy-1.4;3.6-dianhydro-L-idit-2-nitrat. Isolierung als Hydrochlorid. Ausbeute nach Umkristallisation aus Äthanol/Chloroform/Äther: 10%.
Schmp. 177–8° (Z); $[\alpha]_D^{25}$ 31.3 (c 1; Wasser)

Elementaranalyse: $C_{17}H_{23}N_3O_8 \times HCl$ (433.85)
Ber.: C (47.06), H (5.58), N (9.96), Cl (8.17)
Gef.: C (47.06), H (5.68), N (9.39), Cl (8.3)

Beispiel Nr. 54

5-[2-Hydroxy-3-(1-naphthyloxy)-propylamino]-5-desoxy-1.4;3.6-dianhydro-L-idit-2-nitrat

Herstellung analog Beispiel 44 durch Umsetzung von 1.2-Epoxy-3-(1-naphthyloxy)-propan ($Kp_{11}$ =190–195°) mit überschüssigem 5-Amino-5-desoxy-1.4;3.6-dianhydro-L-idit-2-nitrat. Isolierung als Hydrochlorid. Ausbeute nach Umkristallisation aus Äthanol/Chloroform: 49%.
Schmp. 186–190° (Z); $[\alpha]_D^{25}$ 41 (c 0.5; Methanol)

Elementaranalyse: $C_{19}H_{22}N_2O_7 \times HCl$ (426.85)
Ber.: C (53.46), H (5.43), N (6.56), Cl (8.31)
Gef.: C (53.65), H (5.43), N (6.99), Cl (8.9)

Beispiel Nr. 55

5-[2-Hydroxy-3-(1-naphthyloxy)-propylamino]-5-desoxy-1.4;3.6-dianhydro-L-idit-2-nitrat

Herstellung analog Beispiel 44 durch Umsetzung von 1.2-Epoxy-3-(1-naphthyloxy)-propan mit überschüssigem 5-Amino-5-desoxy-1.4;3.6-dianhydro-D-glucit-2-nitrat (hergestellt nach Beispiel 2) in siedendem n-Butanol. Isolierung als Hydrochlorid. Ausbeute nach zweimaliger Umkristallisation aus Äthanol/Chloroform: 9%.
Schmp. 183–187° (Z); $[\alpha]_D^{25}$ 43.7 (c 0.5; Dimethylformamid).

Elementaranalyse: $C_{19}H_{22}N_2O_7 \times HCl$ (426.85)
Ber.: C (53.46), H (5.43), N (6.56), Cl (8.31)
Gef.: C (53.20), H (5.53), N (6.49), Cl (8.5)

Beispiel Nr. 56

5-[2-Hydroxy-3-(1-oxo-1.2.3.4-tetrahydro-5-naphthyloxy)-propylamino]-5-desoxy-1.4;3.6-dianhydro-L-idit-2-nitrat

Herstellung analog Beispiel 44 durch Umsetzung von 5-(2.3-Epoxypropoxy)-1-tetralon (Schmp. 55°; aus Hexan) mit überschüssigem 5-Amino-5-desoxy-1.4;3.6-dianhydro-L-idit-2-nitrat. Isolierung als Hydrochlorid. Ausbeute nach Umfällen aus Äthanol mit Äther: 51%.
Schmp.: 175–6° (Z); $[\alpha]_D^{25}$ 14.8 (c 1; Äthanol)

Elementaranalyse: $C_{19}H_{24}N_2O_8 \times HCl$ (444.87)
Ber.: C (51.30), H (5.66), N (6.30), Cl (7.97)
Gef.: C (50.88), H (5.79), N (6.20), Cl (8.0)

Beispiel Nr. 57

5-[2-Hydroxy-3-(5.6.7.8-tetrahydro-1-naphthyloxy)-propylamino]-5-desoxy-1.4;3.6-dianhydro-L-idit-2-nitrat

Herstellung analog Beispiel 44 durch Umsetzung von 5-(2.3-Epoxypropoxy)-tetralin mit überschüssigem 5-Amino-5-desoxy-1.4;3.6-dianhydro-L-idit-2-nitrat. Isolierung als Hydrochlorid. Ausbeute nach Umkristallisation aus Wasser: 46%.
Schmp. 161–4° (Z); $[\alpha]_D^{25}$ 22 (c 1; Äthanol)

Elementaranalyse: $C_{19}H_{26}N_2O_7 \times HCl$ (430.89)
Ber.: C (52.96), H (6.32), N (6.50), Cl (8.23)
Gef.: C (52.72), H (6.55), N (6.52), Cl (8.3)

## Beispiel Nr. 58

### 5-[3-(3-Trifluormethylphenoxy)-propylamino-5-desoxy-1.4;3.6-dianhydro-L-idit-2-nitrat

Die Mischung aus 8.5 g (30 mmol) 3-(3-Trifluormethylphenoxy)-1-brompropan, 100 ml Äthanol und 17.1 g (90 mmol) 5-Amino-5-desoxy-1.4;3.6-dianhydro-L-idit-2-nitrat wird 20 Stdn. unter Rückfluß gekocht. Nach dem Abkühlen saugt man auskristallisiertes überschüssiges Ausgangsamin (als Hydrobromid vorliegend) ab, dampft das Filtrat unter reduziertem Druck ein, nimmt den Rückstand in 100 ml Chloroform auf, wäscht zur Entfernung restlichen Ausgangsamins 2mal mit je 70 ml Wasser, 1mal mit 30 ml 0.3molarer Essigsäure und 1mal mit 30 ml 1molarer Natronlauge, trocknet über wasserfr. Natriumsulfat und dampft die Chloroformlösung unter vermind. Druck ein. Man erhält 11 g (28 mmol) Rohbase, die in das Hydrochlorid übergeführt und zweimal aus Äthanol umkristallisiert werden. Ausbeute an reinem Hydrochlorid: 8.77 g (20.5 mmol).
Schmp. 172–4° (Z); $[\alpha]_D^{25}$ 36.5 (c 0.43; Wasser)

Elementaranalyse: $C_{16}H_{19}F_3N_2O_6 \times HCl$ (428.80)
Ber.: C (44.82), H (4.70), N (6.53), Cl (8.27)
Gef.: C (44.91), H (4.73), N (6.41), Cl (8.4)

Das als Ausgangsprodukt verwendete 3-(3-Trifluormethylphenoxy)-1-brompropan kann folgendermaßen erhalten werden: In 150 ml wasserfr. Äthanol löst man 3.6 g (157 mmol) Natrium auf, gibt 24.5 g (151 mmol) 98%iges 3-Hydroxybenzoetrifluorid zu, kocht zur vollständigen Bildung des Phenolats kurz auf, läßt auf ca. 50° abkühlen, gibt 50 ml (493 mmol) 1.3-Dibrompropan zu und kocht bis zu vollständigen Umsetzung unter Rückfluß (ca. 10 Stdn.). Nach dem Abkühlen und Abfiltrieren des Natriumbromids dampft man unter reduziertem Druck das Äthanol ab, gibt 100 ml Dichlormethan zu, wäscht nicht umgesetztes 3-Hydroxybenzotrifluorid mit verdünnter Natronlauge heraus, trocknet die Chloroformlösung über wasserfr. Natriumsulfat, destilliert das Chloroform ab und fraktioniert den Rückstand im Vakuum über eine Vigreuxkolonne. Bei 10–11 Torr destillieren zwischen 124° und 132° insgesamt 25.2 g (89 mmol) 3-(3-Trifluormethylphenoxy)-1-brompropan über.

## Beispiel Nr. 59

### 5-[3-4-Tolyloxy)-propylamino]-5-desoxy-1.4;3.6-dianhydro-L-idit-2-nitrat

Herstellung analog Beispiel 58 durch Umsetzung von 3-(4-Tolyloxy)-1-brompropan ($Kp_{0.2}$ = 92–4°) mit überschüssigem 5-Amino-5-desoxy-1.4;3.6-dianhydro-L-idit-2-nitrat. Isolierung als Hydrochlorid. Ausbeute nach Umkristallisation aus Methanol: 25%.
Schmp. 202–3° (Z); $[\alpha]_D^{25}$ 43.8 (c 0.4; Wasser)

Elementaranalyse: $C_{16}H_{22}N_2O_6 \times HCl$ (374.82)
Ber.: C (51.27), H (6.18), N (7.47), Cl (9.46)
Gef.: C (51.22), H (6.19), N (7.44), Cl (9.7)

## Beispiel Nr. 60

### 5-[3-(4-Fluorphenoxy)-propylamino]-5-desoxy-1.4;3.6-dianhydro-L-idit-2-nitrat

Herstellung analog Beispiel 58 durch Umsetzung von 3-(4-Fluorphenoxy)-1-brompropan ($Kp_{0.2}$ =82–88°) mit überschüssigem 5-Amino-5-desoxy-1.4;3.6-dianhydro-L-idit-2-nitrat. Isolierung als Hydrochlorid. Ausbeute nach Umkristallisation aus Methanol: 55%.
Schmp. 198–201° (Z); $[\alpha]_D^{25}$ 41.1 (c 0.4; Wasser)

Elementaranalyse: $C_{15}H_{19}FN_2O_6 \times HCl$ (378.79)
Ber.: C (47.56), H (5.32), N (7.39), Cl (9.36)
Gef.: C (47.90), H (5.50), N (7.44), Cl (9.3)

Beispiel Nr. 61

5-[3-(1-Naphthyloxy)-propylamino]-5-desoxy-1.4;3.6-dianhydro-L-idit-2-nitrat

Herstellung analog Beispiel 58 durch Umsetzung von 3-(1-Naphthyloxy)-1-brompropan ($Kp_{15}$ =157°) mit überschüssigem 5-Amino-5-desoxy-1.4;3.6-dianhydro-L-idit-2-nitrat. Isolierung als Hydrochlorid. Ausbeute nach Umkristallisation aus Methanol: 32.3%.
Schmp. 192—5° (Z); $[\alpha]_D^{25}$ 34.3 (c 0.18; Wasser)

Elementaranalyse: $C_{19}H_{22}N_2O_6 \times HCl$ (410.85)
Ber.: C (55.55), H (5.64), N (6.82), Cl (8.63)
Gef.: C (55.68), H (5.72), N (6.73), Cl (9.0)

Beispiel Nr. 62

5-[3-(4-Methoxyphenoxy)-propylamino]-5-desoxy-1.4;3.6-dianhydro-L-idit-2-nitrat

Herstellung analog Beispiel 58 durch Umsetzung von 3-(4-Methoxyphenoxy)-1-brompropan mit überschüssigem 5-Amino-5-desoxy-1.4;3.6-dianhydro-L-idit-2-nitrat. Isolierung als Hydrochlorid. Ausbeute nach Umkristallisation aus Methanol/Wasser: 24%.
Schmp. 185—8° (Z); $[\alpha]_D^{25}$ 39.2 (c 0.4; Wasser)

Elementaranalyse: $C_{16}H_{22}N_2O_7 \times HCl$ (390.82)
Ber.: C (49.17), H (5.93), N (7.17), Cl (9.07)
Gef.: C (49.02), H (6.10), N (7.06), Cl (9.4)

Beispiel Nr. 63

5-[3-(3-Methoxyphenoxy)-propylamino]-5-desoxy-1.4;3.6-dianhydro-L-idit-2-nitrat

Herstellung analog Beispiel 58 durch Umsetzung von 3-(3-Methoxyphenoxy)-1-brompropan mit überschüssigem 5-Amino-5-desoxy-1.4;3.6-dianhydro-L-idit-2-nitrat. Isolierung als Hydrochlorid. Ausbeute nach Umkristallisation aus Äthanol/Isopropanol: 25.7%.
Schmp.: 187—191° (Z); $[\alpha]_D^{25}$ 39.5 (c 0.4; Wasser)

Elementaranalyse: $C_{16}H_{22}N_2O_7 \times HCl$ (390.82)
Ber.: C (49.17), H (5.93), N (7.17), Cl (9.07)
Gef.: C (49.15), H (6.06), N (7.05), Cl (8.9)

Beispiel Nr. 64

5-[3-(2-Methoxyphenoxy)-propylamino]-5-desoxy-1.4;3.6-dianhydro-L-idit-2-nitrat

Herstellung analog Beispiel 58 durch Umsetzung von 3-(2-Methoxyphenoxy)-1-brompropan mit überschüssigem 5-Amino-5-desoxy-1.4;3.6-dianhydro-L-idit-2-nitrat. Isolierung als Hydrochlorid. Umkristallisation aus Chloroform/Benzol.
Schmp. 130—3°; $[\alpha]_D^{25}$ 38.5 (c 0.47; Wasser)

Elementaranalyse: $C_{16}H_{22}N_2O_7 \times HCl$ (390.82)
Ber.: C (49.17), H (5.93), N (7.17), Cl (9.07)
Gef.: C (49.09), H (6.00), N (7.15), Cl (9.3)

Beispiel Nr. 65

5-[3-(2.6-Dimethoxyphenoxy)-propylamino]-5-desoxy-1.4;3.6-dianhydro-L-idit-2-nitrat

Herstellung analog Beispiel 58 durch Umsetzung von 3-(2.6-Dimethoxyphenoxy)-1-brompropan ($Kp_{0.06}$ = 127—132°) mit überschüssigem 5-Amino-5-desoxy-1.4;3.6-dianhydro-L-idit-2-nitrat. Isolierung als Hydrochlorid-Halbhydrat. Ausbeute 44%.
Schmp. 110—111° (aus Äthylacetat/n-Pentan). $[\alpha]_D^{25}$ 32.5 (c 0.2; Wasser)

Elementaranalyse: $C_{17}H_{24}N_2O_8 \times \frac{1}{2} H_2O \times HCl$ (429.86)
Ber.: C (47.50), H (6.10), N (6.52), Cl (8.25)
Gef.: C (47.50), H (5.96), N (6.45), Cl (9.1)

Beispiel Nr. 66

5-[3-(3.5-Dimethoxyphenoxy)-propylamino]-5-desoxy-1.4;3.6-dianhydro-L-idit-2-nitrat

Herstellung analog Beispiel 58 durch Umsetzung von 3-(3.5-Dimethoxyphenoxy)-1-brompropan ($Kp_{0.15}$ = 140–152°) mit überschüssigem 5-Amino-5-desoxy-1.4;3.6-dianhydro-L-idit-2-nitrat. Isolierung als Hydrochlorid. Ausbeute nach dreimaliger Umkristallisation aus Äthanol: 45%.
Schmp. 186–7° (Z); $[\alpha]_D^{25}$ 36.1 (c 0.4; Wasser).

Elementaranalyse: $C_{17}H_{24}N_2O_8 \times HCl$ (420.85)
Ber.: C (48.52), H (5.99), N (6.66), Cl (8.42)
Gef.: C (48.39), H (6.10), N (6.42), Cl (8.8)

Beispiel Nr. 67

5-[3-(2.3-Dimethoxyphenoxy)-propylamino]-5-desoxy-1.4;3.6-dianhydro-L-idit-2-nitrat

Herstellung analog Beispiel 58 durch Umsetzung von 3-(2.3-Dimethoxyphenoxy)-1-brompropan ($Kp_{0.06}$ = 135–138°) mit überschüssigem 5-Amino-5-desoxy-1.4;3.6-dianhydro-L-idit-2-nitrat. Isolierung als Hydrochlorid. Ausbeute nach Umkristallisation aus Äthanol/Äther: 42.6%.
Schmp.: 144–5° (Z); $[\alpha]_D^{25}$ 36.6 (c 0.4; Wasser).

Elementaranalyse: $C_{17}H_{24}N_2O_8 \times HCl$ (420.85)
Ber.: C (48.52), H (5.99), N (6.66), Cl (8.42)
Gef.: C (48.62), H (6.16), N (6.57), Cl (8.8)

Beispiel Nr. 68

5-[3-(4-Chlorphenoxy)-propylamino]-5-desoxy-1.4;3.6-dianhydro-L-idit-2-nitrat

Herstellung analog Beispiel 58 durch Umsetzung von 3-(4-Chlorphenoxy)-1-brompropan ($Kp_{11}$ =152–5° mit überschüssigem 5-Amino-5-desoxy-1.4;3.6-dianhydro-L-idit-2-nitrat. Isolierung als Hydrochlorid. Ausbeute nach Umkristallisation aus Äthanol/Isopropanol: 63.3%.
Schmp. 179–181° (Z); $[\alpha]_D^{25}$ (c 0.42; Wasser).

Elementaranalyse: $C_{15}H_{19}ClN_2O_6 \times HCl$ (395.25)
Ber.: C (45.58), H (5.10), N (7.09), Cl (17.94)
Gef.: C (45.57), H (5.18), N (6.91), Cl (18.3)

Beispiel Nr. 69

5-[3-(2-Chlorphenoxy)-propylamino]-5-desoxy-1.4;3.6-dianhydro-L-idit-2-nitrat

Herstellung analog Beispiel 58 durch Umsetzung von 3-(2-Chlorphenoxy)-1-brompropan ($Kp_{0.8}$ =115–116°) mit überschüssigem 5-Amino-5-desoxy-1.4;3.6-dianhydro-L-idit-2-nitrat. Isolierung als Hydrochlorid. Ausbeute nach Umkristallisation aus Äthanol: 28.5%.
Schmp. 154–6°; $[\alpha]_D^{25}$ 34.8 (c 0.39; Wasser).

Elementaranalyse: $C_{15}H_{19}ClN_2O_6 \times HCl$ (395.25)
Ber.: C (45.58), H (5.10), N (7.09), Cl (17.94)
Gef.: C (45.61), H (5.09), N (7.00), Cl (17.5)

Beispiel Nr. 70

5-[3-(3-Chlorphenoxy)-propylamino]-5-desoxy-1.4;3.6-dianhydro-L-idit-2-nitrat

Das Gemisch aus 8.8 g (30 mmol) ca. 90%ig. 3-(3-Chlorphenoxy)-propyl-1-methansulfonat, 17.1 g

(90 mmol) 5-Amino-5-desoxy-1.4;3.6-dianhydro-L-idit-2-nitrat und 100 ml Äthanol wird 6 Stdn. unter Rückfluß gekocht. Nach dem Abkühlen und Eindampfen unter reduziertem Druck, nimmt man in 80 ml Chloroform auf, filtriert von unlöslichem Ausgangsamin-Hydrogenmethansulfonat (12 g) ab, und entfernt weiteres Ausgangsamin durch aufeinanderfolgende Extraktion des Filtrats mit 50 ml Wasser und 3mal mit 50 ml 0.2molarer Essigsäure. Die verbleibende Chloroformphase wird mit 30 ml 1molarer Natronlauge gewaschen, über wasserfr. Natriumsulfat getrocknet und unter reduziertem Druck eingedampft. Man erhält 9.8 g (27 mmol) Produkt, die in Äthanol gelöst und mit 14 ml 2molarer Salzsäure in das Hydrochlorid übergeführt werden. Nach dem Eindampfen und Umkristallisieren aus Äthanol + Aktivkohle erhält man 7.96 g (20.1 mmol) reines 5-[3-(3-Chlorphenoxy)-propylamino]-5-desoxy-1.4;3.6-dianhydro-L-idit-2-nitrat-Hydrochlorid.
Schmp. 188–190°; $[\alpha]_D^{25}$ 39.3 (c 0.42; Wasser).

Elementaranalyse: $C_{15}H_{19}ClN_2O_6 \times HCl$ (395.25)
Ber.: C (45.58), H (5.10), N (7.09), Cl (17.94)
Gef.: C (45.78), H (5.20), N (7.08), Cl (18.4)

Das als Ausgangsprodukt verwendete 3-(3-Chlorphenoxy)-propyl-1-methansulfonat erhält man folgendermaßen:
Zur Lösung von 37.3 g (0.2 Mol) 3-(3-Chlorphenoxy)-1-propanol (erhalten durch Umsetzung von 3-Chlorphenol-Natriumsalz mit 3-Chlor-1-propanol in siedendem Äthanol; $Kp_{0.4}$ = 112–116°; Ausbeute 84%) und 42 ml (0.3 Mol) Triäthylamin in 150 ml Chloroform tropft man unter Rühren, Feuchtigkeitsausschluß und Kühlen auf −15°C die Lösung von 20 ml (0.26 Mol) Methansulfonsäurechlorid in 50 ml Chloroform innerhalb 2 Stdn., rührt weitere 2 Stdn. bei −10° nach, läßt über Nacht auf Raumtemperatur kommen und gießt das Reaktionsgemisch unter Rühren auf ein Gemisch aus 300 ml Eiswasser und 5 ml 36%iger Salzsäure. Nach Abtrennen der Chloroformphase wäscht man sie zweimal mit 100 ml Wasser und anschließend mit wäßriger Natriumhydrogencarbonatlösung säurefrei, trocknet über wasserfr. Natriumsulfat und dampft unter reduziertem Druck ein. Man erhält in praktisch quantitativer Ausbeute (58.7 g) noch ca. 10%Chloroform enthaltendes 3-(3-Chlorphenoxy)-propyl-1-methansulfonat als langsam kristallisierendes Öl. Das Kristallisat hat nach dem Waschen mit Petroläther und Trocknen im Vakuumexsiccator den Schmp. 36–37°.

## Beispiel Nr. 71

### 5-[3-(3.4-Dichlorphenoxy)-propylamino]-5-desoxy-1.4;3.6-dianhydro-L-idit-2-nitrat

Herstellung analog Beispiel 70 durch Reaktion von 3.4-Dichlorphenol-Natriumsalz mit 3-Chlor-1-propanol zum 3-(3.4-Dichlorphenoxy)-1-propanol (Schmp. 40–43°; Ausbeute ca. 90%), das quantitativ in das 3-(3.4-Dichlorphenoxy)-propyl-1-methansulfonat (Schmp.: 41–43°) übergeführt und mit überschüssigem 5-Amino-5-desoxy-1.4;3.6-dianhydro-L-idit-2-nitrat in siedendem Äthanol umgesetzt wird. Isolierung als Hydrochlorid.
Ausbeute nach Umkristallisation aus Äthanol: 56%.
Schmp. 175–8°; $[\alpha]_D^{25}$ 32 (c 0.2; Wasser).

Elementaranalyse: $C_{15}H_{18}Cl_2N_2O_6 \times HCl$ (429.69)
Ber.: C (41.93), H (4.46), N (6.52), Cl (24.75)
Gef.: C (41.84), H (4.40), N (6.52), Cl (24.5)

## Beispiel Nr. 72

### 5-[3-(2.4-Dichlorphenoxy)-propylamino]-5-desoxy-1.4;3.6-dianhydro-L-idit-2-nitrat

Herstellung analog Beispiel 71 durch Umsetzung von 3-(2.4-Dichlorphenoxy)-propyl-1-methansulfonat mit überschüssigem 5-Amino-5-desoxy-1.4;3.6-dianhydro-L-idit-2-nitrat. Isolierung als Hydrochlorid. Ausbeute nach zweimaliger Umkristallisation aus Äthanol: 31.6%.
Schmp. 162–4°; $[\alpha]_D^{25}$ 32.7 (c 0.2; Wasser).

Elementaranalyse: $C_{15}H_{18}Cl_2N_2O_6 \times HCl$ (429.69)
Ber.: C (41.93), H (4.46), N (6.52), Cl (24.75)
Gef.: C (41.65), H (4.44), N (6.54), Cl (24.4)

## Beispiel Nr. 73

5-[3-(4-Acetamidophenoxy)-propylamino]-5-desoxy-1.4;3.6-dianhydro-L-idit-2-nitrat

Herstellung analog Beispiel 58 durch Umsetzung von 3-(4-Acetamidophenoxy)-1-brompropan (Schmp. 136–7°; aus Äthanol/Chloroform) mit überschüssigem 5-Amino-5-desoxy-1.4;3.6-dianhydro-L-idit-2-nitrat. Isolierung als Hydrochlorid mit ½ Mol Kristallwasser. Ausbeute nach zweimaliger Umkristallisation aus Äthanol/Methanol: 68.6%.
Schmp. 186–9° (Z); $[\alpha]_D^{25}$ 37.7 (c 0.4; Wasser).

Elementaranalyse: $C_{17}H_{23}N_3O_7 \times HCl \times 0.5\ H_2O$ (426.86)
Ber.: C (47.84), H (5.90), N (9.84), Cl (8.30)
Gef.: C (48.08), H (5.95), N (9.65), Cl (8.4)

## Beispiel Nr. 74

5-[3-(3-Dimethylaminophenoxy)-propylamino]-5-desoxy-1.4;3.6-dianhydro-L-idit-2-nitrat

Herstellung analog Beispiel 70 durch Reaktion von 3-Dimethylaminophenol-Natriumsalz mit 3-Chlor-1-propanol zum 3-(3-Dimethylaminophenoxy)-1-propanol ($Kp_{0.7}$ = 150–5°; Ausbeute 57%), das quantitativ in das 3-(3-Dimethylaminophenoxy)-propyl-1-methansulfonat (Öl) übergeführt und mit überschüssigem 5-Amino-5-desoxy-1.4;3.6-dianhydro-L-idit-2-nitrat in siedendem Äthanol umgesetzt wird. Isolierung als Hydrochlorid. Ausbeute nach Umkristallisation aus Äthanol + Aktivkohle: 38.2%.
Schmp. 173–6° (Z); $[\alpha]_D^{25}$ 35 (c 0.41; Wasser).

Elementaranalyse: $C_{17}H_{25}N_3O_6 \times HCl$ (403.87)
Ber.: C (50.56), H (6.49), N (10.40), Cl (8.78)
Gef.: C (50.00), H (6.59), N (10.16), Cl (9.1)

## Beispiel Nr. 75

5-[3-(4-Chlorphenyl)-propylamino]-5-desoxy-1.4;3.6-dianhydro-L-idit-2-nitrat

Das Gemisch aus 19 g (100 mmol) 5-Amino-5-desoxy-1.4;3.6-dianhydro-L-idit-2-nitrat, 10 g (40 mmol) 3-(4-Chlorphenyl)-propyl-1-methansulfonat (Schmp. 43–45°; hergestellt durch Umsetzung von 3-(4-Chlorphenyl)-1-propanol mit Methansulfonylchlorid) und 150 ml Äthanol wird 20 Stdn. unter Rückfluß gekocht, unter reduziertem Druck eingedampft, in 50 ml Chloroform aufgenommen, 3mal mit je 30 ml 0.3molarer Essigsäure und 1mal mit 30 ml 1molarer Natronlauge gewaschen, über wasserfreiem Natriumsulfat getrocknet, filtriert und erneut unter reduziertem Druck eingedampft. Die so erhaltenen 9.4 g (27.4 mmol) Rohbase liefern nach Überführung in das Hydrochlorid und zweifacher Umkristallisation aus Äthanol 5.08 g 5-[3-(4-Chlorphenyl)-propylamino]-5-desoxy-1.4;3.6-dianhydro-L-idit-2-nitrat-Hydrochlorid vom Schmp. 174.5–176° (Z) und $[\alpha]_D^{25}$ 41.5 (c 0.4; Wasser).

Elementaranalyse: $C_{15}H_{19}ClN_2O_5 \times HCl$ (379.25)
Ber.: C (47.51), H (5.32), N (7.39), Cl (18.70)
Gef.: C (47.82), H (5.48), N (7.52), Cl (18.3)

## Beispiel Nr. 76

5-[4-(4-Chlorphenyl)-butylamino]-5-desoxy-1.4;3.6-dianhydro-L-idit-2-nitrat

Herstellung analog Beispiel Nr. 75 durch Umsetzung von 5-Amino-5-desoxy-1.4;3.6-dianhydro-L-idit-2-nitrat mit 4-(4-Chlorphenyl)-butyl-1-methansulfonat. Ausbeute nach Überführung in das Hydrochlorid und Umkristallisation aus Äthanol: 36%.
Schmp. 173–175°; $[\alpha]_D^{25}$ + 40.0 (c 0.2; Wasser)

Elementaranalyse: $C_{16}H_{21}ClN_2O_5 \times HCl$ (393.28)
Ber.: C (48.87), H (5.64), N (7.12), Cl (18.03)
Gef.: C (49.08), H (5.76), N (7.48), Cl (18.2)

Beispiel Nr. 77

5-Nicotinoylamino-5-desoxy-1.4;3.6-dianhydro-D-mannit-2-nitrat

Das Gemisch aus 2.3 g (10 mmol) 5-Amino-5-desoxy-1.4;3.6-dianhydro-D-mannit-2-nitrat-Hydrochlorid, 3.6 g (20 mmol) Nicotinsäurechlorid-Hydrochlorid und 50 ml wasserfr. Pyridin wird 16 Stdn. bei 60°C unter Feuchtigkeitsausschluß gerührt. Nach dem Abkühlen gießt man in 100 ml Eiswasser, gibt 2.8 g (70 mmol) Natriumhydroxid zu und rührt 30 Min. nach. Durch mehrfache Extraktion mit insgesamt 300 ml Chloroform extrahiert man das Reaktionsprodukt, wäscht die Chloroformphasen 2mal mit je 100 ml Wasser, trocknet sie über wasserfr. Natriumsulfat und dampft im Vakuum ein. Die so erhaltenen 2.5 g (8.5 mmol) Rohbase (Rohausbeute: 85%) ergeben nach Überführung in das Hydrochlorid und Umkristallisation aus wasserfr. Äthanol/Aceton reines 5-Nicotinoylamino-5-desoxy-1.4;3.6-dianhydro-D-mannit-2-nitrat-Hydrochlorid mit Schmp. 162—163° (Z) und $[\alpha]_D^{20}$ + 77.5 (c 0.4; Wasser)

Elementaranalyse: $C_{12}H_{13}N_3O_6 \times HCl$ (331.71)
Ber.: C (43.45), H (4.25), N (12.67), Cl (10.69)
Gef.: C (42.99), H (4.30), N (12.57), Cl (10.7)

Beispiel Nr. 78

5-N-Methyl-N-nicotinoylamino-5-desoxy-1.4;3.6-dianhydro-L-idit-2-nitrat

Herstellung analog Beispiel Nr. 77 durch Umsetzung von 5-Methylamino-5-desoxy-1.4;3.6-dianhydro-L-idit-2-nitrat mit Nicotinsäurechlorid-Hydrochlorid in Pyridin. Rohausbeute: 91%. Überführung in das Hydrochlorid und Umkristallisation aus wasserfr. Äthanol/Aceton ergeben reines 5-N-Methyl-N-nicotinoylamino-5-desoxy-1.4;3.6-dianhydro-L-idit-2-nitrat-Hydrochlorid-Halbhydrat mit Schmp. 173—175° (Z) und $[\alpha]_D^{20}$ + 33.2 (c 0.4; Wasser).

Elementaranalyse: $C_{13}H_{15}N_3O_6 \times HCl \times 0.5\ H_2O$ (354.75)
Ber.: C (44.01), H (4.83), N (11.84), Cl (9.99)
Gef.: C (44.18), H (4.57), N (11.82), Cl (10.4)

Als Vergleichsverbindungen wurden bei der Untersuchung der pharmakologischen Eigenschaften der erfindungsgemäßen Verbindungen stets die im Handel erhältlichen Verbindungen Isosorbiddinitrat (ISDN) und Isosorbidmononitrat (ISMN) verwendet), wobei es sich bei ISMN um das 1.4;3.6-Dianhydro-D-glucit-2-nitrat handelt.

Die koronardurchflußsteigernde Wirksamkeit der erfindungsgemäßen Verbindungen wurde an isolierten Meerschweinchenherzen (isolierte Herzen nach Langendorff, Methode nach Bunger et al., Pflüger's Archiv, 353, 317—325 [1975]) ermittelt. Nach Erreichen des stationären Zustands in der 30. Minute wurden die Herzen mit je 50 ml Tyrodelösung mit einem Gehalt an Prüfsubstanz von jeweils 25 μg/ml perfundiert. Jede Prüfsubstanz wurde an 3—6 Herzen geprüft.

Gemessen wurde jeweils die Inotropie, der Durchfluß und die Frequenz, wobei die in der Tabelle I angegebenen Werte Mittelwerte der prozentualen Änderungen gegenüber dem Vorlauf sind. Der Vergleich der gemessenen Werte zeigt, daß die koronardurchflußsteigernde Wirksamkeit der erfindungsgemäßen Verbindungen größer als diejenige von ISMN ist; die koronardurchflußsteigernde Wirksamkeit einiger Substanzen übertrifft sogar diejenige des ISDN. Viele der erfindungsgemäßen Substanzen zeigen günstige inotropie- und frequenzsenkende Wirkungen.

Die spasmolytische Wirksamkeit der erfindungsgemäßen Verbindungen wurde an isolierten Ratten-Aorta-Präparaten mit Noradrenalin-induzierten und Kaliumchlorid-induzierten Kontraktionen bestimmt (Methode nach Wende und Peiper, Pflüger's Archiv 320, 133—141 (1970); und Towart und Stoepel, Naunyn Schmiedeberg's Archives of Pharmacology; Suppl. Vol. 308, R 18 [1979]).

In Tabelle II sind die Konzentrationen der Prüfsubstanzen angegeben, die für 50% Hemmung des Spasmus notwendig sind ($ED_{50}$-Werte). Die spasmolytischen Wirksamkeiten der erfindungsgemäßen Verbindungen sind ganz überwiegend besser als diejenigen von ISMN und ISDN, besonders wenn man das pharmakologisch wichtige Verhältnis der effektiven Dosen beim Noradrenalinspasmus und Kaliumchloridspasmus berücksichtigt.

Die blutdrucksenkende Wirksamkeit der erfindungsgemäßen Verbindungen wurde im Vergleich zu ISDN und ISMN an narkotisierten Meerschweinchen nach i.v.-Gabe gemessen. Die in Tabelle III (1/2) wiedergegebenen Werte zeigen, daß die erfindungsgemäßen Verbindungen durchweg wirksamer als ISMN sind, wobei die Verbindung Nr. 5 eine stärkere Wirkung als ISDN besitzt.

Die inotrope und frequenzsenkende Herzkreislaufwirksamkeit der erfindungsgemäßen Verbindungen wurde an mischrassigen Katzen von 2,5 bis 3,5 kg Körpergewicht bei intravenöser Applikation bestimmt. Die Tiere wurden mit einem Gemisch von Chloralose-Urethan narkotisiert (1,2 g/kg Urethan

+ 40 mg/kg Chloralose i.p. verabreicht). Sie atmeten spontan durch eine Tracheakanüle. Die A. Carotis sinistra wurde benutzt, um ein Katheter-Tip-Manometer in die linke Herzkammer zu legen. Die V.Jugularis diente zu Injektionszwecken. Über die A.Femoralis dextra wurde ein Katheter bis in die Aorta descendans vorgeschoben und an einen Druckaufnehmer (Statham P 23Db) angeschlossen. Die Herzfrequenz wurde mit einem Pulsfrequenzmesser (Fa. Hugo Sachs Elektronik) aus dem linksventrikulären Drucksignal registriert.

Wie sich aus den in der Tabelle IV (1—6) angegebenen Werten ergibt, ist die Wirksamkeit der geprüften erfindungsgemäßen Verbindungen besser als diejenige der Vergleichsverbindung ISDN. Insbesondere zeigen alle Verbindungen neben der starken Blutdrucksenkung günstige frequenzsenkende Effekte und z. T. langanhaltende Inotropie-Senkung.

Diese Wirkungen wurden weiterhin an mischrassigen Katzen von 2,5 bis 3,5 kg Körpergewicht bei intraduodenaler Applikation bestimmt. Die Tiere wurden mit einem Gemisch von Chloralose-Urethan narkotisiert (1.2 g/kg Urethan + 40 mg/kg Chloralose i.p. verabreicht). Sie atmeten spontan durch eine Tracheakanüle. Die A.Carotis sinistra wurde benutzt, um ein Katheter-Tip-Manometer in die linke Herzkammer zu legen. Die V.Jugularis diente zu Injektionszwecken. Über die A.Femoralis dextra wurde ein Katheter bis in die Aorta descendans vorgeschoben und an einen Druckaufnehmer (Statham P 23 Db) angeschlossen. Die Herzfrequenz wurde mit einem Pulsfreqenzmesser (Fa. Hugo Sachs Elektronik) aus dem linksventrikulären Drucksignal registriert. Durch eine Laparotomie wurde eine Duodenalschlinge freigelegt. Die zu prüfenden Substanzen wurden direkt ins Lumen injiziert.

Wie sich aus den in der Tabelle V angegebenen Werten ergibt, ist die blutdrucksenkende Wirkung der geprüften erfindungsgemäßen Verbindung besser als diejenige der Vergleichsverbindung ISDN.

Tabelle 1

Versuche am Langendorff-Herzen

Die in der Tabelle aufgeführten Werte zeigen die prozentuale Änderung gegenüber dem Vorlauf

| Substanz | Inotropie | Durchfluß | Frequenz | Bemerkungen |
|---|---|---|---|---|
| ISDN | −8,11 | +91,54 | +2,00 | Vergleichsverb. |
| ISMN | −2,67 | +9,11 | −0,51 | Vergleichsverb. |
| 2b | +3,33 | +25,89 | −0,31 | |
| 5 | −4,18 | +21,21 | +3,84 | |
| 10b | +3,33 | +25,89 | −0,31 | |
| 11b | −86,66 | +145,31 | −32,76 | |
| 12b | −2,81 | +16,23 | +1,91 | |
| 13b | −6,76 | +9,05 | −3,52 | |
| 14b | −3,36 | +25,38 | −6,21 | |
| 15b | ±0 | +33,95 | −3,46 | |
| 16b | −1,67 | +14,71 | +2,70 | |
| 17b | +2,26 | +25,07 | −4,16 | |
| 19 | −22,90 | +100,00 | −5,45 | |
| 20 | −49,39 | +66,94 | −23,02 | |
| 21 | −91,00 | +116,87 | −50,60 | |
| 23 | −94,00 | +156,10 | −61,00 | |
| 24 | −85,00 | +200,00 | −18,75 | |
| 25 | −15,87 | +47,76 | ±0 | |
| 27 | −37,67 | +67,24 | −5,64 | |
| 28 | −78,55 | +68,11 | −32,78 | |
| 31b | −19,00 | +79,40 | −11,54 | |
| 34 | −65,00 | +50,00 | −47,60 | |
| 35 | −18,20 | +34,00 | −6,78 | |
| 49 | −78,16 | +190,51 | −9,04 | |
| 51 | −25,60 | +92,41 | −6,96 | |
| 52 | −66,56 | +213,79 | −19,19 | |
| 54 | −77,85 | +274,32 | −15,42 | |
| 55 | −80,75 | +203,22 | −24,34 | |

Tabelle II

| Substanz | Noradrenalinspasmus | Kaliumchloridspasmus |
|---|---|---|
| ISDN | $1{,}30 \times 10^{-6}$ | $3{,}10 \times 10^{-6}$ |
| ISMN | $1{,}60 \times 10^{-5}$ | $2{,}40 \times 10^{-6}$ |
| 1c | $5{,}75 \times 10^{-6}$ | $3{,}80 \times 10^{-5}$ |
| 2b | $6{,}00 \times 10^{-6}$ | $1{,}10 \times 10^{-5}$ |
| 3c | $8{,}00 \times 10^{-6}$ | – |
| 4b | $9{,}40 \times 10^{-6}$ | $2{,}90 \times 10^{-4}$ |
| 5 | $4{,}20 \times 10^{-6}$ | $6{,}00 \times 10^{-6}$ |
| 6 | $2{,}80 \times 10^{-6}$ | $3{,}40 \times 10^{-5}$ |
| 7 | $3{,}40 \times 10^{-7}$ | $3{,}80 \times 10^{-6}$ |
| 8 | $4{,}40 \times 10^{-7}$ | $4{,}60 \times 10^{-6}$ |
| 9b | $6{,}50 \times 10^{-6}$ | $2{,}10 \times 10^{-4}$ |
| 10b | $6{,}00 \times 10^{-6}$ | $6{,}25 \times 10^{-6}$ |
| 11b | $3{,}64 \times 10^{-7}$ | $7{,}50 \times 10^{-6}$ |
| 12b | $5{,}50 \times 10^{-7}$ | $1{,}08 \times 10^{-5}$ |
| 13b | $4{,}80 \times 10^{-7}$ | $7{,}00 \times 10^{-6}$ |
| 14b | $3{,}80 \times 10^{-6}$ | $2{,}90 \times 10^{-6}$ |
| 15b | $4{,}30 \times 10^{-6}$ | $2{,}50 \times 10^{-6}$ |
| 16b | $3{,}00 \times 10^{-6}$ | $4{,}20 \times 10^{-6}$ |
| 17b | $7{,}80 \times 10^{-7}$ | $4{,}30 \times 10^{-6}$ |
| 18 | $3{,}35 \times 10^{-}$ | $3{,}00 \times 10^{-5}$ |
| 19 | $2{,}90 \times 10^{-6}$ | $2{,}30 \times 10^{-6}$ |
| 20 | $2{,}50 \times 10^{-6}$ | $3{,}60 \times 10^{-6}$ |
| 21 | $2{,}00 \times 10^{-8}$ | $2{,}90 \times 10^{-6}$ |
| 22 | $4{,}90 \times 10^{-8}$ | $2{,}60 \times 10^{-6}$ |
| 23 | $2{,}44 \times 10^{-8}$ | $2{,}65 \times 10^{-6}$ |
| 24 | $2{,}00 \times 10^{-7}$ | $2{,}50 \times 10^{-6}$ |
| 25 | $5{,}20 \times 10^{-7}$ | $1{,}33 \times 10^{-5}$ |
| 26 | $1{,}50 \times 10^{-5}$ | $3{,}50 \times 10^{-5}$ |
| 27 | $5{,}20 \times 10^{-7}$ | $9{,}50 \times 10^{-6}$ |
| 28 | $8{,}30 \times 10^{-8}$ | $3{,}10 \times 10^{-6}$ |

Fortsetzung

| Substanz | Noradrenalinspasmus | Kaliumchloridspasmus |
|---|---|---|
| 29 | $4{,}10 \times 10^{-8}$ | $3{,}05 \times 10^{-7}$ |
| 30 | $3{,}45 \times 10^{-8}$ | $9{,}50 \times 10^{-7}$ |
| 31b | $1{,}88 \times 10^{-8}$ | $5{,}00 \times 10^{-7}$ |
| 33 | $5{,}25 \times 10^{-7}$ | $2{,}95 \times 10^{-6}$ |
| 34 | $2{,}10 \times 10^{-8}$ | $1{,}70 \times 10^{-6}$ |
| 35 | – | $4{,}40 \times 10^{-6}$ |
| 36 | $3{,}00 \times 10^{-7}$ | $9{,}50 \times 10^{-7}$ |
| 37 | $4{,}50 \times 10^{-8}$ | $6{,}40 \times 10^{-7}$ |
| 38b | – | $2{,}60 \times 10^{-6}$ |
| 39 | – | $3{,}80 \times 10^{-6}$ |
| 40 | – | $9{,}00 \times 10^{-6}$ |
| 41 | – | $3{,}90 \times 10^{-6}$ |
| 42 | $3{,}20 \times 10^{-7}$ | $1{,}20 \times 10^{-5}$ |
| 43 | $4{,}40 \times 10^{-8}$ | $4{,}60 \times 10^{-7}$ |
| 44 | $2{,}90 \times 10^{-7}$ | $3{,}50 \times 10^{-6}$ |
| 45 | $3{,}40 \times 10^{-7}$ | $3{,}70 \times 10^{-6}$ |
| 46 | $2{,}85 \times 10^{-7}$ | $2{,}80 \times 10^{-6}$ |
| 47 | $2{,}05 \times 10^{-7}$ | $8{,}80 \times 10^{-6}$ |
| 48 | $2{,}50 \times 10^{-7}$ | $4{,}40 \times 10^{-6}$ |
| 49 | $1{,}60 \times 10^{-7}$ | $4{,}60 \times 10^{-7}$ |
| 50 | $2{,}70 \times 10^{-7}$ | $1{,}00 \times 10^{-5}$ |
| 51 | $1{,}10 \times 10^{-7}$ | $2{,}00 \times 10^{-6}$ |
| 52 | $1{,}15 \times 10^{-7}$ | $3{,}40 \times 10^{-7}$ |
| 53 | $1{,}90 \times 10^{-6}$ | $4{,}20 \times 10^{-6}$ |
| 54 | $3{,}60 \times 10^{-8}$ | $1{,}10 \times 10^{-6}$ |
| 55 | $3{,}39 \times 10^{-7}$ | $2{,}40 \times 10^{-6}$ |
| 56 | $1{,}30 \times 10^{-7}$ | $2{,}80 \times 10^{-6}$ |
| 57 | $2{,}39 \times 10^{-7}$ | $1{,}50 \times 10^{-6}$ |
| 58 | – | $2{,}40 \times 10^{-6}$ |
| 59 | – | $1{,}55 \times 10^{-6}$ |

Fortsetzung

| Substanz | Noradrenalinspasmus | Kaliumchloridspasmus |
|---|---|---|
| 60 | – | $6{,}25 \times 10^{-6}$ |
| 62 | $3{,}80 \times 10^{-8}$ | $9{,}80 \times 10^{-7}$ |
| 63 | – | $2{,}40 \times 10^{-6}$ |
| 64 | – | $2{,}90 \times 10^{-5}$ |
| 65 | – | $2{,}20 \times 10^{-6}$ |
| 66 | – | $2{,}00 \times 10^{-6}$ |
| 67 | – | $1{,}89 \times 10^{-6}$ |
| 68 | $2{,}28 \times 10^{-8}$ | $1{,}83 \times 10^{-7}$ |
| 70 | – | $2{,}18 \times 10^{-6}$ |

**Tabelle III**

| Substanz | Dosis | Blutdruck | | Δ |
|---|---|---|---|---|
| | mg/kg | vorher mm Hg | nachher mm Hg | mm Hg |
| ISDN | 0,25 | 68,70 ± 2,30 | 57,00 ± 2,10 | −11,70 |
| | 1,00 | 66,00 ± 3,50 | 46,30 ± 0,90 | −19,70 |
| | 2,50 | 66,70 ± 1,70 | 37,70 ± 0,90 | −29,00 |
| 2b | 0,25 | 56,00 ± 7,91 | 50,25 ± 8,19 | −5,75 |
| | 1,00 | 55,75 ± 6,55 | 41,50 ± 4,66 | −14,25 |
| | 2,50 | 51,25 ± 5,17 | 31,75 ± 3,09 | −19,50 |
| 5 | 0,25 | 64,50 ± 2,75 | 54,00 ± 2,52 | −10,50 |
| | 1,00 | 63,25 ± 2,93 | 40,50 ± 3,66 | −22,75 |
| | 2,50 | 62,50 ± 4,00 | 30,75 ± 8,60 | −31,75 |
| 6 | 0,25 | 70,75 ± 6,36 | 63,75 ± 7,32 | −7,00 |
| | 1,00 | 70,25 ± 6,42 | 55,75 ± 7,94 | −14,50 |
| | 2,50 | 65,50 ± 5,32 | 47,75 ± 5,88 | −17,75 |
| 10b | 0,25 | 43,25 ± 2,21 | 37,25 ± 2,06 | −6,00 |
| | 1,00 | 43,25 ± 2,06 | 32,00 ± 2,04 | −11,25 |
| | 2,50 | 42,75 ± 2,93 | 27,25 ± 1,60 | −15,50 |
| 14b | 0,25 | 71,25 ± 0,63 | 65,75 ± 0,75 | −5,50 |
| | 1,00 | 70,25 ± 2,46 | 56,25 ± 2,17 | −14,00 |
| | 2,50 | 67,75 ± 3,47 | 49,25 ± 3,94 | −18,50 |

Fortsetzung

| Substanz | Dosis mg/kg | Blutdruck vorher mm Hg | nachher mm Hg | Δ mm Hg |
|---|---|---|---|---|
| 15b | 0,25 | 72,00 ± 2,12 | 63,00 ± 1,83 | −9,00 |
| | 1,00 | 71,25 ± 1,89 | 53,50 ± 1,71 | −17,75 |
| | 2,50 | 71,00 ± 2,04 | 49,00 ± 2,48 | −23,00 |
| 16b | 0,25 | 68,75 ± 3,07 | 59,50 ± 5,63 | −9,25 |
| | 1,00 | 68,00 ± 2,86 | 48,25 ± 4,80 | −19,75 |
| | 2,50 | 66,75 ± 3,20 | 43,25 ± 4,91 | −23,50 |
| 17b | 0,25 | 63,50 ± 3,77 | 59,00 ± 2,27 | −4,50 |
| | 1,00 | 64,00 ± 4,42 | 46,25 ± 3,07 | −17,75 |
| | 2,50 | 61,75 ± 5,07 | 38,25 ± 4,55 | −23,50 |
| 47 | 0,25 | 47,00 ± 3,39 | 41,25 ± 2,78 | −5,75 |
| | 1,00 | 44,50 ± 2,40 | 33,75 ± 2,02 | −10,75 |
| | 2,50 | 39,50 ± 1,71 | 25,75 ± 1,31 | −13,75 |
| 49 | 0,25 | 45,75 ± 2,53 | 43,50 ± 3,38 | −2,25 |
| | 1,00 | 43,00 ± 1,08 | 30,25 ± 1,70 | −12,75 |
| | 2,50 | 42,75 ± 1,18 | 20,25 ± 1,03 | −22,50 |
| 50 | 0,25 | 55,50 ± 7,35 | 39,25 ± 6,97 | −16,25 |
| | 1,00 | 52,50 ± 7,10 | 31,00 ± 3,94 | −21,50 |
| | 2,50 | 50,50 ± 5,55 | 22,75 ± 1,80 | −27,75 |
| 51 | 0,25 | 66,75 ± 2,49 | 63,00 ± 2,35 | −3,75 |
| | 1,00 | 66,00 ± 1,78 | 55,75 ± 2,36 | −10,25 |
| | 2,50 | 65,75 ± 1,11 | 42,25 ± 1,32 | −23,50 |
| 52 | 0,25 | 65,50 ± 1,50 | 60,50 ± 1,32 | −5,00 |
| | 1,00 | 61,00 ± 0,70 | 49,25 ± 4,37 | −11,75 |
| | 2,50 | 56,75 ± 3,22 | 39,00 ± 4,38 | −17,75 |
| 54 | 0,25 | 67,00 ± 4,51 | 62,25 ± 8,05 | −4,75 |
| | 1,00 | 67,25 ± 5,36 | 56,50 ± 4,97 | −10,75 |
| | 2,50 | 68,50 ± 3,69 | 44,75 ± 4,50 | −23,75 |
| ISMN | 0,25 | 57,60 ± 3,10 | 53,90 ± 3,10 | −3,70 |
| | 1,00 | 54,70 ± 3,80 | 48,40 ± 3,10 | −6,30 |
| | 2,50 | 52,30 ± 4,80 | 41,40 ± 3,90 | −10,90 |

Tabelle IV

| Substanz | Dosis mg/kg | Herzfrequenz vorher | nachher | Δ | Blutdruck vorher | nachher | Δ | WD min | dp/dt vorher | nachher | Δ | WD min |
|---|---|---|---|---|---|---|---|---|---|---|---|---|
| ISDN | 0,25 | 165,3 ±8,0 | 171,7 ±6,6 | +6,4 | 98,8 ±6,1 | 81,2 ±8,0 | −17,6 | 13,0 ±4,5 | 7650 ±585 | 8050 ±1000 | +400 | – |
| | 0,50 | 167,0 ±8,2 | 173,3 ±5,2 | +6,3 | 95,8 ±4,6 | 73,7 ±5,0 | −22,2 | 16,5 ±7,0 | 7333 ±344 | 7667 ±1003 | +334 | – |
| | 1,00 | 166,7 ±9,5 | 174,3 ±7,2 | +7,6 | 96,5 ±3,9 | 69,2 ±3,8 | −27,3 | 16,0 ±3,4 | 6983 ±367 | 7583 ±1231 | +600 | – |
| | 2,5 | 162,0 ±8,2 | 170,7 ±5,8 | +8,7 | 92,5 ±4,4 | 60,7 ±3,5 | −31,8 | >10 | 6450 ±545 | 7567 ±1540 | +1117 | – |
| 28 | 0,25 | 225,0 ±7,3 | 223,3 ±5,6 | −1,7 | 101,3 ±7,0 | 81,8 ±7,8 | −19,5 | 3,1 ±0,5 | 7667 ±1425 | 7400 ±1172 | −267 | 2,7 ±2,7 |
| | 0,50 | 228,0 ±7,1 | 226,3 ±6,5 | −1,7 | 104,9 ±8,6 | 79,0 ±8,0 | −25,9 | 5,8 ±1,1 | 7567 ±1584 | 6567 ±899 | −1000 | 10,7 ±9,7 |
| | 1,00 | 228,0 ±8,5 | 227,0 ±7,1 | −1,0 | 109,7 ±8,9 | 70,0 ±8,2 | −39,7 | 12,2 ±3,8 | 7067 ±968 | 5533 ±835 | −1534 | 23,0 ±7,0 |
| | 2,50 | 228,3 ±11,0 | 206,3 ±9,1 | −22,0 | 103,5 ±10,2 | 53,2 ±7,1 | −50,3 | >10 | 6667 ±667 | 3600 ±404 | −3067 | >10 |
| 29 | 0,25 | 209,0 ±6,7 | 204,3 ±5,9 | −4,7 | 106,8 ±6,5 | 91,3 ±8,8 | −15,5 | 3,2 ±0,5 | 6480 ±546 | 6000 ±825 | −480 | 1,6 ±1,0 |
| | 0,50 | 212,0 ±6,2 | 204,7 ±7,5 | −7,3 | 113,5 ±5,3 | 91,3 ±7,0 | −22,0 | 4,1 ±0,6 | 7040 ±611 | 6000 ±654 | −1040 | 8,2 ±5,5 |
| | 1,00 | 209,3 ±3,5 | 197,7 ±3,5 | −11,7 | 124,7 ±5,6 | 90,7 ±8,6 | −34,0 | 11,2 ±1,5 | 7360 ±913 | 5000 ±721 | −2360 | 25,5 ±6,6 |
| | 2,50 | 205,0 ±3,4 | 182,0 ±5,8 | −23,0 | 129,5 ±6,7 | 80,8 ±9,0 | −48,7 | >10 | 7500 ±807 | 3920 ±728 | −3580 | >10 |
| 30 | 0,25 | 199,7 ±16,8 | 198,0 ±17,1 | −1,7 | 105,7 ±7,4 | 88,2 ±8,3 | −17,5 | 9,2 ±3,1 | 9600 ±1745 | 8340 ±1674 | −1260 | 10,2 ±1,9 |
| | 0,50 | 201,0 ±19,1 | 200,3 ±17,8 | −0,7 | 106,8 ±8,9 | 81,8 ±9,0 | −25,0 | 11,2 ±3,2 | 9360 ±1873 | 7540 ±1571 | −1820 | 13,7 ±4,7 |
| | 1,00 | 198,3 ±18,2 | 196,0 ±16,4 | −2,3 | 106,3 ±8,6 | 73,3 ±9,4 | −33,3 | 22,2 ±8,4 | 8340 ±1382 | 6160 ±1129 | −2180 | 23,6 ±10,1 |
| | 2,50 | 190,3 ±19,2 | 188,3 ±20,4 | −2,0 | 106,2 ±10,4 | 60,3 ±7,7 | −45,8 | >10 | 7900 ±1505 | 5840 ±1738 | −2060 | >10 |

Fortsetzung

| Substanz | Dosis mg/kg | Herzfrequenz vorher | Herzfrequenz nachher | Δ | Blutdruck vorher | Blutdruck nachher | Δ | WD min | dp/dt vorher | dp/dt nachher | Δ | WD min |
|---|---|---|---|---|---|---|---|---|---|---|---|---|
| 34 | 0,25 | 180,3 ±4,6 | 179,3 ±3,7 | −1,0 | 95,2 ±4,8 | 73,7 ±4,0 | −25,1 | 4,1 ±0,8 | 5333 ±777 | 4650 ±660 | −683 | 2,8 ±1,5 |
| | 0,50 | 179,0 ±4,9 | 178,0 ±3,9 | −1,0 | 99,0 ±5,3 | 74,8 ±4,6 | −24,2 | 4,2 ±0,6 | 5500 ±747 | 4683 ±729 | −817 | 3,7 ±1,6 |
| | 1,00 | 175,0 ±6,7 | 172,0 ±5,4 | −3,0 | 105,3 ±5,0 | 74,5 ±4,3 | −30,8 | 6,5 ±1,4 | 5433 ±786 | 4400 ±686 | −1033 | 4,2 ±1,9 |
| | 2,50 | 173,0 ±6,6 | 158,0 ±3,8 | −15,0 | 108,8 ±5,2 | 69,2 ±4,3 | −39,7 | >9,0 | 5300 ±709 | 3850 ±610 | −1450 | >6,5 1,7 |
| 36 | 0,25 | 200,3 ±4,7 | 204,3 ±4,8 | +4,0 | 114,5 ±7,1 | 94,7 ±9,5 | −19,8 | 5,1 ±0,7 | 7933 ±556 | 7433 ±528 | −500 | 3,3 ±1,3 |
| | 0,50 | 204,0 ±7,6 | 200,3 ±5,9 | −3,7 | 112,5 ±7,8 | 84,5 ±8,5 | −28,7 | 8,5 ±1,9 | 7833 ±605 | 6650 ±595 | −1183 | 7,9 ±1,6 |
| | 1,00 | 202,3 ±6,3 | 194,3 ±7,2 | −8,0 | 116,8 ±6,7 | 74,3 ±5,7 | −42,5 | 14,8 ±2,3 | 7883 ±535 | 5467 ±674 | −2416 | 19,0 ±2,3 |
| | 2,50 | 197,7 ±5,8 | 175,3 ±7,9 | −22,4 | 120,8 ±6,8 | 62,5 ±3,4 | −58,3 | >10 | 7883 ±573 | 4000 ±732 | −3883 | >10 |
| 47 | 0,25 | 187,3 ±6,6 | 188,7 ±6,6 | +1,4 | 125,7 ±5,2 | 121,7 ±5,4 | −4,0 | 3,0 ±1,3 | 8333 ±741 | 7433 ±515 | −900 | 3,7 ±1,7 |
| | 0,50 | 195,7 ±8,8 | 196,0 ±0,3 | +0,3 | 125,5 ±6,0 | 114,2 ±5,5 | −11,3 | 5,7 ±1,6 | 8567 ±947 | 7150 ±857 | −1417 | 9,5 ±4,6 |
| | 1,00 | 192,0 ±8,3 | 187,0 ±10,3 | −5,0 | 125,2 ±4,4 | 100,8 ±4,2 | −24,3 | 8,2 ±0,8 | 8050 ±804 | 5067 ±579 | −2983 | 10,9 ±3,2 |
| | 2,50 | 192,0 ±11,7 | 171,7 ±10,1 | −20,3 | 124,7 ±3,7 | 79,5 ±6,0 | −45,2 | >9,0 | 7433 ±535 | 2933 ±526 | −4500 | >10 |
| 49 | 0,25 | 200,4 ±7,0 | 201,2 ±7,9 | +0,8 | 116,6 ±9,4 | 113,6 ±8,9 | −3,0 | 2,8 ±1,4 | 7840 ±1007 | 7360 ±924 | −480 | 3,1 ±1,9 |
| | 0,50 | 200,0 ±6,4 | 202,0 ±7,6 | +2,0 | 119,8 ±8,8 | 110,6 ±9,6 | −9,2 | 3,7 ±1,4 | 7600 ±916 | 6540 ±736 | −1060 | 14,0 ±5,5 |
| | 1,00 | 194,0 ±12,3 | 188,4 ±8,8 | −5,6 | 122,6 ±9,8 | 105,8 ±12,1 | −16,8 | 11,6 ±2,3 | 6940 ±828 | 4680 ±840 | −2260 | >40 |
| | 2,50 | 191,2 ±9,0 | 170,4 ±7,0 | −20,8 | 121,6 ±12,3 | 86,6 ±12,8 | −35,0 | >10 | 6680 ±1037 | 3780 ±661 | −2900 | >10 |

Fortsetzung

| Substanz | Dosis mg/kg | Herzfrequenz vorher | Herzfrequenz nachher | Δ | Blutdruck vorher | Blutdruck nachher | Δ | WD min | dp/dt vorher | dp/dt nachher | Δ | WD min |
|---|---|---|---|---|---|---|---|---|---|---|---|---|
| 50 | 0,25 | 190,7 ±7,1 | 195,3 ±5,1 | +4,6 | 121,0 ±8,8 | 108,3 ±10,7 | −12,7 | 8,5 ±3,0 | 6553 ±658 | 6033 ±1055 | −500 | 8,2 ±1,9 |
| | 0,50 | 192,0 ±6,8 | 195,3 ±6,3 | +3,3 | 120,2 ±8,4 | 102,2 ±11,5 | −18,0 | 9,8 ±2,7 | 6400 ±695 | 5133 ±710 | −1267 | 8,2 ±1,9 |
| | 1,00 | 191,7 ±6,6 | 191,7 ±6,1 | 0 | 122,0 ±7,3 | 94,2 ±11,7 | −27,8 | 14,8 ±3,5 | 6267 ±457 | 4200 ±596 | −2067 | 14,8 ±3,7 |
| | 2,50 | 189,0 ±6,4 | 178,7 ±7,3 | −10,3 | 123,3 ±7,2 | 77,5 ±10,8 | −45,8 | >10 | 6317 ±322 | 2733 ±410 | −3584 | >10 |
| 52 | 0,25 | 216,7 ±9,3 | 216,7 ±10,4 | 0 | 91,7 ±7,7 | 77,2 ±6,7 | −14,5 | 5,6 ±1,6 | 6150 ±737 | 5517 ±837 | −633 | 4,2 ±2,3 |
| | 0,50 | 217,0 ±10,8 | 217,0 ±11,7 | 0 | 94,7 ±7,2 | 74,8 ±8,0 | −19,9 | 11,7 ±2,4 | 5983 ±677 | 5133 ±720 | −850 | 10,5 ±2,7 |
| | 1,00 | 218,3 ±11,7 | 217,3 ±12,3 | −1,0 | 95,7 ±10,0 | 69,2 ±10,6 | −26,5 | 14,7 ±2,0 | 5583 ±628 | 4250 ±545 | −1333 | 15,3 ±1,8 |
| | 2,50 | 218,3 ±12,0 | 204,0 ±13,9 | −14,3 | 95,0 ±11,6 | 60,5 ±10,8 | −34,5 | >10 | 5233 ±479 | 2733 ±345 | −2500 | >10 |
| 54 | 0,25 | 188,7 ±5,4 | 187,7 ±6,0 | −1,0 | 115,2 ±5,6 | 103,8 ±4,3 | −11,4 | 1,9 ±0,8 | 8783 ±1764 | 8150 ±1736 | −633 | 3,9 ±2,1 |
| | 0,50 | 187,3 ±5,9 | 186,7 ±6,2 | −0,7 | 117,7 ±7,1 | 101,8 ±4,4 | −15,9 | 3,2 ±1,0 | 8767 ±1897 | 6833 ±1385 | −1934 | 7,6 ±3,1 |
| | 1,00 | 184,0 ±6,4 | 172,0 ±5,0 | −12,0 | 118,8 ±8,4 | 91,0 ±5,8 | −27,8 | 5,6 ±1,3 | 8333 ±1793 | 5050 ±1108 | −3283 | 15,2 ±3,9 |
| | 2,50 | 178,3 ±5,6 | 158,7 ±4,3 | −20,0 | 123,3 ±8,7 | 78,5 ±4,8 | −44,8 | 6,4 ±1,1 | 8000 ±1595 | 2817 ±520 | −5183 | >10 |

Fortsetzung

| Substanz | Dosis mg/kg | Herzfrequenz vorher | Herzfrequenz nachher | Δ | Blutdruck vorher | Blutdruck nachher | Δ | WD min | dp/dt vorher | dp/dt nachher | Δ | WD min |
|---|---|---|---|---|---|---|---|---|---|---|---|---|
| 55 | 0,25 | 161,7 ±16,8 | 164,7 ±16,5 | +3,0 | 113,8 ±6,2 | 107,0 ±6,1 | −6,8 | 4,8 ±1,3 | 5633 ±863 | 5200 ±757 | −433 | 4,2 ±2,1 |
| | 0,50 | 155,3 ±17,9 | 157,3 ±17,3 | +2,0 | 111,5 ±6,1 | 98,8 ±7,0 | −12,7 | 2,8 ±0,6 | 5200 ±759 | 4667 ±684 | −533 | 5,2 ±1,6 |
| | 1,00 | 155,3 ±18,6 | 154,0 ±17,2 | −1,3 | 115,0 ±7,7 | 96,8 ±8,8 | −18,2 | 5,2 ±1,0 | 5117 ±745 | 4000 ±576 | −1117 | 8,9 ±2,8 |
| | 2,50 | 148,0 ±17,1 | 148,0 ±12,5 | 0 | 120,3 ±6,1 | 94,3 ±7,5 | −26,0 | 6,3 ±0,8 | 4783 ±579 | 3517 ±534 | −1266 | >7,8 ±1,4 |

Tab. IV (1–6): Herzfrequenz ($min^{-1}$), Blutdruck (mm Hg) und Inotropie (mm Hg/sec) nach intravenöser Applikation der erfindungsgemäßen Verbindungen an der Katze.
Mittelwerte ± Standardfehler aus je 6 Tieren.

Tabelle V

| Substanz + Dosis | Zeit (min) nach Appl. | Frequenz $min^{-1}$ | Blutdruck mm Hg | Δ | dp/dt mm sec. | Δ |
|---|---|---|---|---|---|---|
| ISDN | 0 | 174,3 ± 9,3 | 104,3 ± 7,7 | | 8800 ± 831 | |
| 5 mg/kg | 10 | 179,3 ± 7,7 | 90,4 ± 12,5 | −13,9 | 8000 ± 1097 | −800 |
| | 30 | 177,7 ± 7,7 | 91,2 ± 10,0 | −13,1 | 7667 ± 807 | −1133 |
| | 60 | 175,0 ± 8,6 | 94,2 ± 9,2 | −10,1 | 7633 ± 743 | −1167 |
| | 120 | 166,7 ± 9,4 | 95,0 ± 9,6 | −9,3 | 7050 ± 661 | −1750 |
| 52 | 0 | 209,6 ± 15,7 | 113,8 ± 5,8 | | 6540 ± 631 | |
| 5 mg/kg | 10 | 212,4 ± 13,8 | 100,4 ± 11,4 | −13,4 | 6200 ± 374 | −340 |
| | 30 | 212,8 ± 13,1 | 95,6 ± 9,9 | −18,2 | 6120 ± 270 | −440 |
| | 60 | 216,0 ± 12,3 | 95,8 ± 8,0 | −18,0 | 5480 ± 314 | −1060 |
| | 120 | 214,4 ± 11,6 | 98,4 ± 9,0 | −15,4 | 5460 ± 768 | −1080 |

Herzfrequenz ($min^{-1}$), Blutdruck (mm Hg) und Inotropie (mm Hg/sec) nach intraduodenaler Applikation von ISDN oder Substanz Nr. 52 an Katzen.
Mittelwerte ± Standardfehler aus je 6 Tieren.

Zur orientierenden Prüfung der akuten Toxizität einiger der erfindungsgemäßen Verbindungen wurden diese in physiologischer Kochsalzlösung an weiblichen NMRI-Albinomäusen intravenös in Dosen von 50, 100 und 200 mg/kg appliziert. Die Substanzen wurden an mindestens 3 Tieren pro Dosis gespritzt. Wenn nach der höchsten applizierten Dosis noch kein Tier gestorben war, wurden keine weiteren Substanzdosen geprüft. Im Zweifelsfall wurde die Prüfung an mindestens 3 weiteren Tieren mit der gleichen Dosis wiederholt. Beobachtet wurde die Todesrate innerhalb 24 Stunden nach der Applikation.

In der Tabelle VI sind die ermittelten Todesraten und der daraus abgeschätzte $LD_{50}$-Bereich dargestellt.

Tabelle VI

| Beispiel Nr. | Todeshäufigkeit bei der i. v. Dosis von 200 mg/kg | 100 mg/kg | 50 mg/kg | Geschätzter $LD_{50}$-Bereich (mg/kg) |
|---|---|---|---|---|
| 1c | 0/3 | – | – | >200 |
| 2c | 0/3 | – | – | >200 |
| 6 | 0/3 | – | – | >200 |
| 12b | 0/3 | – | – | >200 |
| 13b | 6/6 | 0/3 | – | 100 – 200 |
| 14b | 1/3 | – | – | ≥200 |
| 15b | 6/6 | 2/3 | 0/3 | 50 – 100 |
| 16b | 0/3 | – | – | ≥200 |
| 17b | 0/3 | – | – | >200 |
| 19 | 6/6 | 0/3 | – | 100 – 200 |
| 21 | 6/6 | 3/3 | 0/3 | 50 – 100 |
| 22 | 6/6 | 3/3 | 1/3 | ≥ 50 |
| 25 | 6/6 | 0/3 | – | 100 – 200 |
| 28 | 3/3 | 5/6 | 0/10 | 50 – 100 |
| 29 | 3/3 | 5/6 | 5/6 | < 50 |
| 32 | – | 10/10 | 1/10 | 50 – 100 |
| 35 | 3/3 | 2/6 | – | ≥100 |
| 36 | – | 5/6 | 0/3 | 50 – 100 |
| 37 | 3/3 | 10/10 | 3/10 | ≥ 50 |
| 42 | 6/6 | 2/3 | 0/3 | 50 – 100 |
| 43 | 6/6 | 3/3 | 1/3 | ≥ 50 |
| 44 | 2/3 | 0/3 | – | 100 – 200 |
| 46 | 2/3 | 0/3 | – | 100 – 200 |
| 50 | 4/6 | 0/3 | – | 100 – 200 |
| 51 | 6/6 | 3/3 | 0/3 | 50 – 100 |
| 52 | 6/6 | 1/3 | – | 100 – 200 |
| 58 | – | 7/12 | – | ca. 100 |
| 65 | 3/3 | 10/10 | 4/10 | ca. 50 |
| 66 | 3/3 | 2/10 | – | 100 – 200 |
| 67 | 3/3 | 3/3 | 0/10 | ca. 70 |
| 68 | – | 4/9 | – | ca. 100 |
| 69 | – | 3/6 | 0/10 | 50 – 100 |
| 72 | – | 0/6 | – | >100 |
| 73 | 0/3 | 0/3 | – | >200 |

**Patentansprüche**

1. Amino-desoxy-1.4;3.6-dianhydro-hexit-nitrate der allgemeinen Formel I,

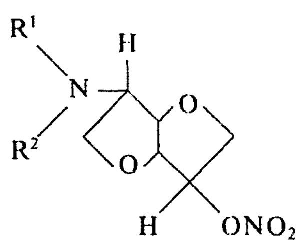

(I)

worin

$R^1$ und $R^2$ jeweils unabhängig voneinander ein Wasserstoffatom oder eine Niedrigalkylgruppe mit 1 bis 4 C-Atomen bedeuten

oder worin

$R^1$ ein Wasserstoffatom oder eine Niedrigalkylgruppe mit 1 bis 4 C-Atomen und

$R^2$ einen Acylrest einer aliphatischen oder einfach ungesättigten, gegebenenfalls methylsubstituierten Monocarbonsäure mit 2 bis 8 C-Atomen, einen Nicotinoyl-, 2-O-Acetylsalicoylrest oder einen 1-Adamantylrest bedeuten

oder worin

$R^1$ ein Wasserstoffatom und
$R^2$ einen 2-Hydroxy-3-(subst.)-phenoxy-prop-1-yl-Rest der allgemeinen Formel Ia

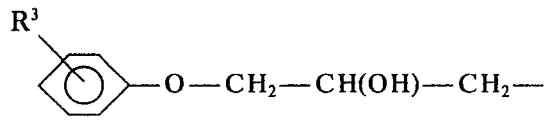
(Ia)

bedeuten, worin

$R^3$ ein Wasserstoffatom, eine Niedrigalkylgruppe mit 1 bis 4 C-Atomen oder eine Niedrigalkenylgruppe mit 2 bis 4 C-Atomen, den Trifluormethylrest, eine Hydroxygruppe, eine Niedrigalkoxygruppe mit 1 bis 4 C-Atomen oder eine Niedrigalkenyloxygruppe mit 2 bis 4 C-Atomen, eine Cyanogruppe oder einen Carbamoylmethylrest bedeuten,

oder worin

$R^1$ ein Wasserstoffatom und
$R^2$ einen 2-Hydroxy-3-($\alpha$-naphthyloxy)-prop-1-yl-Rest bedeuten, wobei der nicht mit der Hydroxypropylgruppe verätherte Ring des Naphthalingerüstes ganz oder teilweise hydriert und durch eine Oxogruppe substituiert sein kann,

oder worin

$R^1$ ein Wasserstoffatom oder eine Niedrigalkylgruppe mit 1 bis 4 C-Atomen und
$R^2$ eine $\omega$-(subst.)-Phenylalkylgruppe der allgemeinen Formel Ib

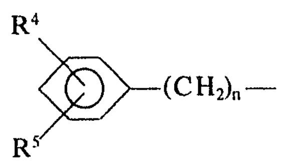
(Ib)

bedeuten, worin

n = eine ganze Zahl von 1–6 sein kann und
$R^4$ und $R^5$ jeweils unabhängig voneinander ein Wasserstoffatom, eine Niedrigalkylgruppe mit 1 bis 4 C-Atomen oder eine Niedrigalkenylgruppe mit 2 bis 4 C-Atomen, den Trifluormethylrest, eine Hydroxygruppe, eine Niedrigalkoxygruppe mit 1 bis 4 C-Atomen oder eine Niedrigalkenyloxygruppe mit 2 bis 4 C-Atomen oder ein Fluor- oder Chloratom bedeuten

oder worin

$R^1$ ein Wasserstoffatom oder eine Niedrigalkylgruppe mit 1 bis 4 C-Atomen und
$R^2$ den Diphenylmethyl-Rest oder Cinnamyl-Rest bedeuten

oder worin

$R^1$ ein Wasserstoffatom oder eine Niedrigalkylgruppe mit 1 bis 4 C-Atomen und
$R^2$ eine $\omega$-(subst.)-Phenoxyalkylgruppe der allgemeinen Formel Ic

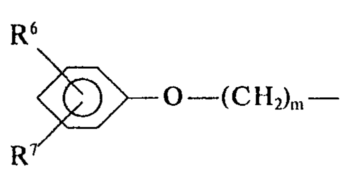
(Ic)

bedeuten, worin

m = eine ganze Zahl von 2–8 sein kann und
$R^6$ und $R^7$ unabhängig voneinander ein Wasserstoffatom, eine Niedrigalkylgruppe mit 1 bis 4 C-Atomen oder eine Niedrigalkenylgruppe mit 2 bis 4 C-Atomen, den Trifluormethylrest, eine Hydroxygruppe, eine Niedrigalkoxygruppe mit 1 bis 4 C-Atomen oder eine Niedrigalkenyloxygruppe mit 2 bis 4 C-Atomen, ein Fluor- oder Chloratom, die Amino- oder Acetylaminogruppe, eine mono- oder di-Niedrigalkylaminogruppe mit 1 bis 4 C-Atomen bedeuten oder
$R^6$ und $R^7$ zusammen mit dem Phenylrest den $\alpha$-Naphthylrest bilden

oder worin

$R^1$ und $R^2$ gemeinsam mit dem Stickstoffatom, an das sie gebunden sind, den Rest eines cyclischen, nicht-aromatischen, gegebenenfalls ein weiteres Heteroatom enthaltenden sekundären Amins mit 5 bis 7 Ringatomen darstellen

oder worin

$R^1$ und $R^2$ gemeinsam den Pyridoxylidenrest bedeuten,

sowie deren pharmakologisch unbedenkliche Säureadditionssalze.

2. 5-Amino-5-desoxy-1.4;3.6-dianhydro-L-idit-2-nitrate der allgemeinen Formel V

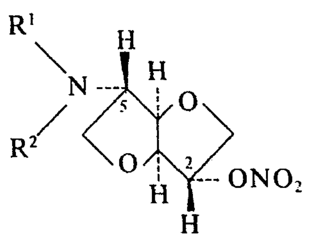

(V)

worin $R^1$ und $R^2$ die in Anspruch 1 angegebenen Bedeutungen besitzen, sowie deren pharmakologisch unbedenkliche Säureadditionssalze.

3. 5-Amino-5-desoxy-1.4;3.6-dianhydro-D-glucit-2-nitrate der allgemeinen Formel VI

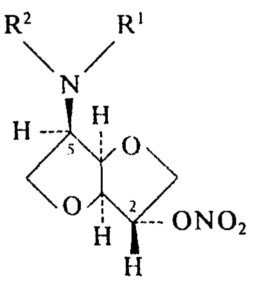

(VI)

worin $R^1$ und $R^2$ die in Anspruch 1 angegebenen Bedeutungen besitzen, sowie deren pharmakologisch unbedenkliche Säureadditionssalze.

4. 2-Amino-2-desoxy-1.4;3.6-dianhydro-D-glucit-5-nitrate der allgemeinen Formel VII

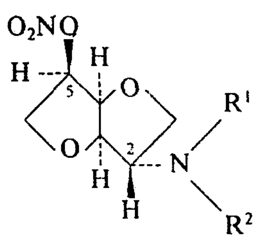

(VII)

worin $R^1$ und $R^2$ die in Anspruch 1 angegebenen Bedeutungen besitzen, sowie deren pharmakologisch unbedenkliche Säureadditionssalze.

5. 5-Amino-5-desoxy-1.4;3.6-dianhydro-D-mannit-2-nitrate der allgemeinen Formel VIII

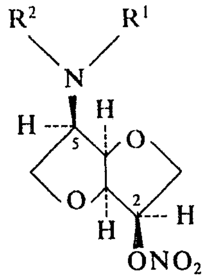

(VIII)

worin $R^1$ und $R^2$ die in Anspruch 1 angegebenen Bedeutungen besitzen, sowie deren pharmakologisch unbedenkliche Säureadditionssalze.

6. 5-Amino-5-desoxy-1.4;3.6-dianhydro-L-idit-2-nitrat.
7. 5-Amino-5-desoxy-1.4;3.6-dianhydro-D-glucit-2-nitrat.
8. 2-Amino-2-desoxy-1.4;3.6-dianhydro-D-glucit-5-nitrat.
9. 5-Amino-5-desoxy-1.4;3.6-dianhydro-D-mannit-2-nitrat.
10. 5-Pivaloylamino-5-desoxy-1.4;3.6-dianhydro-L-idit-2-nitrat.
11. 5-Acetylamino-5-desoxy-1.4;3.6-dianhydro-L-idit-2-nitrat.
12. 5-Nicotinoylamino-5-desoxy-1.4;3.6-dianhydro-L-idit-2-nitrat.
13. 5-(2-Acetoxybenzoylamino)-5-desoxy-1.4;3.6-dianhydro-L-idit-2-nitrat.
14. 5-Methylamino-5-desoxy-1.4;3.6-dianhydro-L-idit-2-nitrat.
15. 5-Äthylamino-5-desoxy-1.4;3.6-dianhydro-L-idit-2-nitrat.
16. 5-Adamant-1-ylamino-5-desoxy-1.4;3.6-dianhydro-L-idit-2-nitrat.
17. 5-Dimethylamino-5-desoxy-1.4;3.6-dianhydro-L-idit-2-nitrat.
18. 5-Diäthylamino-5-desoxy-1.4;3.6-dianhydro-L-idit-2-nitrat.
19. 5-Pyrrolidino-5-desoxy-1.4;3.6-dianhydro-L-idit-2-nitrat.
20. 5-Piperidino-5-desoxy-1.4;3.6-dianhydro-L-idit-2-nitrat.
21. 5-Morpholino-5-desoxy-1.4;3.6-dianhydro-L-idit-2-nitrat.
22. 5-(4-Methylpiperazino)-5-desoxy-1.4;3.6-dianhydro-L-idit-2-nitrat.
23. 5-Pyridoxylidenamino-5-desoxy-1.4;3.6-dianhydro-L-idit-2-nitrat.
24. 5-(N-Benzyl-N-methylamino)-5-desoxy-1.4;3.6-dianhydro-L-idit-2-nitrat.
25. 5-[N-(2-Phenyläthyl)-N-methylamino]-5-desoxy-1.4;3.6-dianhydro-L-idit-2-nitrat.
26. 5-[N-Methyl-N-(3-phenylpropyl)-amino]-5-desoxy-1.4;3.6-dianhydro-L-idit-2-nitrat.
27. 5-[N-(4-Phenylbutyl)-N-methylamino]-5-desoxy-1.4;3.6-dianhydro-L-idit-2-nitrat.
28. 5-[N-Methyl-N-(5-phenylpentyl)-amino]-5-desoxy-1.4;3.6-dianhydro-L-idit-2-nitrat.
29. 5-(N-Cinnamyl-N-methylamino)-5-desoxy-1.4;3.6-dianhydro-L-idit-2-nitrat.
30. 5-Benzylamino-5-desoxy-1.4;3.6-dianhydro-L-idit-2-nitrat.
31. 5-Diphenylmethylamino-5-desoxy-1.4;3.6-dianhydro-L-idit-2-nitrat.
32. 5-(2-Phenyläthylamino)-5-desoxy-1.4;3.6-dianhydro-L-idit-2-nitrat.
33. 5-(3-Phenylpropylamino)-5-desoxy-1.4;3.6-dianhydro-L-idit-2-nitrat.
34. 5-(4-Phenylbutylamino)-5-desoxy-1.4;3.6-dianhydro-L-idit-2-nitrat.
35. 5-(5-Phenylpentylamino)-5-desoxy-1.4;3.6-dianhydro-L-idit-2-nitrat.
36. 5-[3-(3.4-Dimethoxyphenyl)-propylamino]-5-desoxy-1.4;3.6-dianhydro-L-idit-2-nitrat.
37. 5-[4-(4-Methoxyphenyl)-butylamino]-5-desoxy-1.4;3.6-dianhydro-L-idit-2-nitrat.
38. 5-[N-(2-Phenoxyäthyl)-N-methylamino]-5-desoxy-1.4;3.6-dianhydro-L-idit-2-nitrat.
39. 5-[N-Methyl-N-(3-phenoxypropyl)-amino]-5-desoxy-1.4;3.6-dianhydro-L-idit-2-nitrat.
40. 5-(2-Phenoxyäthylamino)-5-desoxy-1.4;3.6-dianhydro-L-idit-2-nitrat.
41. 5-(3-Phenoxypropylamino)-5-desoxy-1.4;3.6-dianhydro-L-idit-2-nitrat.
42. 5-(4-Phenoxybutylamino)-5-desoxy-1.4;3.6-dianhydro-L-idit-2-nitrat.
43. 5-(5-Phenoxypentylamino)-5-desoxy-1.4;3.6-dianhydro-L-idit-2-nitrat.
44. 5-(6-Phenoxyhexylamino)-5-desoxy-1.4;3.6-dianhydro-L-idit-2-nitrat.
45. 5-(7-Phenoxyheptylamino)-5-desoxy-1.4;3.6-dianhydro-L-idit-2-nitrat.
46. 5-(8-Phenoxyoctylamino)-5-desoxy-1.4;3.6-dianhydro-L-idit-2-nitrat.
47. 2-(3-Phenoxypropylamino)-2-desoxy-1.4;3.6-dianhydro-D-glucit-5-nitrat.
48. 2-(4-Phenylbutylamino)-2-desoxy-1.4;3.6-dianhydro-D-glucit-5-nitrat.
49. 5-[2-Hydroxy-3-(4-methoxyphenoxy)-propylamino]-5-desoxy-1.4;3.6-dianhydro-L-idit-2-nitrat.
50. 5-[2-Hydroxy-3-(3-methoxyphenoxy)-propylamino]-5-desoxy-1.4;3.6-dianhydro-L-idit-2-nitrat.
51. 5-[2-Hydroxy-3-(2-methoxyphenoxy)-propylamino]-5-desoxy-1.4;3.6-dianhydro-L-idit-2-nitrat.
52. 5-[2-Hydroxy-3-(2-äthoxyphenoxy)-propylamino]-5-desoxy-1.4;3.6-dianhydro-L-idit-2-nitrat.
53. 5-[2-Hydroxy-3-(2-allyloxyphenoxy)-propylamino]-5-desoxy-1.4;3.6-dianhydro-L-idit-2-nitrat.

54. 5-[2-Hydroxy-3-(2-allylphenoxy)-propylamino]-5-desoxy-1.4;3.6-dianhydro-L-idit-2-nitrat.
55. 5-[2-Hydroxy-3-(2-cyanophenoxy)-propylamino]-5-desoxy-1.4;3.6-dianhydro-L-idit-2-nitrat.
56. 5-[2-Hydroxy-3-(3-tolyloxy)-propylamino]-5-desoxy-1.4;3.6-dianhydro-L-idit-2-nitrat.
57. 5-[2-Hydroxy-3-(3-trifluormethylphenoxy)-propylamino]-5-desoxy-1.4;3.6-dianhydro-L-idit-2-nitrat.
58. 5-[2-Hydroxy-3-(4-carbamoylmethylphenoxy)-propylamino]-5-desoxy-1.4;3.6-dianhydro-L-idit-2-nitrat.
59. 5-[2-Hydroxy-3-(1-naphthyloxy)-propylamino]-5-desoxy-1.4;3.6-dianhydro-L-idit-2-nitrat.
60. 5-[2-Hydroxy-3-(1-naphthyloxy)-propylamino]-5-desoxy-1.4;3.6-dianhydro-D-glucitol 2-nitrat.
61. 5-[2-Hydroxy-3-(1-oxo-1.2.3.4-tetrahydro-5-naphthyloxy)-propylamino]-5-desoxy-1.4;3.6-dianhydro-L-idit-2-nitrat.
62. 5-[2-Hydroxy-3-(5.6.7.8-tetrahydro-1-naphthyloxy)-propylamino]-5-desoxy-1.4;3.6-dianhydro-L-idit 2-nitrat.
63. 5-[3-(3-Trifluormethylphenoxy)-propylamino]-5-desoxy-1.4;3.6-dianhydro-L-idit-2-nitrat.
64. 5-[3-(4-Tolyloxy)-propylamino]-5-desoxy-1.4;3.6-dianhydro-L-idit-2-nitrat.
65. 5-[3-(4-Fluorphenoxy)-propylamino]-5-desoxy-1.4;3.6-dianhydro-L-idit-2-nitrat.
66. 5-[3-(1-Naphthyloxy)-propylamino]-5-desoxy-1.4;3.6-dianhydro-L-idit-2-nitrat.
67. 5-[3-(4-Methoxyphenoxy)-propylamino]-5-desoxy-1.4;3.6-dianhydro-L-idit-2-nitrat.
68. 5-[3-(3-Methoxyphenoxy)-propylamino]-5-desoxy-1.4;3.6-dianhydro-L-idit-2-nitrat.
69. 5-[3-(2-Methoxyphenoxy)-propylamino]-5-desoxy-1.4;3.6-dianhydro-L-idit-2-nitrat.
70. 5-[3-(2.6-Dimethoxyphenoxy)-propylamino]-5-desoxy-1.4;3.6-dianhydro-L-idit-2-nitrat.
71. 5-[3-(3.5-Dimethoxyphenoxy)-propylamino]-5-desoxy-1.4;3.6-dianhydro-L-idit-2-nitrat.
72. 5-[3-(2.3-Dimethoxyphenoxy)-propylamino]-5-desoxy-1.4;3.6-dianhydro-L-idit-2-nitrat.
73. 5-[3-(4-Chlorphenoxy)-propylamino]-5-desoxy-1.4;3.6-dianhydro-L-idit-2-nitrat.
74. 5-[3-(2-Chlorphenoxy)-propylamino]-5-desoxy-1.4;3.6-dianhydro-L-idit-2-nitrat.
75. 5-[3-(3-Chlorphenoxy)-propylamino]-5-desoxy-1.4;3.6-dianhydro-L-idit-2-nitrat.
76. 5-[3-(3.4-Dichlorphenoxy)-propylamino]-5-desoxy-1.4;3.6-dianhydro-L-idit-2-nitrat.
77. 5-[3-(2.4-Dichlorphenoxy)-propylamino]-5-desoxy-1.4;3.6-dianhydro-L-idit-2-nitrat.
78. 5-[3-(4-Acetamidophenoxy)-propylamino]-5-desoxy-1.4;3.6-dianhydro-L-idit-2-nitrat.
79. 5-[3-(3-Dimethylaminophenoxy)-propylamino]-5-desoxy-1.4;3.6-dianhydro-L-idit-2-nitrat.
80. 5-[3-(4-Chlorphenyl)-propylamino]-5-desoxy-1.4;3.6-dianhydro-L-idit-2-nitrat.
81. 5-[4-(4-Chlorphenyl)-butylamino]-5-desoxy-1.4;3.6-dianhydro-L-idit-2-nitrat.
82. 5-Nicotinoylamino-5-desoxy-1.4;3.6-dianhydro-D-mannit-2-nitrat.
83. 5-N-Methyl-N-nicotinoylamino-5-desoxy-1.4;3.6-dianhydro-L-idit-2-nitrat.
84. Verfahren zur Herstellung der Verbindungen gemäß Anspruch 1, dadurch gekennzeichnet, daß

(A) L-Isoidid, D-Isosorbid oder D-Isomannid mit einem Sulfonsäurechlorid in den entsprechenden Mono-O-acyl-1.4;3.6-dianhydro-hexit übergeführt wird,

(B) welcher durch Ammonolyse mit wäßriger Ammoniaklösung oder durch Aminolyse mit einem primären oder sekundären Alkylamin mit 1 bis 4 C-Atomen, mit einem primären Amin oder mit einem cyclischen, nicht-aromatischen sekundären Amin, gegebenenfalls unter Zusatz eines geeigneten Lösungsmittels, zum entsprechenden Amino-desoxy-1.4;3.6-dianhydro-hexit umgesetzt wird,

(C) wonach die freie Hydroxylgruppe des entstandenen Amino-desoxy-1.4;3.6-dianhydro-hexits oder gegebenenfalls dessen Säureadditionssalzes mit Salpetersäure, Nitriersäure oder mit einem Gemisch aus Salpetersäure und Eisessig/Acetanhydrid in Gegenwart von Harnstoff zum entsprechenden Aminodesoxy-1.4;3.6-dianhydro-hexit-nitrat verestert wird, und

(D) wonach gegebenenfalls anschließend das Nitrat mit der gewünschten, den Substituenten $R^2$ bildenden reaktiven Verbindung umgesetzt wird, nämlich mit einem Carbonsäurechlorid, einem Aldehyd, einem $\omega$-(subst.)-Aryl-alkylhalogenid, $\omega$-Phenylalkenylhalogenid, $\omega$-(subst.)-Arylalkylmethansulfonat, $\omega$-(subst.)-Aryloxyalkylhalogenid, 1.2-Epoxy-$\omega$-(subst.)-phenoxy-alkan, 1.2-Epoxy-$\omega$-(subst.)-(1-naphthyloxy)-alkan oder $\omega$-(subst.)-Phenoxyalkyl-methansulfonat umgesetzt wird.

85. Verfahren zur Herstellung der Verbindungen gemäß Anspruch 2, dadurch gekennzeichnet, daß

(A') D-Isosorbid mit einem Überschuß an Sulfonsäurechlorid quantitativ zum entsprechenden Isosorbid-diacylat umgesetzt wird,

(B') welches durch Ammonolyse mit wäßriger Ammoniaklösung oder durch Aminolyse mit einem primären oder sekundären Alkylamin mit 1 bis 4 C-Atomen oder mit einem cyclischen, nicht-aromatischen sekundären Amin zum entsprechenden 5-Amino-5-desoxy-1.4;3.6-dianhydro-L-idit-2-sulfonat umgesetzt wird,

(C') wonach das unter (B') erhaltene Reaktionsgemisch einer sauren oder alkalischen Hydrolyse unterworfen wird und
(D') der so entstandene 5-Amino-5-desoxy-1.4;3.6-dianhydro-L-idit gemäß Anspruch 84, (C) und gegebenenfalls (D), weiter aufgearbeitet wird.

86. Verfahren zur Herstellung der Verbindungen gemäß Anspruch 3, dadurch gekennzeichnet, daß

(A") D-Isosorbid mit einem Überschuß an Sulfonsäurechlorid quantitativ zum entsprechenden Isosorbid-diacylat umgesetzt wird,
(B") welches mit Natriumbenzoat selektiv zum entsprechenden 1.4;3.6-Dianhydro-L-idit-2-sulfonat-5-benzoat umgesetzt wird,
(C") wonach letzteres durch Ammonolyse mit wäßriger Ammoniaklösung oder durch Aminolyse mit einem primären oder sekundären Alkylamin mit 1 bis 4 C-Atomen oder mit einem cyclischen, nicht-aromatischen sekundären Amin, wobei gleichzeitig der Benzoesäurerest hydrolytisch abgespalten wird, zum entsprechenden 5-Amino-5-desoxy-1.4;3.6-dianhydro-D-glucit umgesetzt wird und
(D") der so entstandene 5-Amino-5-desoxy-1.4;3.6-dianhydro-D-glucit gemäß Anspruch 84, (C) und gegebenenfalls (D), weiter aufgearbeitet wird.

87. Verfahren nach einem der Ansprüche 84 bis 86, dadurch gekennzeichnet, daß als Sulfonsäurechlorid Methansulfonsäurechlorid oder Toluolsulfonsäurechlorid verwendet wird.
88. Verfahren nach Anspruch 86, dadurch gekennzeichnet, daß die Umsetzung mit Natriumbenzoat (B") in einem wasserfreien, dipolaren, aprotischen Lösungsmittel unter Inertgasatmosphäre bei erhöhter Temperatur durchgeführt wird.
89. Verfahren nach Anspruch 88, dadurch gekennzeichnet, daß die Umsetzung mit Natriumbenzoat in Dimethylformamid bei einer Temperatur von zwischen 100 und 180°C durchgeführt wird.
90. Verfahren nach einem der Ansprüche 84 bis 88, dadurch gekennzeichnet, daß die Ammonolyse bzw. Aminolyse unter erhöhtem Druck und erhöhter Temperatur durchgeführt wird.
91. Verfahren nach Anspruch 90, dadurch gekennzeichnet, daß die Ammonolyse bzw. Aminolyse bei einer Temperatur von zwischen 90 und 180°C durchgeführt wird.
92. Verfahren nach Anspruch 85, dadurch gekennzeichnet, daß die Ammonolyse bzw. Aminolyse bei einer Temperatur von zwischen 90 und 120°C durchgeführt wird.
93. Pharmazeutische Zubereitung, enthaltend eine Verbindung gemäß einem der Ansprüche 1 bis 83 sowie übliche Träger und Zusatzstoffe.

**Claims**

1. Aminodesoxy-1.4;3.6-dianhydrohexitol nitrates of the general formula I,

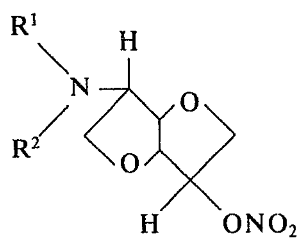

(I)

wherein

$R^1$ and $R^2$ independently of one another, signify a hydrogen atom or a lower alkyl group with 1 to 4 C-atoms

or wherein

$R^1$ signifies a hydrogen atom or a lower alkyl group with 1 to 4 C-atoms and
$R^2$ an acyl residue of an aliphatic or singly unsaturated, possibly methylsubstituted monocarboxylic acid with 2 to 8 C-atoms, a nicotinoyl, 2-O-acetylsalicoyl radical or a 1-adamantyl radical;

or wherein

$R^1$ signifies a hydrogen atom and
$R^2$ a 2-hydroxy-3-(subst.)-phenoxyprop-1-yl radical of the general formula Ia

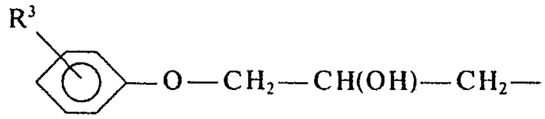

(Ia)

wherein

$R^3$ signifies a hydrogen atom, a lower alkyl group with 1 to 4 C-atoms or a lower alkenyl group with 2 to 4 C-atoms, the trifluoromethyl radical, a hydroxyl group, a lower alkoxy group with 1 to 4 C-atoms or a lower alkenyloxy group with 2 to 4 C-atoms, a cyano group or a carbamoylmethyl radical;

or wherein

$R^1$ signifies a hydrogen atom and
$R^2$ a 2-hydroxy-3-($\alpha$-naphthyloxy)-prop-1-yl radical, whereby the ring of the naphthalene skeleton which is not etherified with the hydroxypropyl group can be completely or partly hydrogenated or substituted by an oxo group;

or wherein

$R^1$ signifies a hydrogen atom or a lower alkyl group with 1 to 4 C-atoms and
$R^2$ an $\omega$-(subst.)-phenylalkyl group of the general formula Ib

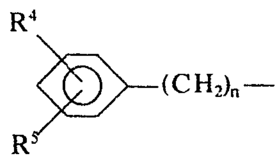

(Ib)

wherein

n can be = a whole number from 1 to 6 and
$R^4$ and $R^5$ in each case independently of one another, signify a hydrogen atom, a lower alkyl group with 1 to 4 C-atoms or a lower alkenyl group with 2 to 4 C-atoms, the trifluoromethyl radical, a hydroxyl group, a lower alkoxy group with 1 to 4 C-atoms or a lower alkenyloxy group with 2 to 4 C-atoms or a fluorine or chlorine atom;

or wherein

$R^1$ signifies a hydrogen atom or a lower alkyl group with 1 to 4 C-atoms and
$R^2$ the diphenylmethyl radical or cinnamyl radical;

or wherein

$R^1$ signifies a hydrogen atom or a lower alkyl group with 1 to 4 C-atoms and
$R^2$ an $\omega$-(subst.)-phenoxyalkyl group of the general formula Ic

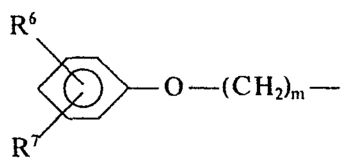

(Ic)

wherein

m can be = a whole number from 2—8 and
$R^6$ and $R^7$ independently of one another, signify a hydrogen atom, a lower alkyl group with 1 to 4 C-atoms or a lower alkenyl group with 2 to 4 C-atoms, the trifluoromethyl radical, a hydroxyl group, a lower alkoxy group with 1 to 4 C-atoms or a lower alkenyloxy group with 2 to 4 C-atoms, a fluorine or chlorine atom, the amino or acetylamino group, a mono- or di-lower alkylamino group with 1 to 4 C-atoms or
$R^6$ and $R^7$ together with the phenyl radical, from the $\alpha$-naphthyl radical;

or wherein

$R^1$ and $R^2$ together with the nitrogen atom to which they are attached, represent the residue of a cyclic, non-aromatic secondary amine with 5 to 7 ring atoms which possibly contains a further hetero atom;

or wherein

$R^1$ and $R^2$ together signify a pyridoxylidene radical; as well as their pharmacologically acceptable acid-addition salts.

2. 5-Amino-5-desoxy-1.4;3.6-dianhydro-L-iditol 2-nitrates of general formula V,

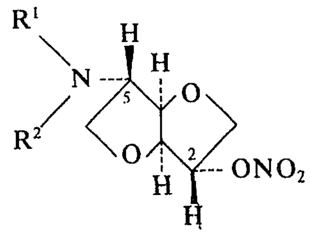

(V)

wherein $R^1$ an $R^2$ possess the meanings given in claim 1, as well as their pharmacologically acceptable acid-addition salts.

3. 5-Amino-5-desoxy-1.4;3.6-dianhydro-D-glucitol 2-nitrates of the general formula VI,

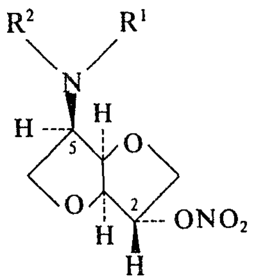

(VI)

wherein $R^1$ and $R^2$ possess the meanings given in claim 1, as well as their pharmacologically acceptable acid-addition salts.

4. 2-Amino-2-desoxy-1.4;3.6-dianhydro-D-glucitol 5-nitrates of the general formula VII,

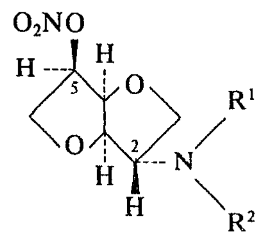

(VII)

wherein $R^1$ and $R^2$ possess the meanings given in claim 1, as well as their pharmacologically acceptable acid-addition salts.

5. 5-Amino-5-desoxy-1.4;3.6-dianhydro-D-mannitol 2-nitrates of the general formula VIII,

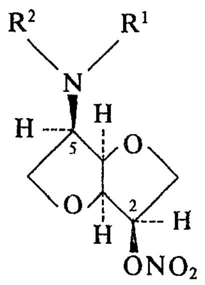

(VIII)

wherein $R^1$ and $R^2$ possess the meanings given in claim 1, as well as their pharmacologically acceptable acid-addition salts.

6. 5-Amino-5-desoxy-1.4;3.6-dianhydro-L-iditol 2-nitrate.
7. 5-Amino-5-desoxy-1.4;3.6-dianhydro-D-glucitol 2-nitrate.
8. 2-Amino-2-desoxy-1.4;3.6-dianhydro-D-glucitol 5-nitrate.
9. 5-Amino-5-desoxy-1.4;3.6-dianhydro-D-mannitol 2-nitrate.
10. 5-Pivaloylamino-5-desoxy-1.4;3.6-dianhydro-L-iditol 2-nitrate.
11. 5-Acetylamino-5-desoxy-1.4;3.6-dianhydro-L-iditol 2-nitrate.
12. 5-Nicotinoylamino-5-desoxy-1.4;3.6-dianhydro-L-iditol 2-nitrate.
13. 5-(2-Acetoxybenzoylamino)-5-desoxy-1.4;3.6-dianhydro-L-iditol 2-nitrate.
14. 5-Methylamino-5-desoxy-1.4;3.6-dianhydro-L-iditol 2-nitrate.
15. 5-Ethylamino-5-desoxy-1.4;3.6-dianhydro-L-iditol 2-nitrate.
16. 5-Adamant-1-ylamino-5-desoxy-1.4;3.6-dianhydro-L-iditol 2-nitrate.
17. 5-Dimethylamino-5-desoxy-1.4;3.6-dianhydro-L-iditol 2-nitrate.
18. 5-Diethylamino-5-desoxy-1.4;3.6-dianhydro-L-iditol 2-nitrate.
19. 5-Pyrrolidino-5-desoxy-1.4;3.6-dianhydro-L-iditol 2-nitrate.
20. 5-Piperidino-5-desoxy-1.4;3.6-dianhydro-L-iditol 2-nitrate.
21. 5-Morpholino-5-desoxy-1.4;3.6-dianhydro-L-iditol 2-nitrate.
22. 5-(4-Methylpiperazino)-5-desoxy-1.4;3.6-dianhydro-L-iditol 2-nitrate.
23. 5-Pyridoxylideneamino-5-desoxy-1.4;3.6-dianhydro-L-iditol 2-nitrate.
24. 5-(N-Benzyl-N-methylamino)-5-desoxy-1.4;3.6-dianhydro-L-iditol 2-nitrate.
25. 5-[N-(2-Phenylethyl)-N-methylamino]-5-desoxy-1.4;3.6-dianhydro-L-iditol 2-nitrate.
26. 5-[N-Methyl-N-(3-phenylpropyl)-amino]-5-desoxy-1.4;3.6-dianhydro-L-iditol 2-nitrate.
27. 5-[N-(4-Phenylbutyl)-N-methylamino]-5-desoxy-1.4;3.6-dianhydro-L-iditol 2-nitrate.
28. 5-[N-Methyl-N-(5-phenylpentyl)-amino]-5-desoxy-1.4;3.6-dianhydro-L-iditol 2-nitrate.
29. 5-(N-Cinnamyl-N-methylamino)-5-desoxy-1.4;3.6-dianhydro-L-iditol 2-nitrate.
30. 5-Benzylamino-5-desoxy-1.4;3.6-dianhydro-L-iditol 2-nitrate.
31. 5-Diphenylmethylamino-5-desoxy-1.4;3.6-dianhydro-L-iditol 2-nitrate.
32. 5-(2-Phenylethylamino)-5-desoxy-1.4;3.6-dianhydro-L-iditol 2-nitrate.
33. 5-(3-Phenylpropylamino)-5-desoxy-1.4;3.6-dianhydro-L-iditol 2-nitrate.
34. 5-(4-Phenylbutylamino-5-desoxy-1.4;3.6-dianhydro-L-iditol-2-nitrate.
35. 5-(5-Phenylpentylamino)-5-desoxy-1.4;3.6-dianhydro-L-iditol 2-nitrate.
36. 5-[3-(3.4-Dimethoxyphenyl)-propylamino]-5-desoxy-1.4;3.6-dianhydro-L-iditol 2-nitrate.
37. 5-[4-(4-Methoxyphenyl)-butylamino]-5-desoxy-1.4;3.6-dianhydro-L-iditol 2-nitrate.
38. 5-[N-(2-Phenoxyethyl)-N-methylamino]-5-desoxy-1.4;3.6-dianhydro-L-iditol 2-nitrate.
39. 5-[N-Methyl-N-(3-phenoxypropyl)-amino]-5-desoxy-1.4;3.6-dianhydro-L-iditol 2-nitrate.
40. 5-(2-Phenoxyethylamino)-5-desoxy-1.4;3.6-dianhydro-L-iditol 2-nitrate.
41. 5-(3-Phenoxypropylamino)-5-desoxy-1.4;3.6-dianhydro-L-iditol 2-nitrate.
42. 5-(4-Phenoxybutylamino)-5-desoxy-1.4;3.6-dianhydro-L-iditol 2-nitrate.
43. 5-(5-Phenoxypentylamino)-5-desoxy-1.4;3.6-dianhydro-L-iditol 2-nitrate.
44. 5-(6-Phenoxyhexylamino)-5-desoxy-1.4;3.6-dianhydro-L-iditol 2-nitrate.
45. 5-(7-Phenoxyheptylamino)-5-desoxy-1.4;3.6-dianhydro-L-iditol 2-nitrate.
46. 5-(8-Phenoxyoctylamino)-5-desoxy-1.4;3.6-dianhydro-L-iditol 2-nitrate.
47. 2-(3-Phenoxypropylamino)-2-desoxy-1.4;3.6-dianhydro-D-glucitol 5-nitrate.
48. 2-(4-Phenylbutylamino)-2-desoxy-1.4;3.6-dianhydro-D-glucitol 5-nitrate.
49. 5-[2-Hydroxy-3-(4-methoxyphenoxy)-propylamino]-5-desoxy-1.4;3.6-dianhydro-L-iditol 2-nitrate.
50. 5-[2-Hydroxy-3-(3-methoxyphenoxy)-propylamino]-5-desoxy-1.4;3.6-dianhydro-L-iditol 2-nitrate.
51. 5-[2-Hydroxy-3-(2-methoxyphenoxy)-propylamino]-5-desoxy-1.4;3.6-dianhydro-L-iditol 2-nitrate.
52. 5-[2-Hydroxy-3-(2-ethoxyphenoxy)-propylamino]-5-desoxy-1.4;3.6-dianhydro-L-iditol 2-nitrate.
53. 5-[2-Hydroxy-3-(2-allyloxyphenoxy)-propylamino]-5-desoxy-1.4;3.6-dianhydro-L-iditol 2-nitrate.
54. 5-[2-Hydroxy-3-(2-allylphenoxy)-propylamino]-5-desoxy-1.4;3.6-dianhydro-L-iditol 2-nitrate.
55. 5-[2-Hydroxy-3-(2-cyanophenoxy)-propylamino]-5-desoxy-1.4;3.6-dianhydro-L-iditol 2-nitrate.
56. 5-[2-Hydroxy-3-(3-tolyloxy)-propylamino]-5-desoxy-1.4;3.6-dianhydro-L-iditol 2-nitrate.
57. 5-[2-Hydroxy-3-(3-trifluormethylphenoxy)-propylamino]-5-desoxy-1.4;3.6-dianhydro-L-iditol 2-nitrate.
58. 5-[2-Hydroxy-3-(4-carbamoylmethylphenoxy)-propylamino]-5-desoxy-1.4;3.6-dianhydro-L-iditol-2 nitrate.
59. 5-[2-Hydroxy-3-(1-naphthyloxy)-propylamino]-5-desoxy-1.4;3.6-dianhydro-L-iditol 2-nitrate.
60. 5-[2-Hydroxy-3-(1-naphthyloxy)-propylamino]-5-desoxy-1.4;3.6-dianhydro-D-glucitol 2-nitrate.

61. 5-[2-Hydroxy-3-(1-oxo-1.2.3.4-tetrahydro-5-naphthyloxy)-propylamino]-5-desoxy-1.4; 3.6-dianhydro-L-iditol 2-nitrate.

62. 5-[2-Hydroxy-3-(5.6.7.8-tetrahydro-1-naphthyloxy)-propylamino]-5-desoxy-1.4;3.6-dianhydro-L-iditol 2-nitrate.

63. 5-[3-(3-Trifluoromethylphenoxy)-propylamino]-5-desoxy-1.4;3.6-dianhydro-L-iditol 2-nitrate.

64. 5-[3-(4-Tolyloxy)-propylamino]-5-desoxy-1.4;3.6-dianhydro-L-iditol 2-nitrate.

65. 5-[3-(4-Fluorophenoxy)-propylamino]-5-desoxy-1.4;3.6-dianhydro-L-iditol 2-nitrate.

66. 5-[3-(1-Naphthyloxy)-propylamino]-5-desoxy-1.4;3.6-dianhydro-L-iditol 2-nitrate.

67. 5-[3-(4-Methoxyphenoxy)-propylamino]-5-desoxy-1.4;3.6-dianhydro-L-iditol 2-nitrate.

68. 5-[3-(3-Methoxyphenoxy)-propylamino]-5-desoxy-1.4;3.6-dianhydro-L-iditol 2-nitrate.

69. 5-[3-(2-Methoxyphenoxy)-propylamino]-5-desoxy-1.4;3.6-dianhydro-L-iditol 2-nitrate.

70. 5-[3-(2,6-Dimethoxyphenoxy)-propylamino]-5-desoxy-1.4;3.6-dianhydro-L-iditol 2-nitrate.

71. 5-[3-(3,5-Dimethoxyphenoxy)-propylamino]-5-desoxy-1.4;3.6-dianhydro-L-iditol 2-nitrate.

72. 5-[3-(2,3-Dimethoxyphenoxy)-propylamino]-5-desoxy-1.4;3.6-dianhydro-L-iditol 2-nitrate.

73. 5-[3-(4-Chlorophenoxy)-propylamino]-5-desoxy-1.4;3.6-dianhydro-L-iditol 2-nitrate.

74. 5-[3-(2-Chlorophenoxy)-propylamino]-5-desoxy-1.4;3.6-dianhydro-L-iditol 2-nitrate.

75. 5-[3-(3-Chlorophenoxy)-propylamino]-5-desoxy-1.4;3.6-dianhydro-L-iditol 2-nitrate.

76. 5-[3-(3,4-Dichlorophenoxy)-propylamino]-5-desoxy-1.4;3.6-dianhydro-L-iditol 2-nitrate.

77. 5-[3-(2,4-Dichlorophenoxy)-propylamino]-5-desoxy-1.4;3.6-dianhydro-L-iditol 2-nitrate.

78. 5-[3-(4-Acetamidophenoxy)-propylamino]-5-desoxy-1.4;3.6-dianhydro-L-iditol 2-nitrate.

79. 5-[3-(3-Dimethylaminophenoxy)-propylamino]-5-desoxy-1.4;3.6-dianhydro-L-iditol 2-nitrate.

80. 5-[3-(4-Chlorophenyl)-propylamino]-5-desoxy-1.4;3.6-dianhydro-L-iditol 2-nitrate.

81. 5-[4-(4-Chlorophenyl)-butylamino]-5-desoxy-1.4;3.6-dianhydro-L-iditol 2-nitrate.

82. 5-Nicotinoylamino-5-desoxy-1.4;3.6-dianhydro-D-mannitol 2-nitrate.

83. 5-N-Methyl-N-nicotinoylamino-5-desoxy-1.4;3.6-dianhydro-L-iditol 2-nitrate.

84. Process for the preparation of the compounds according to claim 1, characterised in that

(A) L-isoidide, D-isosorbide or D-isomannide is converted with a sulphonic acid chloride into the corresponding mono-O-acyl-1.4;3.6-dianhydrohexitol,

(B) which, by ammonolysis with aqueous ammonia solution or by aminolysis with a primary or secondary alkylamine with 1 to 4 C-atoms, with a primary amine or with a cyclic, non-aromatic secondary amine, is reacted to give the corresponding aminodesoxy-1.4;3.6-dianhydrohexitol, possibly with the addition of a suitable solvent,

(C) whereafter the free hydroxyl group of the resultant aminodesoxy-1.4;3.6-dianhydrohexitol or possibly its acid-addition salt is esterified with nitric acid, nitrating acid or with a mixture of nitric acid and glacial acetic acid/acetic anhydride in the presence of urea to give the corresponding aminodesoxy-1.4;3.6-dianhydrohexitol nitrate, and

(D) whereafter the nitrate is possibly reacted with the desired reactive compound forming the substituent $R^2$, namely, with a carboxylic acid chloride, an aldehyde, an $\omega$-(subst.)-arylalkyl halide, $\omega$-phenylalkenyl halide, $\omega$-(subst.)-arylalkyl methanesulphonate, $\omega$-(subst.)-aryloxyalkyl halide, 1,2-epoxy-$\omega$-(subst.)-phenoxyalkane, 1,2-epoxy-$\omega$-(subst.)-(1-naphthyloxy)-alkane or $\omega$-(subst.)-phenoxyalkyl methanesulphonate.

85. Process for the preparation of the compounds according to claim 2, characterised in that

(A') D-isosorbide is reacted quantitatively with an excess of sulphonic acid chloride to give the corresponding isosorbide diacylate,

(B') which, by ammonolysis with aqueous ammonia solution or by aminolysis with a primary or secondary alkylamine with 1 to 4 C-atoms or with a cyclic, non-aromatic secondary amine, is reacted to give the corresponding 5-amino-5-desoxy-1.4;3.6-dianhydro-L-iditol 2-sulphonate,

(C') whereafter the reaction mixture obtained under (B') is subjected to an acidic or alkaline hydrolysis and

(D') the so obtained 5-amino-5-desoxy-1.4;3.6-dianhydro-L-iditol is further worked up according to claim 84 (C) and possibly (D).

86. Process for the preparation of the compounds according to claim 3, characterised in that

(A'') D-isosorbide is reacted quantitatively with an excess of sulphonic acid chloride to give the corresponding isosorbide diacylate,

(B'') which is selectively reacted with sodium benzoate to give the corresponding 1.4;3.6-dianhydro-L-iditol 2-sulphonate 5-benzoate,

(C'') whereafter the latter, by ammonolysis with aqueous ammonia solution or by aminolysis with a primary or secondary alkylamine with 1 to 4 C-atoms or with a cyclic, non-aromatic secondary amine, is reacted to give the corresponding 5-amino-5-desoxy-1.4;3.6-dianhydro-D-glucitol, whereby the benzoic acid radical is simultaneously split off hydrolytically and

(D") the so obtained 5-amino-5-desoxy-1.4;3.6-dianhydro-D-glucitol is further worked up according to claim 84 (C) and possibly (D).

87. Process according to one of claims 84 to 86, characterised in that methanesulphonyl chloride or toluenesulphonyl chloride is used as said sulphonic acid chloride.

88. Process according to claim 86, characterised in that the reaction with sodium benzoate (B") is carried out in an anhydrous, dipolar, aprotic solvent under an inert gas atmosphere at an elevated temperature.

89. Process according to claim 88, characterised in that the reaction with sodium benzoate is carried out in dimethylformamide at a temperature of between 100 and 180°C.

90. Process according to one of claims 84 to 88, characterised in that the ammonolysis or aminolysis is carried out under an elevated pressure and elevated temperature.

91. Process according to claim 90, characterised in that the ammonolysis or aminolysis is carried out at a temperature of between 90 and 180°C.

92. Process according to claim 85, characterised in that the ammonolysis or aminolysis is carried out at a temperature of between 90 and 120°C.

93. Pharmaceutical composition, containing a compound according to one of claims 1 to 83, as well as conventional carriers and additive materials.

**Revendications**

1. Amino-désoxy-1.4;3.6-dianhydro-hexit-nitrates de formule générale I

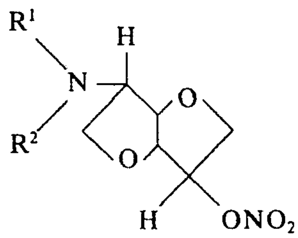

(I)

où

$R^1$ et $R^2$ représentent chacun indépendamment l'un de l'autre un atome d'hydrogène ou un groupe alcoyle inférieur en $C_1$ à $C_4$,

ou bien où

$R^1$ représente un atome d'hydrogène ou un groupe alcoyle inférieur en $C_1$ à $C_4$ et
$R^2$ représente un radical acyle d'un acide monocarboxylique aliphatique ou ne comportant qu'une liaison multiple, éventuellement substitué par un méthyle, en $C_2$ à $C_8$, un radical nicotinoyle, 2,0-acétylsalicoyle ou un radical 1-adamantyle,

ou bien où

$R^1$ représente un atome d'hydrogène et
$R^2$ représente un radical 2-hydroxy-3-(subst.)-phénoxyprop-1-yle de formule générale Ia

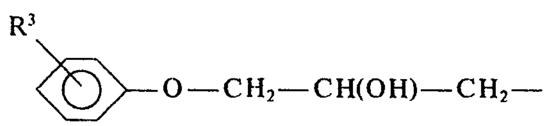

(Ia)

où

$R^3$ représente un atome d'hydrogène, un groupe alcoyle inférieur en $C_1$ à $C_4$ ou un groupe alcényle inférieur en $C_2$ à $C_4$, le radical trifluorométhyle, un groupe hydroxy, un groupe alcoxy inférieur en $C_1$ à $C_4$ ou un groupe alcényloxy inférieur en $C_2$ à $C_4$, un groupe cyano ou un radical carbamoylméthyle,

ou bien où

$R^1$ représente un atome d'hydrogène et

$R^2$ représente un radical 2-hydroxy-3-($\alpha$-naphthyloxy)-prop-1-yle, où le noyau non éthérifié avec le groupe hydroxypropyle du squelette naphtalène peut être entièrement ou partiellement hydrogéné et substitué par un groupe oxo,

ou bien où

$R^1$ représente un atome d'hydrogène ou un groupe alcoyle inférieur en $C_1$ à $C_4$ et
$R^2$ représente un groupe $\omega$-(subst.)-phénylalcoyle de formule générale Ib

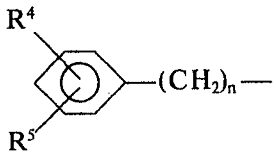

(Ib)

où

n peut être un nombre entier allant de 1 à 6 et
$R^4$ et $R^5$ représentent chacun indépendamment l'un de l'autre un atome d'hydrogène, un groupe alcoyle inférieur en $C_1$ à $C_4$ ou un groupe alcényle inférieur en $C_2$ à $C_4$, le radical trifluorométhyle, un groupe hydroxy, un groupe alcoxy inférieur en $C_1$ à $C_4$ ou un groupe alcényloxy inférieur en $C_2$ à $C_4$ ou un atome de fluor ou de chlore,

ou bien où

$R^1$ représente un atome d'hydrogène ou un groupe alcoyle inférieur en $C_1$ à $C_4$ et
$R^2$ représente le radical diphénylméthyle ou le radical cinnamyle,

ou bien où

$R^1$ représente un atome d'hydrogène ou un groupe alcoyle inférieur en $C_1$ à $C_4$ et
$R^2$ représente un groupe $\omega$-(subst.)-phénoxyalcoyle de formule générale Ic

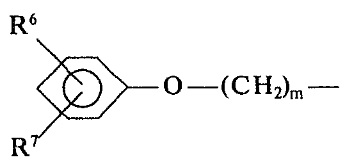

(Ic)

où

m peut être un nombre entier allant de 2 à 8 et
$R^6$ et $R^7$ représentent indépendamment l'un de l'autre un atome d'hydrogène, un groupe alcoyle inférieur en $C_1$ à $C_4$ ou un groupe alcényle inférieur en $C_2$ à $C_4$, le radical trifluorométhyle, un groupe hydroxy, un groupe alcoxy inférieur en $C_1$ à $C_4$ ou un groupe alcényloxy inférieur en $C_2$ à $C_4$, un atome de fluor ou de chlore, le groupe amino ou acétylamino, un groupe mono- ou di-alcoyle inférieur-amino en $C_1$ à $C_4$ ou
$R^6$ et $R^7$ formente ensemble avec le radical phényle le radical $\alpha$-naphtyle,

ou bien où

$R^1$ et $R^2$ représentent ensemble avec l'atome d'azote auquel ils sont liés le radical d'une amine secondaire cyclique, non aromatique, contenant éventuellement un autre hétéroatome et ayant de 5 à 7 atomes dans son noyau,

ou bien où

$R^1$ et $R^2$ représentent ensemble le radical pyridoxylidène,
ainsi que leurs sels d'addition d'acides pharmacologiquement acceptables.

2. 5-amino-5-désoxy-1.4;3.6-dianhydro-L-idit-2-nitrates de formula générale V

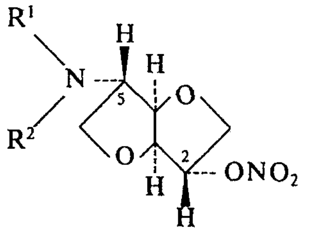

(V)

où $R^1$ et $R^2$ ont les significations données dans la revendication 1, ainsi que leurs sels d'addition d'acides pharmacologiquement acceptables.

3. 5-amino-5-désoxy-1.4;3.6-dianhydro-D-glucit-2-nitrates de formule générale VI

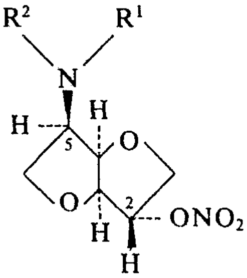

(VI)

où $R^1$ et $R^2$ ont les significations données dans la revendication 1, ainsi que leurs sels d'addition d'acides pharmacologiquement acceptables.

4. 2-amino-2-désoxy-1.4;3.6-dianhydro-D-glucit-5-nitrates de formule générale VII

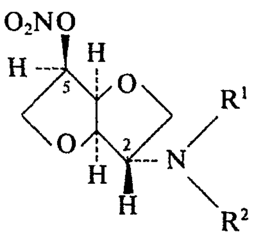

(VII)

où $R^1$ et $R^2$ ont les significations données dans la revendication 1, ainsi que leurs sels d'addition d'acides pharmacologiquement acceptables.

5. 5-amino-4-désoxy-1.4;3.6-dianhydro-D-mannit-2-nitrates de formule générale VIII

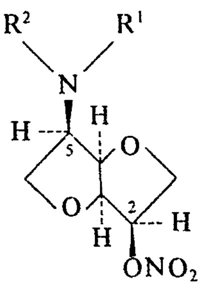

(VIII)

où $R^1$ et $R^2$ ont la signification donnée dans la revendication 1, ainsi que leurs sels d'addition d'acides pharmacologiquement acceptables.

6. 5-amino-5-désoxy-1.4;3.6-dianhydro-L-idit-2-nitrate.
7. 5-amino-5-désoxy-1.4;3.6-dianhydro-D-glucit-2-nitrate.
8. 2-amino-2-désoxy-1.4;3.6-dianhydro-D-glucit-5-nitrate.
9. 5-amino-5-désoxy-1.4;3.6-dianhydro-D-mannit-2-nitrate.
10. 5-Pivaloylamino-5-désoxy-1.4;3.6-dianhydro-L-idit-2-nitrate.
11. 5-Acétylamino-5-désoxy-1.4;3.6-dianhydro-L-idit-2-nitrate.
12. 5-Nicotinoylamino-5-désoxy-1.4;3.6-dianhydro-L-idit-2-nitrate.
13. 5-(2-Acétoxybenzoylamino)-5-désoxy-1.4;3.6-dianhydro-L-idit-2-nitrate.
14. 5-Méthylamino-5-désoxy-1.4;3.6-dianhydro-L-idit-2-nitrate.
15. 5-éthy'amino-5-désoxy-1.4;3.6-dianhydro-L-idit-2-nitrate.
16. 5-Adamant-1-ylamino-5-désoxy-1.4;3.6-dianhydro-L-idit-2-nitrate.

17. 5-Diméthylamino-5-désoxy-1.4;3.6-dianhydro-L-idit-2-nitrate.
18. 5-Diéthylamino-5-désoxy-1.4;3.6-dianhydro-L-idit-2-nitrate.
19. 5-Pyrrolidino-5-désoxy-1.4;3.6-dianhydro-L-idit-2-nitrate.
20. 5-Pipéridino-5-désoxy-1.4;3.6-dianhydro-L-idit-2-nitrate.
21. 5-Morpholino-5-désoxy-1.4;3.6-dianhydro-L-idit-2-nitrate.
22. 5-(4-Méthylpiperazino)-5-désoxy-1.4;3.6-dianhydro-L-idit-2-nitrate.
23. 5-Pyridoxylidènamino-5-désoxy-1.4;3.6-dianhydro-L-idit-2-nitrate.
24. 5-(N-Benzyl-N-méthylamino)-5-désoxy-1.4;3.6-dianhydro-L-idit-2-nitrate.
25. 5-[N-(2-Phényléthyl)-N-méthylamino]-5-désoxy-1.4;3.6-dianhydro-L-idit-2-nitrate.
26. 5-[N-Méthyl-N-(3-phénylpropyl)-amino]-5-désoxy-1.4;3.6-dianhydro-L-idit-2-nitrate.
27. 5-[N-(4-Phénylbutyl)-N-méthylamino]-5-désoxy-1.4;3.6-dianhydro-L-idit-2-nitrate.
28. 5-[N-Méthyl-N-(5-phénylpentyl)-amino]-5-désoxy-1.4;3.6-dianhydro-L-idit-2-nitrate.
29. 5-[N-Cinnamyl-N-méthylamino)-5-désoxy-1.4;3.6-dianhydro-L-idit-2-nitrate.
30. 5-Benzylamino-5-désoxy-1.4;3.6-dianhydro-L-idit-2-nitrate.
31. 5-Diphénylméthylamino-5-désoxy-1.4;3.6-dianhydro-L-idit-2-nitrate.
32. 5-(2-Phényléthylamino)-5-désoxy-1.4;3.6-dianhydro-L-idit-2-nitrate.
33. 5-(3-Phénylpropylamino)-5-désoxy-1.4;3.6-dianhydro-L-idit-2-nitrate.
34. 5-(4-Phénylbutylamino)-5-désoxy-1.4;3.6-dianhydro-L-idit-2-nitrate.
35. 5-(5-Phénylpentylamino)-5-désoxy-1.4;3.6-dianhydro-L-idit-2-nitrate.
36. 5-[3-(3.4-Diméthoxyphényl)-propylamino]-5-désoxy-1.4;3.6-dianhydro-L-idit-2-nitrate.
37. 5-[4-(4-Méthoxyphényl)-butylamino]-5-désoxy-1.4;3.6-dianhydro-L-idit-2-nitrate.
38. 5-[N-(2-Phénoxyéthyl)-N-méthylamino]-5-désoxy-1.4;3.6-dianhydro-L-idit-2-nitrate.
39. 5-[N-Méthyl-N-(3-phénoxypropyl)-amino]-5-désoxy-1.4;3.6-dianhydro-L-idit-2-nitrate.
40. 5-(2-Phénoxyéthylamino)-5-désoxy-1.4;3.6-dianhydro-L-idit-2-nitrate.
41. 5-(3-Phénoxypropylamino)-5-désoxy-1.4;3.6-dianhydro-L-idit-2-nitrate.
42. 5-(4-Phénoxybutylamino)-5-désoxy-1.4;3.6-dianhydro-L-idit-2-nitrate.
43. 5-(5-Phénoxypentylamino)-5-désoxy-1.4;3.6-dianhydro-L-idit-2-nitrate.
44. 5-(6-Phénoxyhexylamino)-5-désoxy-1.4;3.6-dianhydro-L-idit-2-nitrate.
45. 5-(7-Phénoxyheptylamino)-5-désoxy-1.4;3.6-dianhydro-L-idit-2-nitrate.
46. 5-(8-Phénoxyoctylamino)-5-désoxy-1.4;3.6-dianhydro-L-idit-2-nitrate.
47. 2-(3-Phénoxypropylamino)-2-désoxy-1.4;3.6-dianhydro-D-glucit-5-nitrate.
48. 2-(4-Phénylbutylamino)-2-désoxy-1.4;3.6-dianhydro-D-glucit-5-nitrate.
49. 5-[(2-Hydroxy-3-(4-méthoxyphénoxy)-propylamino]-5-désoxy-1.4;3.6-dianhydro-L-idit-2-nitrate.
50. 5-[2-Hydroxy-3-(3-méthoxyphénoxy)-propylamino]-5-désoxy-1.4;3.6-dianhydro-L-idit-2-nitrate.
51. 5-[2-Hydroxy-3-(2-méthoxyphénoxy)-propylamino]-5-désoxy-1.4;3.6-dianhydro-L-idit-2-nitrate.
52. 5-[2-Hydroxy-3-(2-éthoxyphénoxy)-propylamino]-5-désoxy-1.4;3.6-dianhydro-L-idit-2-nitrate.
53. 5-[2-Hydroxy-3-(2-allyloxyphénoxy)-propylamino]-5-désoxy-1.4;3.6-dianhydro-L-idit-2-nitrate.
54. 5-[2-Hydroxy-3-(2-allylphénoxy)-propylamino]-5-désoxy-1.4;3.6-dianhydro-L-idit-2-nitrate.
55. 5-[2-Hydroxy-3-(2-cyanophénoxy)-propylamino]-5-désoxy-1.4;3.6-dianhydro-L-idit-2-nitrate.
56. 5-[2-Hydroxy-3-(3-tolyloxy)-propylamino]-5-désoxy-1.4;3.6-dianhydro-L-idit-2-nitrate.
57. 5-[2-Hydroxy-3-(3-trifluorométhylphénoxy)-propylamino]-5-désoxy-1.4;3.6-dianhydro-L-idit-2-nitrate.
58. 5-[2-Hydroxy-3-(4-carbamoylméthylphénoxy)-propylamino]-5-désoxy-1.4;3.6-dianhydro-L-idit-2-nitrate.
59. 5-[2-Hydroxy-3-(1-naphtyloxy)-propylamino]-5-désoxy-1.4;3.6-dianhydro-L-idit-2-nitrate.
60. 5-[2-Hydroxy-3-(1-naphtyloxy)-propylamino]-5-désoxy-1.4;3.6-dianhydro-D-glucit-2-nitrate.
61. 5-[(2-Hydroxy-3-(1-oxo-1.2.3.4-tétrahydro-5-naphtyloxy)-propylamino]-5-désoxy-1.4;3.6-dianhydro-L-idit-2-nitrate.
62. 5-[2-Hydroxy-3-(5.6.7.8-tétrahydro-1-naphtyloxy)-propylamino]-5-désoxy-1.4;3.6-dianhydro-L-idit-2-nitrate.
63. 5-[3-(3-Trifluorométhylphénoxy)-propylamino]-5-désoxy-1.4;3.6-dianhydro-L-idit-2-nitrate.
64. 5-[3-(4-Tolyloxy)-propylamino]-5-désoxy-1.4;3.6-dianhydro-L-idit-2-nitrate.
65. 5-[3-(4-Fluorophénoxy)-propylamino]-5-désoxy-1.4;3.6-dianhydro-L-idit-2-nitrate.
66. 5-[3-(1-Naphtyloxy)-propylamino]-5-désoxy-1.4;3.6-dianhydro-L-idit-2-nitrate.
67. 5-[3-(4-Méthoxyphénoxy)-propylamino]-5-désoxy-1.4;3.6-dianhydro-L-idit-2-nitrate.
68. 5-[3-(3-Méthoxyphénoxy)-propylamino]-5-désoxy-1.4;3.6-dianhydro-L-idit-2-nitrate.
69. 5-[3-(2-Méthoxyphénoxy)-propylamino]-5-désoxy-1.4;3.6-dianhydro-L-idit-2-nitrate.
70. 5-[3-(2.6-Diméthoxyphénoxy)-propylamino]-5-désoxy-1.4;3.6-dianhydro-L-idit-2-nitrate.
71. 5-[3-(3.5-Diméthoxyphénoxy)-propylamino]-5-désoxy-1.4;3.6-dianhydro-L-idit-2-nitrate.
72. 5-[3-(2.3-Diméthoxyphénoxy)-propylamino]-5-désoxy-1.4;3.6-dianhydro-L-idit-2-nitrate.

73. 5-[3-(4-Chlorophénoxy)-propylamino]-5-désoxy-1.4;3.6-dianhydro-L-idit-2-nitrate.
74. 5-[3-(2-Chlorophénoxy)-propylamino]-5-désoxy-1.4;3.6-dianhydro-L-idit-2-nitrate.
75. 5-[3-(3-Chlorophénoxy)-propylamino]-5-désoxy-1.4;3.6-dianhydro-L-idit-2-nitrate.
76. 5-[3-(3.4-Dichlorophénoxy)-propylamino]-5-désoxy-1.4;3.6-dianhydro-L-idit-2-nitrate.
77. 5-[3-(2.4-Dichlorophénoxy)-propylamino]-5-désoxy-1.4;3.6-dianhydro-L-idit-2-nitrate.
78. 5-[3-(4-Acétamidophénoxy)-propylamino]-5-désoxy-1.4;3.6-dianhydro-L-idit-2-nitrate.
79. 5-[3-(3-Diméthylaminophénoxy)-propylamino]-5-désoxy-1.4;3.6-dianhydro-L-idit-2-nitrate.
80. 5-[3-(4-Chlorophényl)-propylamino]-5-désoxy-1.4;3.6-dianhydro-L-idit-2-nitrate.
81. 5-[4-(4-Chlorophényl)-butylamino]-5-désoxy-1.4;3.6-dianhydro-L-idit-2-nitrate.
82. 5-Nicotinoylamino-5-désoxy-1.4;3.6-dianhydro-D-mannit-2-nitrate.
83. 5-N-Méthyl-N-nicotinoylamino-5-désoxy-1.4;3.6-dianhydro-L-idit-2-nitrate.
84. Procédé de préparation des composés selon la revendication 1, caractérisé en ce que

(A) On transforme du L-isoidite, du D-isosorbide ou du D-isomannide avec un chlorure d'acide sulfonique pour donner le mono-O-acyl-1.4;3.6-dianhydro-hexite correspondant,
(B) que l'on fait réagir par ammonolyse avec une solution aqueuse d'ammoiaque ou par aminolyse avec une alcoylamine primaire ou secondaire en $C_1$ à $C_4$, avec une amine primaire ou avec une amine secondaire cyclique, non aromatique, éventuellement en ajoutant un solvant approprié, pour donner l'amino-désoxy-1.4;3.6-dianhydro-hexite correspondant,
(C) après quoi on estérifie le groupe hydroxyle libre de l'amino-désoxy-1.4;3.6-dianhydro-hexite paru ou éventuellement de son sel d'addition d'acide avec de l'acide nitrique, un mélange sulfonitrique ou avec un mélange d'acide nitrique et d'acide acétique glacial/acétanhydride en présence d'urée pour donner l'amino-désoxy-1.4;3.6-dianhydro-hexitnitrate correspondant, et
(D) après quoi si on le désire on fait le cas échéant réagir le nitrate avec le composé réactif désiré formant le substituant $R^2$, à savoir un chlorure d'acide carboxylique, un aldéhyde, un halogénure d'$\omega$-(subst.)-aryl-alcoyle, un halogénure d'$\omega$-phényl-alcényle, un $\omega$-(subst.)-arylalcoyl-méthanesulfonate, un halogénure d'$\omega$-(subst.)-aryloxyalcoyle, un 1,2-époxy-$\omega$-(subst.)-phénoxy-alcane, un 1.2-époxy-$\omega$-(subst.)-(1-naphtyloxy)-alcane ou un $\omega$-(subst.)-phénoxyalcoyl-méthanesulfonate.

85. Procédé de préparation des composés selon la revendication 2, caractérisé en ce que

(A') On fait réagir de façon quantitative du D-isosorbide avec un excès de chlorure d'acide sulfonique pour donner l'isosorbide-diacylate correspondant,
(B') que l'on fait réagir par ammonolyse avec une solution aqueuse d'ammoniaque ou par aminolyse avec une alcoylamine primaire ou secondaire en $C_1$ à $C_4$ ou avec une amine secondaire cyclique, non aromatique, pour donner le 5-amino-5-désoxy-1.4;3.6-dianhydro-L-idit-2-sulfonate correspondant,
(C') après quoi on soumet le mélange réactionnel obtenu en (B') à une hydrolyse acide ou alcaline et
(D') on traite plus avant le 5-amino-5-désoxy-1.4;3.6-dianhydro-L-idite ainsi apparu selon la revendication 84, (C) et le cas échéant (D).

86. Procédé de préparation des composés selon la revendication 3, caractérisé en ce que

(A'') On fait réagir quantitativement du D-isosorbide avec un excès de chlorure d'acide sulfonique pour donner l'isosorbide-diacylate correspondant,
(B'') que l'on fait réagir sélectivement avec du benzoate de sodium pour donner le 1.4;3.6-dianhydro-L-idit-2-sulfonate-5-benzoate correspondant,
(C'') après quoi on fait réagir ce dernier par ammonolyse avec une solution aqueuse d'ammoniaque ou par aminolyse avec une alcoylamine primaire ou secondaire en $C_1$ à $C_4$ ou avec une amine secondaire cyclique, non aromatique, et l'on sépare simultanément par hydrolyse le radical acide benzoïque, pour donner le 5-amino-5-désoxy-1.4;3.6-dianhydro-D-glucite correspondant et
(D'') on traite plus avant le 5-amino-5-désoxy-1.4;3.6-dianhydro-D-glucite ainsi apparu selon la revendication 84, (C) et le cas échéant (D).

87. Procédé selon l'une des revendications 84 à 86, caractérisé en ce qu'on utilise comme chlorure d'acide sulfonique le chlorure de l'acide méthanesulfonique ou le chlorure de l'acide toluènesulfonique.

88. Procédé selon la revendication 86, caractérisé en ce qu'on conduit la réaction avec du benzoate de sodium (B'') dans un solvant aprotique dipolaire anhydride sous une atmosphère de gaz inerte à température augmentée.

89. Procédé selon la revendication 88, caractérisé en ce qu'on conduit la réaction avec du benzoate de sodium dans le diméthylformamide à une température comprise entre 100 et 180°C.

90. Procédé selon l'une des revendications 84 à 88, caractérisé en ce qu'on conduit l'ammonolyse ou l'aminolyse à pression accrue et à température augmentée.

91. Procédé selon la revendication 90, caractérisé en ce qu'on conduit l'ammonolyse ou selon les cas l'aminolyse à une température comprise entre 90 et 180°C.

92. Procédé selon la revendication 85, caractérisé en ce qu'on conduit l'ammonolyse ou selon les cas l'aminolyse à une température comprise entre 90 et 120°C.

93. Préparation pharmaceutique contenant un composé selon l'une des revendications 1 à 83 ainsi que les supports et additifs habituels.